# EUROPEAN PATENT SPECIFICATION

(11) **EP 2 897 970 B1**
(45) Date of publication and mention of the grant of the patent: **13.07.2016**
(21) Application number: 13776739.8
(22) Date of filing: 16.09.2013
(51) Int. Cl.: C07H 15/26, A61K 31/7048, A61K 31/7052, A61P 31/04, A61P 31/06

(54) **MACROLIDE DERIVATIVES, PREPARATION THEREOF AND THERAPEUTIC USE THEREOF**
MACROLIDDERIVATE, HERSTELLUNG DAVON UND THERAPEUTISCHE VERWENDUNG DAVON
DÉRIVÉS DE MACROLIDE, LEUR PRÉPARATION ET LEUR UTILISATION THÉRAPEUTIQUE

(30) Priority: 18.09.2012 FR 1258744
(43) Date of publication of application: 29.07.2015
(73) Proprietor: SANOFI, 75008 Paris (FR)
(72) Inventor: BAURIN, Nicolas, F-75008 Paris (FR); BENEDETTI, Yannick, F-75008 Paris (FR); BOULEY, Emmanuel, F-75008 Paris (FR); ZHANG, Jidong, F-75008 Paris (FR)
(74) Representative: Werner, Alain Henri
(86) International application number: PCT/EP2013/069185
(87) International publication number: WO 2014/044645

(56) References cited:
- FR-A1- 2 110 246
- US-A- 4 585 759
- BERNADETTE ARNOUX: "23672 RP, a New Macrolide Antibiotic from Streptomyces chryseus. Mass Spectrometry Study and X-ray Structure Determination", JOURNAL OF THE AMERICAN CHEMICAL SOCIETY, vol. 102, no. 10, 1 January 1980 (1980-01-01), pages 3605-3608, XP055055399, cited in the application
- KENJI ARAKAWA ET AL: "Isolation, structural elucidation, and biosynthesis of 15-norlankamycin derivatives produced by a type-II thioesterase disruptant of Streptomyces rochei", TETRAHEDRON, vol. 67, no. 29, 1 July 2011 (2011-07-01), pages 5199-5205, XP055055425, ISSN: 0040-4020, DOI: 10.1016/j.tet.2011.05.047
- K. FALZARI ET AL: "In Vitro and In Vivo Activities of Macrolide Derivatives against Mycobacterium tuberculosis", ANTIMICROBIAL AGENTS AND CHEMOTHERAPY, vol. 49, no. 4, 1 April 2005 (2005-04-01), pages 1447-1454, XP055055436, ISSN: 0066-4804, DOI: 10.1128/AAC.49.4.1447-1454.2005

## Description

The present invention relates to macrolide derivatives, and to the preparation and therapeutic use thereof. The compounds according to the present invention have substantial antimicrobial activity, mainly on gram-positive microorganisms, and also on mycobacteria, especially in the treatment of tuberculosis.

Due to the appearance of resistance, the development of novel antibacterial agents is necessary to make it possible to kill or to prevent the growth of mycobacteria, especially those which induce tuberculosis.

Tuberculosis is a disease which, at the present time, is still a worldwide health threat. Globally, a third of the human population is infected with *Mycobacterium tuberculosis.* Despite the fact that treatments exist and that the disease is curable, tuberculosis killed approximately 1.82 million people in 2008, and its global incidence increases by 1% per year, with an estimation in 2008 of 9.4 million annual new cases of declared disease. Added to this are the difficulties of correct prescription and of adherence to the treatment protocols, and also the emergence of multi-resistant strains of *M. tuberculosis.* Drug-drug interactions also interfere with the optimum treatment of AIDS and tuberculosis in the case of co-infected patients.

The common treatment protocols for combating sensitive strains of M. *tuberculosis* are mainly based on a combination of three or, more frequently, of four molecules: isoniazide (INH), rifampicin (RIF), pyrazinamide (PZA) and ethambutol (EMB). These drugs constitute the "first-line" treatment.

In recent decades, tuberculosis has become resistant to each of these molecules. Strains that are resistant at least to isoniazide and to rifampicin are referred to as "multi-resistant" (MDR-TB). Recently, novel strains have appeared which are resistant to a larger number of molecules: those that are resistant to isoniazide, to rifampicin, to fluoroquinolones and to at least one injectable second-line drug are defined as being "ultra-resistant" (XDR-TB).

According to an estimation made by the WHO in 2009, there were 0.5 million cases of MDR-TB in 2007. Other evaluations report a relative incidence of about 11 % of multi-resistant strains among all new cases of tuberculosis.

Another therapeutic drawback in the treatment of tuberculosis is the interaction of rifampicin with treatments for combating HIV (human immunodeficiency virus), which represents an obstacle in the treatment of patients co-infected with tuberculosis and HIV. The current anti-HIV therapeutic recommendations favour, as a first-line treatment, an anti-retroviral triple therapy combining a protease inhibitor (PI) or a non-nucleoside reverse transcriptase inhibitor (NNRTI) with two nucleoside reverse transcriptase inhibitors (NRTI). PI and NNRTI are metabolized by CYP3A4. Metabolic interactions between anti-retrovirals (ATRV) and certain combined drugs have been demonstrated. Thus, rifampicin, which is a powerful inducer of intestinal and hepatic CYP3A4, reduces the concentrations of ATRV.

There is an urgent need to develop improved therapies for combating tuberculosis. These novel anti-tuberculosis treatments should be capable of satisfying one or more of the following criteria:
- shorten the treatment time to improve the adherence to the treatment protocols and reduce the appearance of resistant bacteria,
- be well tolerated, acting via novel mechanisms of action and thus effective against multi-resistant and/or ultra-resistant strains,
- be active against tuberculosis,
- have a shortened latent tuberculosis (asymptomatic first infection) treatment time, so as to address the problem of the biological reservoir of *M*. *tuberculosis.*

FR 2 126 108 and Arnoux et al. (Journal of the American Chemical Society 102(10), 1980, 3605) describe sequanamycin (A), having the following formula:
(3S,4S,5R,7S,9S,10S,11R,12S,13R)-12-[(4,5-dihydroxy-4,6-dimethyltetrahydro-2*H*-pyran-2-yl)oxy]-7-hydroxy-2-{1-[(5-hydroxy-3,4-dimethoxy-6-methyltetrahydro-2*H-*pyran-2-yl)oxy]propan-2-yl}-10-[(3-hydroxy-6-methyl-4-oxotetrahydro-2*H*-pyran-2-yl)oxy]-3,5,7,9,11,13-hexamethyl-6,14-dioxooxacyclotetradecan-4-yl) 3-methylbutanoate.

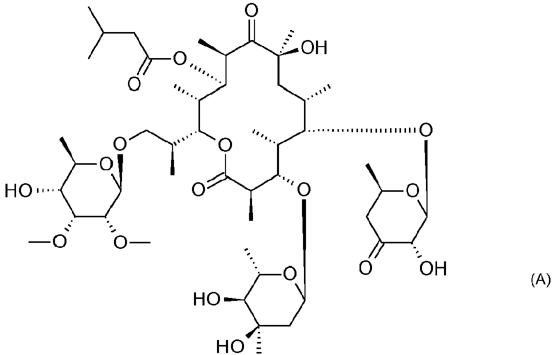

This compound is described therein as an antimicrobial agent and especially enables the treatment of tuberculosis. However, this compound may show instability, in particular in acidic or basic aqueous medium, and/or may also show metabolic instability, which makes it difficult to use as a drug.

It is therefore necessary to develop compounds with improved and/or more active pharmacokinetic properties, so as to enable their use as medicaments.

A subject of the present invention is in particular macrolide derivatives, which have bacteriostatic and/or bactericidal action, mainly on gram-positive microorganisms, and also on mycobacteria, especially against strains of sensitive *Mycobacterium* or *Corynebacterium* that are resistant to the first-line antibiotics, and the preparation and therapeutic uses thereof.

### [COMPOUNDS]

The present invention relates to compounds corresponding to formula (I): 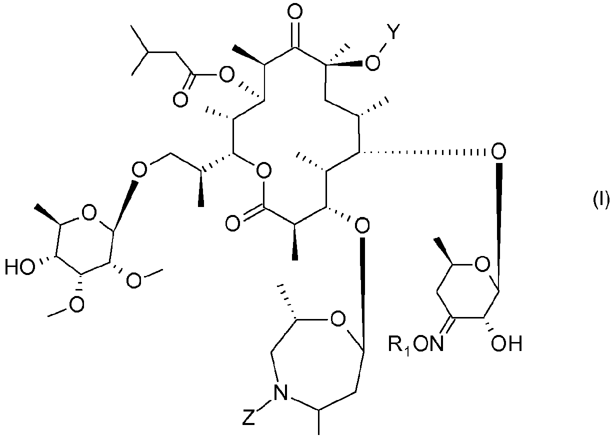 in which:
- Y represents a hydrogen atom, a group -(C=O)-NR₂R₃ or a group -(C=O)-O-R₁₈;
- Z represents:
   - a hydrogen atom,
   - a group -C₁₋₆-alkyl, which is unsubstituted or substituted with one or more groups R₄,
   - a group -C₃₋₇-cycloalkyl, which is unsubstituted or substituted with a group -NH-(C=O)-R₁₉ or with a group -NH-SO₂-R₂₀,
   - a group -C₃₋₆-heterocycloalkyl,
   - a group -NH-(C=O)-R₅;
- R₁ represents a hydrogen atom, a group -C₂₋₆-alkenyl, a group -C₂₋₆-alkynyl or a group -C₁₋₆-alkyl which is unsubstituted or substituted with a group -C₁₋₄-fluoroalkyl or with a heteroaryl group which is unsubstituted or substituted with a group 3-(3-fluorophenyl)-2-oxo-1,3-oxazolidin-5-ylmethyl;
- R₂ represents a hydrogen atom or a group -C₁₋₆-alkyl;
- R₃ represents:
   - a group -C₃₋₇-cycloalkyl, which is unsubstituted or substituted with a group -C₁₋₃-alkyl substituted with a group -NH-SO₂-R₂₁,
   - a heteroaryl group,
   - a linear or branched group -C₁₋₆-alkyl, which is unsubstituted or substituted with a group chosen from:
      - a group -NH-R₆,
      - a group -NH-SO₂-R₇,
      - a group -NH-(C=O)-R₈,
      - a group -C₃₋₇-cycloalkyl, which is unsubstituted or substituted with a group -C₃₋₆-heterocycloalkyl,
      - a group -C₃₋₆-heterocycloalkyl,
      - an aryl group, which is unsubstituted or substituted with one or more groups chosen independently from a halogen atom and a group -C₁₋₄-fluoroalkyl,
      - a heteroaryl group, which is unsubstituted or substituted with a group -C₁₋₃-alkyl, a group -C₁₋₄-alkoxy, a group -C₁₋₄-fluoroalkyl or a group -C₃₋₆-heterocycloalkyl,
      - or alternatively with one or more groups -C₁₋₄-alkoxy;
- or alternatively R₂ and R₃, together with the nitrogen atom to which they are attached, constitute a group -C₃₋₆-heterocycloalkyl chosen from: aziridine, azetidine, pyrrolidine, piperidine, morpholine, thiomorpholine or piperazine; the said heterocycloalkyl group being unsubstituted or substituted with a heteroaryl group, the said heteroaryl group being unsubstituted or substituted with a group -C₁₋₄-fluoroalkyl;
- R₄ independently represents a group chosen from:
   - a hydroxyl group,
   - a deuterium,
   - a halogen atom,
   - a group -C₃₋₇-cycloalkyl,
   - an aryl group, which is unsubstituted or substituted with one or more groups -Rg,
   - a heteroaryl group,
   - a group -C₃₋₆-heterocycloalkyl,
   - a group -C₁₋₄-alkoxy,
   - a group -(C=O)-NH-R₁₀,
   - a group -NH-R₁₁,
   - a group -NH-(C=O)-R₁₂,
   - or a group -NH(SO₂)-R₁₃;
- R₅ represents a heteroaryl group;
- R₆ represents a heteroaryl group, which is unsubstituted or substituted with one or more halogen atoms;
- R₇ represents a group -C₁₋₄-fluoroalkyl, an aryl group or a heteroaryl group, the said aryl and heteroaryl groups being unsubstituted or substituted with one or more groups R_{1'};
- R₈ represents a heteroaryl group, which is unsubstituted or substituted with one or more groups R_{2'};
- R₉ represents a halogen atom, a group -C₁₋₄-alkoxy, a formyl group (CHO) or a group -C₁₋₄-alkyl, which is unsubstituted or substituted with a hydroxyl group;
- R₁₀ represents a heteroaryl group, which is unsubstituted or substituted with a group -C₁₋₃-alkyl;
- R₁₁ represents:
   - a group -C₃₋₁₀-heterocycloalkyl, which is unsubstituted or substituted with one or more oxide groups,
   - a heteroaryl group or an aryl-C₁₋₄-alkyl group, the said heteroaryl or aryl groups being unsubstituted or substituted with one or more groups independently chosen from a halogen atom, a hydroxyl group, a nitro group and a group -C₁₋₃-alkyl;
- R₁₂ represents:
   - a group -C₁₋₄-alkoxy,
   - a group -C₁₋₄-alkyl, which is unsubstituted or substituted with a group -NR₁₄R₁₅ or with a heteroaryl group, the said heteroaryl group being unsubstituted or substituted with a group -C₁₋₃-alkyl,
   - a heteroaryl group, which is unsubstituted or substituted with one or more groups chosen from a hydroxyl group and a group -C₁₋₃-alkyl;
- R₁₃ represents:
   - a group -C₁₋₄-alkyl,
   - a group -C₁₋₄-fluoroalkyl,
   - an aryl group, which is unsubstituted or substituted with a nitro group,
   - or a heteroaryl group, which is unsubstituted or substituted with a group -NR₁₆R₁₇;
- R₁₄, R₁₅, R₁₆ and R₁₇ each independently represent:
   - a hydrogen atom,
   - or a group -C₁₋₄-alkyl;
- R₁₈ represents a group -C₁₋₄-alkyl or a benzyl group;
- R₁₉ represents an aryl group or a heteroaryl group;
- R₂₀ represents a group -C₁₋₄-alkyl or an aryl group;
- R₂₁ represents an aryl group;
- R_{1'} represents:
   - a halogen atom,
   - a group -C₁₋₄-alkoxy,
   - a group -C₁₋₄-fluoroalkyl,
   - a group -OCF₃,
   - a nitro group,
   - a group -NH₂,
   - a group -NHCH₃;
- R_{2'} represents:
   - a hydroxyl group,
   - a group -C₁₋₆-alkyl.

The compounds of general formula (I) may comprise one or more asymmetric carbons. They may therefore exist in the form of enantiomers or diastereoisomers. These enantiomers, diastereoisomers, and also mixtures thereof, including racemic mixtures, form part of the invention.

The compounds of formula (I) may exist in the form of bases or acid-addition salts. Such addition salts form part of the invention.

These salts are advantageously prepared with pharmaceutically acceptable acids, but salts of other acids, for example for purifying or isolating the compounds of general formula (I), also form part of the invention.

The compounds of formula (I) according to the present invention also comprise those in which one or more hydrogen, carbon or halogen atoms, especially chlorine or fluorine atoms, have been replaced with their radioactive isotopes, for example deuterium or tritium to replace hydrogen or carbon-14 to replace carbon-12. Such labelled compounds are useful in research, metabolism or pharmacokinetic studies, and also in biological and pharmacological tests as tools.

In the context of the present invention:
- alkyl represents a saturated, linear or branched aliphatic group; for example, a group C₁₋₃-alkyl represents a linear or branched carbon-based chain of 1 to 3 carbon atoms, especially a methyl, ethyl, propyl or isopropyl. Similarly, a group C₁₋₄-alkyl represents a linear or branched carbon-based chain of 1 to 4 carbon atoms, especially a methyl, ethyl, propyl, isopropyl, butyl, isobutyl, sec-butyl or *tert*-butyl. Similarly, a group C₁₋₆-alkyl represents a linear or branched carbon-based chain of 1 to 6 carbon atoms, especially a methyl, ethyl, propyl, isopropyl, butyl, isobutyl, *sec*-butyl, *tert*-butyl, pentyl, isopentyl, neopentyl, *tert*-pentyl, hexyl or isohexyl.
- alkenyl represents a linear or branched hydrocarbon-based aliphatic group comprising at least one unsaturation in the form of a double bond, and comprising from 2 to 6 carbon atoms. Examples that may be mentioned include the vinyl and allyl groups.
- alkynyl represents a linear or branched hydrocarbon-based aliphatic group comprising at least one unsaturation in the form of a triple bond, and comprising from 2 to 6 carbon atoms. Examples that may be mentioned include the ethynyl and 2-propynyl groups.
- cycloalkyl represents a saturated cyclic aliphatic group comprising from 3 to 7 carbon atoms. Examples that may be mentioned include cyclopropyl, cyclobutyl, cyclopentyl, cyclohexyl and cycloheptyl groups.
- halogen represents a fluorine, chlorine, bromine or iodine atom.
- fluoroalkyl represents an alkyl group comprising from 1 to 4 carbon atoms, in which one or more hydrogen atoms are replaced with a fluorine atom. Examples of fluoroalkyl groups that may be mentioned include trifluoromethyl, difluoromethyl, 3,3,3-trifluoropropyl, 2,2,2-trifluoroethyl, 2,2-difluoroethyl, 2,2,3,3-tetrafluoropropyl, 1,1-difluoroethyl and 3,3,3-trifluoro-2-(trifluoromethyl)propyl.
- heterocycloalkyl represents a saturated or partially saturated, monocyclic or polycyclic, optionally substituted 3- to 9-membered ring including one or more heteroatoms such as nitrogen, oxygen or sulfur atoms. The sulfur atoms may be in the form of sulfoxide or sulfone. By way of example, a heterocycloalkyl may be a pyrrolidine, a morpholine, a piperazine, a diazetidine, a dihydropyrrolidine, a piperidine, an azepane, an imidazolidine, a thiomorpholine, a tetrahydropyran, a tetrahydrothiophene, a tetrahydrothiopyran, a diazepane or an azabicyclooctane, a tropane, a 3,6-diazabicyclo[3.1.0]hexane, a tetrahydrofuran, a 3,7-diazabicyclo[3.3.1]nonane or a tetrahydrothiophene 1,1-dioxide.
- aryl represents a monocyclic or polycyclic, optionally substituted aromatic system comprising from 6 to 14 carbon atoms. According to one embodiment of the invention, the aryl group comprises 6 to 10 carbon atoms. When the system is polycyclic, at least one of the rings is aromatic. Examples of aryl groups that may be mentioned include phenyl, naphthyl, indanyl, tetrahydronaphthyl, anthracenyl and azulenyl.
- heteroaryl represents a monocyclic or polycyclic, optionally substituted 5- to 14-membered aromatic system. According to one embodiment of the invention, the heteroaryl is 5- to 10-membered and comprises one or more heteroatoms such as nitrogen, oxygen or sulfur atoms. When the system is polycyclic, at least one of the rings is aromatic. Examples of monocyclic heteroaryls that may be mentioned include thiazole, thiadiazole, thiophene, imidazole, triazole, tetrazole, pyridine, furan, oxazole, isoxazole, oxadiazole, pyrrole, pyrazole, pyrimidine, pyridazine and pyrazine. Examples of polycyclic heteroaryls that may be mentioned include indole, benzofuran, benzimidazole, benzothiophene, benzotriazole, benzothiazole, benzoxazole, quinoline, isoquinoline, indazole, quinazoline, phthalazine, quinoxaline, naphthyridine, 2,3-dihydro-1H-indole, 2,3-dihydrobenzofuran, tetrahydroquinoline, tetrahydroisoquinoline, tetrahydroisoquinazoline, furo[3,2-c]pyridine, 1*H*-pyrrolo[2,3-b]pyridine or tetrahydroquinazoline.
- alkoxy represents a group O-alkyl containing a saturated, linear or branched aliphatic chain comprising 1 to 4 carbon atoms. Examples of alkoxy groups that may be mentioned include methoxy and ethoxy.

According to the present invention, distinguished compounds are those of formula (I) in which Y represents a group -(C=O)-NR₂R₃, of formula: 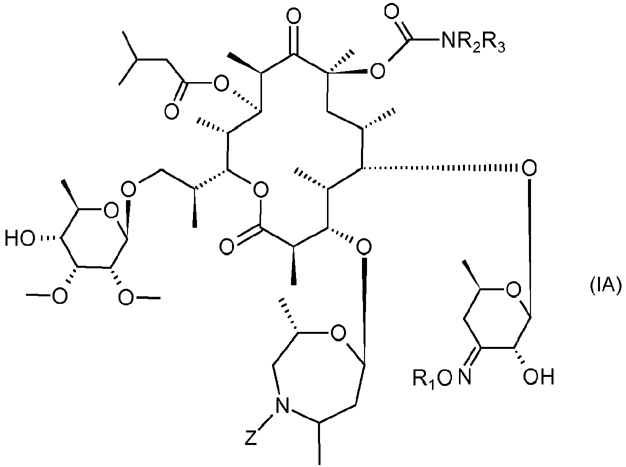 in which R₁, R₂, R₃ and Z are as defined for the compounds of formula (I); in the form of bases or of acid-addition salts.

According to the present invention, distinguished compounds are also those of formula (I) in which Y represents a hydrogen atom, of formula: 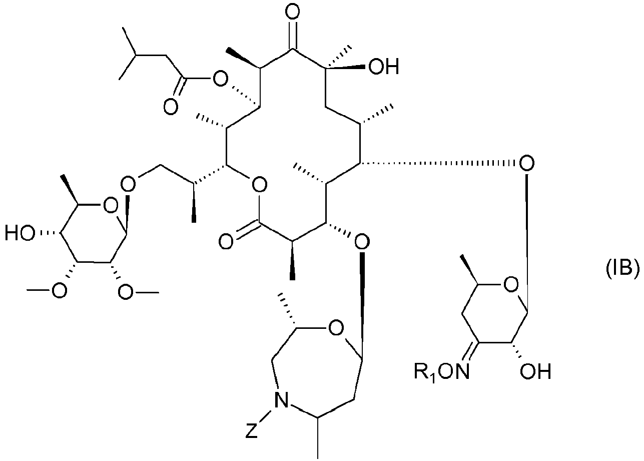 in which R₁ and Z are as defined for a compound of formula (I);
in the form of bases or of acid-addition salts.

According to a first variant of formula (IA), R₂ represents a hydrogen atom and R₃ represents a linear C₁₋₆-alkyl (Alk), which is unsubstituted or substituted with a group as defined for the compounds of formula (I), the compounds then having the formula (IC) below: 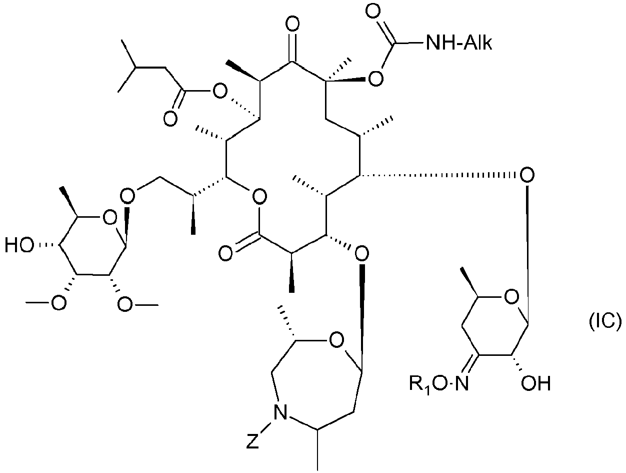 in which R₁ and Z are as defined for the compounds of formula (I);
in the form of bases or of acid-addition salts.

Within the compounds of formula (IC), distinguished compounds are those of formula (ID) below in which Alk represents a methyl substituted with a phenyl group: 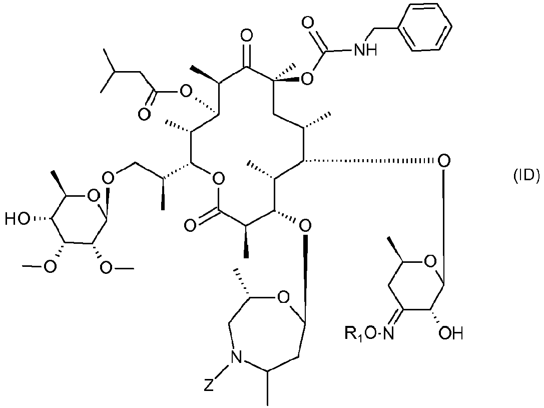 and R₁ and Z are as defined for a compound of formula (I);
in the form of bases or of acid-addition salts.

According to a second variant of formula (IA), distinguished compounds are those of formula (IE) in which R₂ represents a hydrogen atom and R₃ represents a branched C₁₋₆-alkyl (-C(CH₃)₂-Alk'), which is unsubstituted or substituted with a group as defined for the compounds of formula (I): 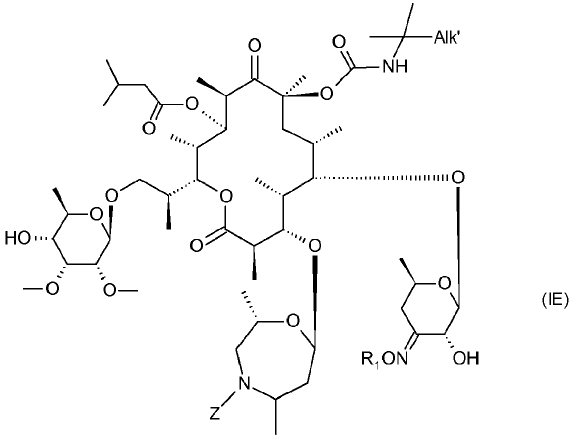 and R₁ and Z are as defined for the compounds of formula (I);
in the form of bases or of acid-addition salts.
Within the compounds of formula (IE), distinguished compounds are those of formula (IG) below in which Alk' represents a [(phenylsulfonyl)amino]methyl group: 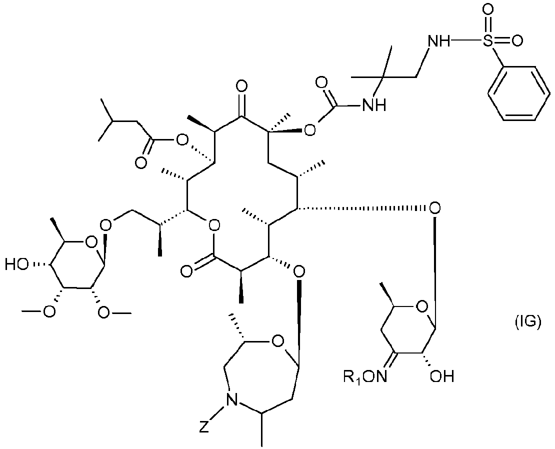 and R₁ and Z are as defined for the compounds of formula (I);
in the form of bases or of acid-addition salts.

According to a third variant of formula (IA), R₂ and R₃ represent an unsubstituted group -C₁₋₆-alkyl (Alk), the compounds then having the formula (IF) below: 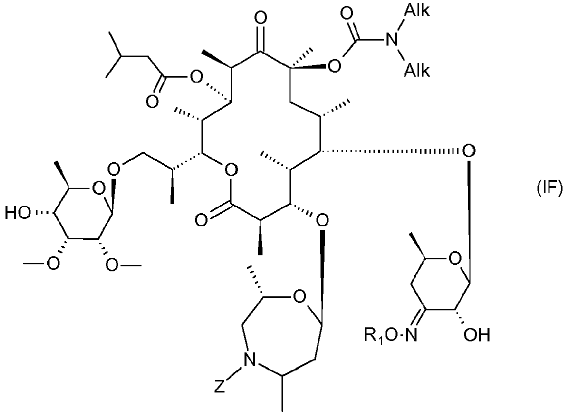 in which R₁ and Z are as defined for a compound of formula (I);
in the form of bases or of acid-addition salts.

According to a fourth variant of formula (IA), R₂ represents a hydrogen atom and R₃ represents an unsubstituted group -C₃₋₇-cycloalkyl (cycloAlk), the compounds then having the formula (IH) below: 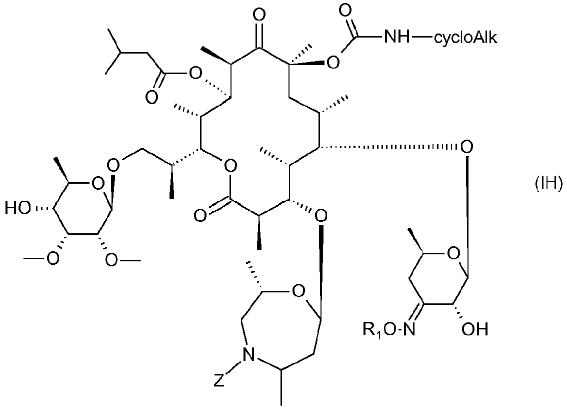 in which R₁ and Z are as defined for a compound of formula (I);
in the form of bases or of acid-addition salts.

According to the present invention, distinguished compounds are also those of formula (I) in which Y represents a group -(C=O)-OR₁₈, of formula: 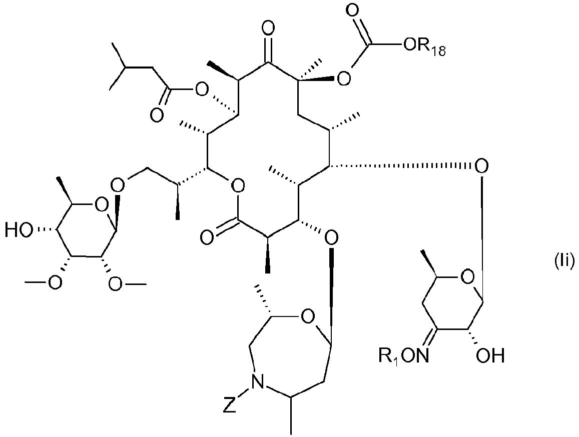 in which R₁, R₁₈ and Z are as defined for a compound of formula (I);
in the form of bases or of acid-addition salts.

According to the present invention, distinguished compounds are those of formula (I) in which:
- Y represents a hydrogen atom, a group -(C=O)-NR₂R₃ or a group -(C=O)-OMe;
- Z represents:
   - a hydrogen atom,
   - a group -C₁₋₆-alkyl, which is unsubstituted or substituted with one or more groups R₄,
   - a cyclopropyl group, a cyclobutyl group, a 3-(benzoylamino)cyclobutyl group, a 3-[(pyrazin-2-ylcarbonyl)amino]cyclobutyl group, a 3-[(methylsulfonyl)amino] cyclobutyl group, a 3-[(phenylsulfonyl)amino]cyclobutyl group, a cyclopentyl group, a cyclohexyl group,
   - a tetrahydro-2H-pyranyl group,
   - a group -NH-(C=O)-R₅;
- R₁ represents a hydrogen atom, an ethyl group, a 2,2,2-trifluoroethyl group or a methyl group, which is unsubstituted or substituted with a 1,2,3-triazole group substituted with a 3-(3-fluorophenyl)-2-oxo-1,3-oxazolidin-5-ylmethyl group;
- R₂ represents a hydrogen atom or a methyl group;
- R₃ represents:
   - a cyclohexyl group, a 1-{[(phenylsulfonyl)amino]methyl}cyclohexyl group or a 1-{[(phenylsulfonyl)amino]methyl}cyclopentyl group,
   - a 5,6,7,8-tetrahydroquinolin-5-yl group,
   - or a linear or branched group C₁₋₄-alkyl, which is unsubstituted or substituted with a group chosen from:
      - -NH-R₆,
      - -NH-SO₂-R₇,
      - -NH-(C=O)-R₈,
      - a 1-morpholin-4-ylcyclopentyl group,
      - a tetrahydro-2H-pyranyl group, a tetrahydrofuranyl group or a morpholin-4-yl group,
      - a phenyl group, which is unsubstituted or substituted with one or more groups chosen independently from a chlorine atom and a group -CF₃,
      - a 1 H-pyrrolo[2,3-b]pyridinyl group, a 4-methyl-5,6,7,8-tetrahydroquinazolin-2-yl group, a 6-methoxy-1H-benzimidazol-2-yl group, a pyridinyl group, which is unsubstituted or substituted with a group -CF₃ or with a morpholin-4-yl group,
      - or alternatively with one or more methoxy groups;
- or alternatively R₂ and R₃, together with the nitrogen atom to which they are attached, constitute a -C₃₋₆-heterocycloalkyl group chosen from: azetidine, morpholine, 4-[5-(trifluoromethyl)pyridin-2-yl]piperazine;
- R₄ independently represents a group chosen from:
   - a hydroxyl group,
   - a deuterium,
   - a fluorine atom,
   - a cyclopropyl group,
   - a phenyl group, which is unsubstituted or substituted with one or more groups chosen independently from a fluorine atom, a methoxy group, a -CH₂OH group and a -CHO group,
   - a pyridyl group,
   - a morpholinyl group, a tetrahydro-2H-pyranyl group,
   - a methoxy group,
   - a group -(C=O)-NH-R₁₀,
   - a group -NH-R₁₁,
   - a group -NH-(C=O)-R₁₂,
   - or a group -NH(SO₂)-R₁₃;
- R₅ represents a pyridyl group;
- R₆ represents a quinolyl group, the said quinolyl group being unsubstituted or substituted with a chlorine atom;
- R₇ represents a -CF₃ group, a phenyl, pyridyl, pyrazolyl, 1 H-pyrrolo[2,3-b]pyridyl or indolyl group, the said phenyl, pyridyl, pyrazolyl, 1 H-pyrrolo[2,3-b]pyridyl or indolyl groups being unsubstituted or substituted with one or more groups R_{1'};
- R₈ represents a pyrazinyl group, the said pyrazinyl group being unsubstituted or substituted with one or more groups R_{2'};
- R₁₀ represents a 1,8-naphthyridinyl group substituted with a methyl group;
- R₁₁ represents a tetrahydrothiophene-1,1-dioxide, quinolyl, pyridyl or benzyl group, the said quinolyl, pyridyl or benzyl groups being unsubstituted or substituted with a chlorine atom, a hydroxyl group, a nitro group or a methyl group;
- R₁₂ represents:
   - a tert-butoxy group,
   - a group -C₁₋₄-alkyl, which is unsubstituted or substituted with a group chosen from a group -NR₁₄R₁₅, pyridyl or pyrazolyl, the said pyridyl or pyrazolyl groups being unsubstituted or substituted with a methyl group,
   - a pyrazinyl or pyridyl, which is unsubstituted or substituted with one or more groups chosen from a hydroxyl group and a methyl group;
- R₁₃ represents:
   - a group -CF₃,
   - a phenyl group, which is unsubstituted or substituted with a nitro group,
   - or a pyridyl group, which is unsubstituted or substituted with a group -NR₁₆R₁₇;
- R₁₄, R₁₅, R₁₆ and R₁₇ each independently represent:
   - a hydrogen atom,
   - a methyl group or an isopropyl group;
- R_{1'} represents:
   - a fluorine atom, a chlorine atom,
   - a methoxy group,
   - a group -CF₃,
   - a group -OCF₃,
   - a nitro group,
   - a group -NH₂,
   - a group -NHCH₃;
- R_{2'} represents:
   - a hydroxyl group,
   - a methyl group;
in the form of bases or of acid-addition salts.

According to the present invention, distinguished compounds are those of formula (I) in which:
- Y represents a hydrogen atom or a group -(C=O)-NR₂R₃;
- Z represents:
   - a hydrogen atom,
   - a methyl group, an isopropyl group, a 2,2-dimethylpropyl group,
   - a group CD₃,
   - a 2-fluoroethyl group,
   - a cyclopropylmethyl group,
   - a 2-phenylethyl group,
   - a [(7-methyl-1,8-naphthyridin-2-yl)amino]-4-oxobutyl group,
   - a 2-{[(2-nitrophenyl)sulfonyl]amino}ethyl group,
   - a cyclopropyl group,
   - a tetrahydro-2H-pyranyl group;
- R₁ represents a hydrogen atom, an ethyl group, a 2,2,2-trifluoroethyl group or a methyl group;
- R₂ represents a hydrogen atom or a methyl group;
- R₃ represents:
   - a methyl group,
   - a 2-{[(2,6-difluorophenyl)sulfonyl]amino}-1,1-dimethylethyl group,
   - a 1,1-dimethyl-2-({[4-(trifluoromethyl)phenyl]sulfonyl}amino)ethyl group,
   - a 2-{[(2-fluorophenyl)sulfonyl]amino}-1,1-dimethylethyl group,
   - a 1,1-dimethyl-2-({[2-(trifluoromethoxy)phenyl]sulfonyl}amino)ethyl group,
   - a 1,1-dimethyl-2-({[4-(trifluoromethoxy)phenyl]sulfonyl}amino)ethyl group,
   - a 2-methyl-1-[(phenylsulfonyl)amino]propan-2-yl group,
   - a 2-methyl-1-{[(5-nitro-1H-pyrazol-4-yl)sulfonyl]amino}propan-2-yl group,
   - a 2-methyl-1-{[(trifluoromethyl)sulfonyl]amino}propan-2-yl group,
   - a 2-methyl-1-{[(2-nitrophenyl)sulfonyl]amino}propan-2-yl group,
   - a 1-{[(5-hydroxypyrazin-2-yl)carbonyl]amino}-2-methylpropan-2-yl group,
   - a 1,1-dimethyl-2-morpholin-4-ylethyl group,
   - a benzyl group,
   - a 2-(4-pyridyl)ethyl group;
in the form of bases or of acid-addition salts.

Among the compounds according to the invention, mention may be made especially of the compounds below:
- (2R,3S,4R,5R,7S,9S,10S,11R,12S,13R)-7-[(benzylcarbamoyl)oxy]-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5S,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-7-[(benzylcarbamoyl)oxy]-2-(1{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5R,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-12-{[(2S,7R)-4-cyclopropyl-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-7-{[(1-{[(5-hydroxypyrazin-2-yl)carbonyl]amino}-2-methylpropan-2-yl)carbamoyl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-7-[(benzylcarbamoyl)oxy]-12-{[(2S,5R,7R)-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-7-({[2-(pyridin-4-yl)ethyl]carbamoyl}oxy)-12-{[(2S,5S,7R)-2,4,5-trimethyl-1,4-oxazepan-7-yl]oxy}oxacyclotetradecan-4-yl3-methylbutanoate;
- (2R,3S,4R,5R,7S,9S,10S,11R,12S,13R)-7-[(benzylcarbamoyl)oxy]-12-{[(2S,7R)-4-cyclopropyl-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-7-[(benzylcarbamoyl)oxy]-12-{[(2S,7R)-2,5-dimethyl-4-(²H₃)methyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-7-[(dimethylcarbamoyl)oxy]-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5R,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-7-[(benzylcarbamoyl)oxy]-12-{[(2S,7R)-2,5-dimethyl-4-(2-{[(2-nitrophenyl)sulfonyl]amino}ethyl)-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-7-[(benzylcarbamoyl)oxy]-12-{[(2S,7R)-4-(2-fluoroethyl)-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-7-[(benzylcarbamoyl)oxy]-12-{[(2S,7R)-2,5-dimethyl-4-{4-[(7-methyl-1,8-naphthyridin-2-yl)amino]-4-oxobutyl}-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-7-[(benzylcarbamoyl)oxy]-12-{[(2S,7R)-4-(2,2-dimethylpropyl)-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-12-{[(2S,7R)-2,5-dimethyl-4-(2-phenylethyl)-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-7-{[(1-{[(5-hydroxypyrazin-2-yl)carbonyl]amino}-2-methylpropan-2-yl)carbamoyl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(5-nitro-1H-pyrazol-4-yl)sulfonyl]amino}propan-2-yl)carbamoyl]oxy}-6,14-dioxo-12-{[(2S,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-7-hydroxy-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5R,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(trifluoromethyl)sulfonyl]amino}propan-2-yl)carbamoyl]oxy}-6,14-dioxo-12-{[(2S,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-12-{[(2S,7R)-2,5-dimethyl-4-(2-phenylethyl)-1,4-oxazepan-7-yl]oxy}-7-hydroxy-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(2-nitrophenyl)sulfonyl]amino}propan-2-yl)carbamoyl]oxy}-6,14-dioxo-12-{[(2S,5R,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(2-nitrophenyl)sulfonyl]amino}propan-2-yl)carbamoyl]oxy}-6,14-dioxo-12-{[(2S,5S,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-[({2-methyl-1-[(phenylsulfonyl)amino]propan-2-yl}carbamoyl)oxy]-6,14-dioxo-12-{[(2S,5S,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-[({2-methyl-1-[(phenylsulfonyl)amino]propan-2-yl}carbamoyl)oxy]-6,14-dioxo-12-{[(2S,5R,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-7-[({1,1-dimethyl-2-[(phenylsulfonyl)amino]ethyl}carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-12-{[(2S,7R)-4-isopropyl-2,5-dimethyl-1,4-oxazepan-7-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-12-{[(2S,5R,7R)-4-(cyclopropylmethyl)-2,5-dimethyl-1,4-oxazepan-7-yl]oxy}-7-[({1,1-dimethyl-2-[(phenylsulfonyl)amino]ethyl}carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-12-{[(2S,7R)-4-(cyclopropylmethyl)-2,5-dimethyl-1,4-oxazepan-7-yl]oxy}-7-[({1,1-dimethyl-2-[(phenylsulfonyl)amino]ethyl} carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-7-[({1,1-dimethyl-2-[(phenylsulfonyl)amino] ethyl}carbamoyl)oxy]-12-{[(2S,7R)-2,5-dimethyl-4-(tetrahydro-2H-pyran-4-yl)-1,4-oxazepan-7-yl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-12-{[(2S,5S,7R)-4-(cyclopropylmethyl)-2,5-dimethyl-1,4-oxazepan-7-yl]oxy}-7-[({1,1-dimethyl-2-[(phenylsulfonyl)amino]ethyl} carbamoyl)oxy]-2-(2-{[(2R,3R,4R,5R,6R)-5-hydroxy-3,4-dimethoxy-6-methyltetrahydro -2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-7-[({1,1-dimethyl-2-[(phenylsulfonyl)amino]ethyl}carbamoyl)oxy]-12-{[(2S,5R,7R)-4-(2,2-dimethylpropyl)-2,5-dimethyl-1,4-oxazepan-7-yl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-7-[({1,1-dimethyl-2-[(phenylsulfonyl)amino]ethyl}carbamoyl)oxy]-12-{[(2S,7R)-4-(2,2-dimethylpropyl)-2,5-dimethyl-1,4-oxazepan-7-yl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
- (2R,3S,4R,5R,7S,9S,10S,11R,12S,13R)-7-{[(1,1-dimethyl-2-morpholin-4-ylethyl)carbamoyl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-7-{[(2-{[(2,6-difluorophenyl)sulfonyl]amino}-1,1-dimethylethyl)carbamoyl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-7-({[1,1-dimethyl-2-({[4-(trifluoromethyl) phenyl]sulfonyl}amino)ethyl]carbamoyl}oxy)-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy} oxacyclotetradecan-4-yl 3-methylbutanoate;
- (2R,3S,4R,5R,7S,9S,10S,11R,12S,13R)-7-{[(2-{[(2-fluorophenyl)sulfonyl]amino}-1,1-dimethylethyl)carbamoyl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-7-({[1,1-dimethyl-2-({[2-(trifluoromethoxy)phenyl]sulfonyl}amino)ethyl]carbamoyl}oxy)-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-7-({[1,1-dimethyl-2-({[4-(trifluoromethoxy)phenyl]sulfonyl}amino)ethyl]carbamoyl}oxy)-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-7-[({1,1-dimethyl-2-[(phenylsulfonyl)amino]ethyl}carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-({(2S,3R,6R)-3-hydroxy-6-methyl-4-[(2,2,2-trifluoroethoxy)imino]tetrahydro-2H-pyran-2-yl}oxy)-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
- (2R,3S,4R,5R,7S,9S,10S,11R,12S,13R)-7-[({1,1-dimethyl-2-[(phenylsulfonyl)amino] ethyl}carbamoyl)oxy]-10-{[(2S,3R,6R)-4-(ethoxyimino)-3-hydroxy-6-methyltetrahydro-2H-pyran-2-yl]oxy}-2-(2-{[(2R,3R,4R,5R,6R)-5-hydroxy-3,4-dimethoxy-6-methyltetrahydro-2H-pyran-2-yl]oxy}-1-methylethyl)-3,5,7,9,11,13-hexamethyl-6,14-dioxo-12-{[(2S,5R,7R)-2,4,5-trimethyl-1,4-oxazepan-7-yl]oxy}oxacyclotetradecan-4-yl 3-methylbutanoate;
in the form of bases or of acid-addition salts.

### [PREPARATION]

### [Nature of the strain]

The strain described in FR 2 126 108 deposited at the Northern Regional Research Laboratory (NRRL) under the number NRRL 3892 may be used.

The strain named *Allokutzneria albata* deposited at the Deutsche Sammlung Von Mikroorganismen und Zellkulturen GmbH (DSMZ) by the group Sanofi-Aventis (Sanofi Aventis Deutschland GmbH, Industriepark Höchst H831, 65926 Frankfurt am Main) under the identification reference ST108942 may also be used.

### [Fermentation and purification to isolate sequanamycin of formula (A)]

The fermentation and purification process described in FR 2 126 108 makes it possible to isolate sequanamycin of formula (A) from the strain *Allokutzneria albata.* This may be performed by application of the protocol below. This protocol is given as a nonlimiting illustration: it may be adapted to other conditions.

Thus, the fermentation process described below was performed for 500 litres, but may be adapted for smaller or larger proportions.

The preculture medium (named "medium 5294") used is typically the following:

| **Component** | **g/L** |
|---|---|
| Glucose | 4 |
| Yeast extract | 4 |
| Malt extract | 10 |
| CaCO₃ | 2 |

The pH of the medium before sterilization is 7.2.

The main culture medium (named "medium 5254-Seq01") used is typically the following:

| **Component** | **g/L** |
|---|---|
| Glucose | 15 |
| Soybean meal | 10 |
| Corn maceration water | 3 |
| CaCO₃ | 1 |
| NaCl | 5 |

The fermentation process is typically as follows:
1 vial from the Banque de Cellules de Travail (BCT) ↓
   Step 1: Preculture 1 500 µL of BCT were placed in a 300 ml conical flask comprising twice 100 ml of medium 5294. The mixture was stirred for 96 hours at 28°C. ↓
   Step 2: Preculture 2 25 ml of the culture medium from step 1 were placed in 4 times 500 ml of medium 5294 in a 2-litre conical flask, and the mixture was then stirred for 72 hours at 28°C. ↓
   Step 3: Preculture 3 1.5 L of the culture medium from step 2 were placed in 30 litres of medium 5294 in a 42-litre bioreactor, and the mixture was then stirred and aerated for 24 hours at 28°C, without monitoring the pH. ↓
   Step 4 (main culturing): 30 kg of the culture medium from step 3 were placed in 500 litres of medium 5294-Seq1 in an 800-litre bioreactor, and the mixture was then stirred and aerated for 96±5 hours at 28°C, without monitoring the pH. ↓ Harvesting

The fermentation process described above was performed for 500 litres, but may be adapted for smaller or larger proportions. It was performed, for example, at a scale of 7000 litres as follows, using the same culture media:
Preculture 1 = 250 ml, inoculum: one vial of BCT.
Preculture 2 = 5 litres in flasks (2 × 2.5 litres), inoculum of 0.5% from preculture 1.
Preculture 3 = 400 litres of medium in a 600 litre bioreactor, seeding rate of 1.25% from
preculture 2.
Main culture = 7000 litres of medium in a 10 000 litre bioreactor, seeding rate of 5.7% from preculture 3.

The fermentation process is followed by the purification process below (performed on the 500 litre fermentation broth described above).

Once the fermentation was complete, the fermentation broth was separated into culture supernatant and mycelium using a cylindrical seed grader. The separation led to about 440 litres of culture supernatant.

In separate batches, 100-120 litres of culture supernatant comprising, inter alia, the macrolide (sequanamycin (A)) were placed on a column filled with adsorption resin (glass column filled with styrene-divinylbenzene copolymer, inside diameter of 200, length of about 180 mm, flow rate of 250 ml/min). The resin was then washed with 30% 2-propanol.

The sequanamycin (A) was isolated by eluting the column with the following elution gradient: 30-70% B over 45 minutes, 70% B over 10 minutes, 100% B over 25 min; with A = H₂O, B = 2-propanol, modifier: 1 vol% NH₄Ac 50 g/L adjusted to pH 7).

The fractions comprising the sequanamycin (A) were combined and the 2-propanol was evaporated off. The pH of the solution obtained was adjusted to above 7.5 and the solution was then extracted twice with EtOAc. The organic phases were combined and the solvents were evaporated off. The oil obtained (about 10 g per 100 litres of culture supernatant) was purified on silica gel (column of 40 mm × 260 mm), the column being eluted with an n-heptane to 30/70 n-heptane/EtOAc gradient over 45 minutes, followed by 30/70 n-heptane/EtOAc maintained for about 40 minutes (with a flow of 100 ml/minute). The monitoring of the purification may be performed by thin-layer chromatography, eluting with EtOAc and revealing the sequanamycins (in the form of blue spots) with a reagent such as vanillin.

According to the concentration of sequanamycin (A) in the individual 100 litre batches, about 2.5 to 3.5 g of sequanamycin (A) with a purity of 68-75% (determined by NMR) were obtained per batch.

If a higher purity is required, the sequanamycin (A) obtained may be repurified by reverse-phase chromatography on a WatersAtlantis machine with a 50×100 mm, 5 µ column. An elution gradient of H₂O (A) and acetonitrile (B) and 1 vol% NH₄Ac 50 g/L adjusted to pH 7 was used (40-60% B over 30 minutes, flow rate of 140 ml/min). The chromatography was monitored by a light-scattering electrical signal. The fractions comprising the sequanamycin (A) were combined and lyophilized after having evaporated off the acetonitrile. The sequanamycin (A) yield after this final purification step was 57%, with an 85% pure compound according to the NMR analyses.

The compounds of formula (I) according to the invention are prepared from sequanamycin of formula (A).

### [Processes for preparing the compounds of formula (I) from sequanamycin of formula (A)]

In the steps described below, the usual organic chemistry reactions may be followed, especially those described in *"*Comprehensive Organic Transformations: A Guide to Functional Group Preparations" by Richard C. Larock, published by John Wiley & Sons Inc.

In the text hereinbelow, the term "protecting group PG" means a group that can, firstly, protect a reactive function such as a hydroxyl or an amine during the synthesis and, secondly, regenerate the intact reactive function at the end of the synthesis. Examples of protecting groups and also protection and deprotection methods are given in Protective Groups in Organic Synthesis, Greene et al., 4th Edition (John Wiley & Sons, Inc., New York), 2007.
In the text hereinbelow, the term "leaving group LG" means a group that can be readily cleaved from a molecule by breaking a heterolytic bond, with loss of an electron pair. This group may thus be readily replaced with another group, for example during a substitution reaction. Such leaving groups are, for example, halogens or an activated hydroxyl group such as a methanesulfonate, benzenesulfonate, p-toluenesulfonate, triflate, acetate, etc. Examples of leaving groups and also references for their preparation are given in Advanced Organic Chemistry, M.B. Smith and J. March, 6th Edition, Wiley Interscience, 2007, pp. 496-501.

In accordance with the invention, the compounds of formula (I) in which Y represents a group -(C=O)-NR₂R₃ may be prepared according to the process characterized in that: a compound of formula (I), in which Y represents a hydrogen atom below: 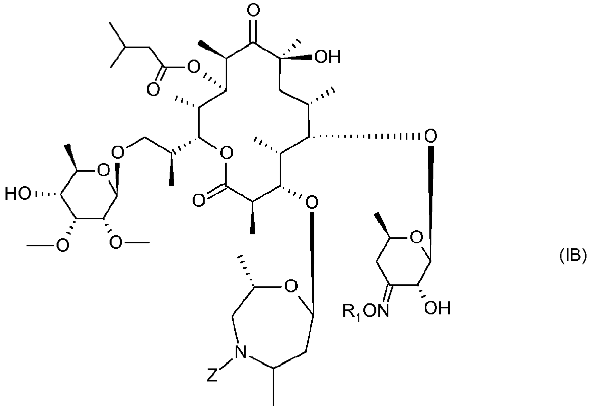 and R1 and Z are as defined for the compounds of formula (I), is reacted with a compound of formula (II) HNR2R3 in which R2 and R3 are as defined for the compounds of formula (I), in the presence of a carbonyl derivative and a base.

The introduction of a group Y representing a group -(C=O)-NR₂R₃ into the compounds of formula (IB) typically comprises the following four successive steps:
- a-1) protection of the hydroxyl functions of the compound of formula (IB),
- a-2) formation of a carbonyl intermediate from the hydroxyl function in position 7 of the macrocycle,
- a-3) reaction of the carbonyl intermediate with a compound of formula (II) HNR₂R₃,
- a-4) deprotection of the hydroxyl functions.

In step a-1), the hydroxyl functions of the compound of formula (IB) are protected to form a compound of formula (III) below, the hydroxyl function in position 7 of the macrocycle (onto which the group Y will be introduced) remaining free: 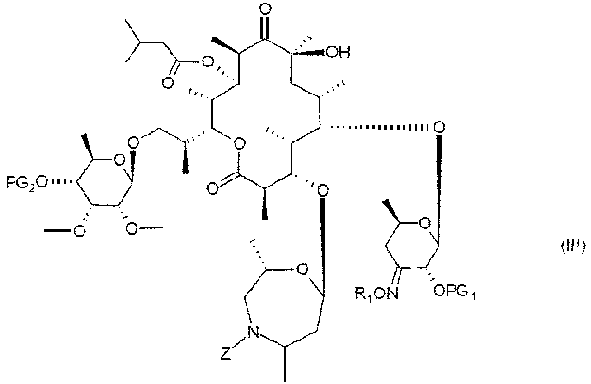 in which:
- R₁ and Z are as defined for the compounds of formula (I);
- PG₁ and PG₂ independently represent a hydroxyl-function protecting group.

In step a-2), the hydroxyl function in position 7 of the macrocycle of the compound of formula (III) is used to form a carbonyl intermediate of formula (IV) below: 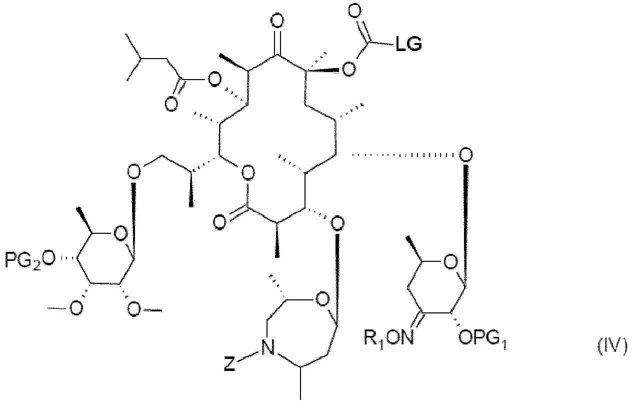 in which:
- R₁ and Z are as defined for the compounds of formula (I);
- LG represents a leaving group;
- PG₁ and PG₂ independently represent a hydroxyl-function protecting group.

In step a-3), the carbonyl intermediate of formula (IV) is reacted with a compound of formula (II) HNR₂R₃ in which R₂ and R₃ are as defined for the compounds of formula (I).

Step a-3) is typically performed in a polar solvent, for instance dimethylformamide (DMF), generally for 10 to 48 hours and at room temperature.

In step a-4), the hydroxyl functions of the compound obtained in step a-3) are deprotected.

Step a-4) is typically performed according to the deprotection processes described in Protective Groups in Organic Chemistry, J.F.W. McOmie, Plenum Press, 1973 or in Greene's Protective Groups in Organic Synthesis, by Theodora W. Greene published by John Wiley & Sons Inc., 2006.

As regards step a-1), the hydroxyl functions of compound (IB) are protected, for example, with acetate functions. This protection reaction may be performed by placing the compound of formula (IB) in contact with acetic anhydride in the presence of a base, especially a nitrogenous base, for example pyridine, at room temperature, the hydroxyl function in position 7 of the macrocycle onto which the group Y will be introduced remaining free, to form a compound of formula (IIIα) below: 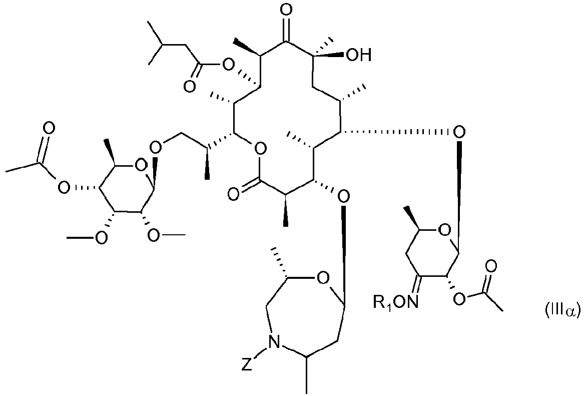 in which:
- R₁ and Z are as defined for the compounds of formula (I).

In a first embodiment of step a-2), a compound of formula (III) as defined above is reacted, for example, with 4-N,N-dimethylaminopyridine (DMAP) and trichloromethyl chloroformate, generally in the presence of a base, especially a nitrogenous base, for example pyridine, in an apolar aprotic solvent, for example dichloromethane, at a temperature between -20°C and room temperature and for a time of between 5 and 30 hours, to form two carbonyl intermediates of formulae (IVα) and (IV_{β}) below: 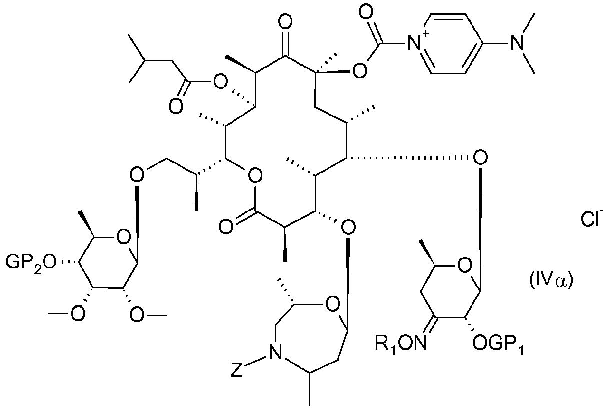 in which:
- R₁ and Z are as defined for the compounds of formula (I);
- PG₁ and PG₂ independently represent a hydroxyl-function protecting group; or 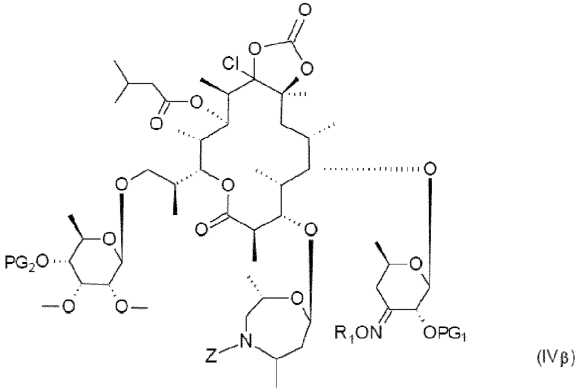 in which:
   - R₁ and Z are as defined for the compounds of formula (I);
   - PG₁ and PG₂ independently represent a hydroxyl-function protecting group, for example an acetate function.

In a second embodiment of step a-2), a compound of formula (III) is reacted, for example, with imidazole and diphosgene to form a carbonyl intermediate of formula (IVγ) below: 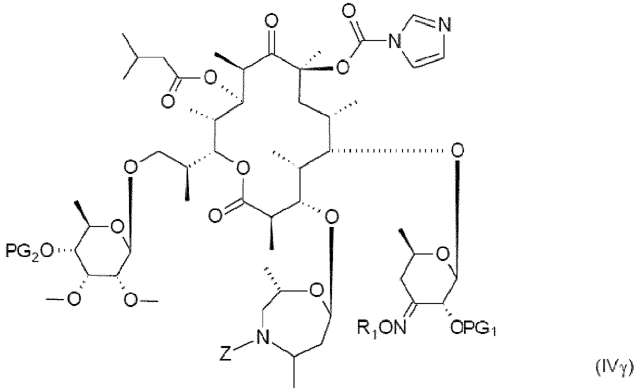 in which:
- R₁ and Z are as defined for the compounds of formula (I);
- PG₁ and PG₂ independently represent a hydroxyl-function protecting group, for example an acetate function.

In a third embodiment of step a-2), a compound of formula (III) is reacted, for example, with diphosgene to form a carbonyl intermediate of formula (IVδ) below: 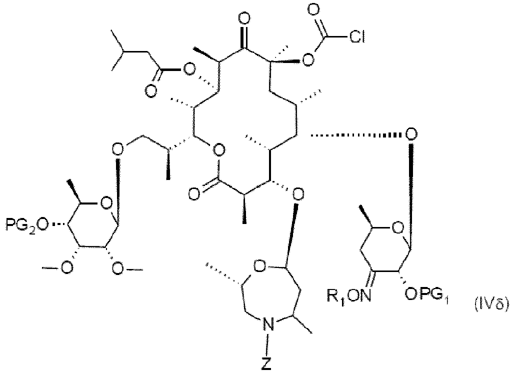 in which:
- R₁ and Z are as defined for the compounds of formula (I);
- PG₁ and PG₂ independently represent a hydroxyl-function protecting group, for example an acetate function.

In particular, steps a-1), a-2), a-3) and a-4) may be performed simultaneously or in reverse order. Thus, for example:
a'-1) the hydroxyl functions of the compound of formula (IB) are protected, and a carbonyl intermediate is formed from the hydroxyl function in position 7 of the macrocycle by microwave heating of the compound of formula (IB) with, for example, N,N'-carbonyldiimidazole, in a solvent, for instance cyclohexane, and at a temperature of between 80°C and 100°C, to obtain a compound of formula: 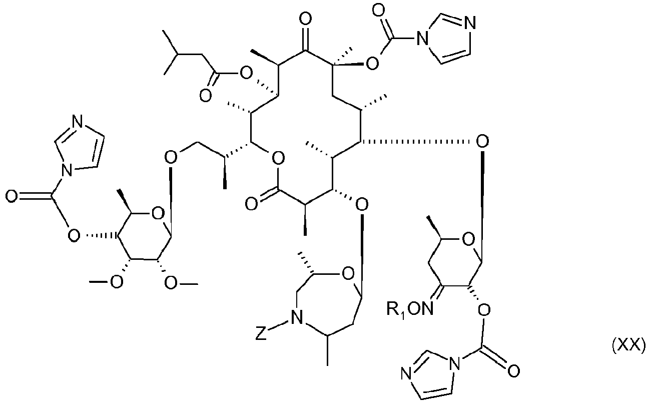 in which Z and R₁ are as defined for a compound of formula (I);
a'-2) the hydroxyl functions are deprotected by placing the compound of formula (XX) in contact with an acid, for instance hydrochloric acid, in a solvent, for instance tetrahydrofuran, to obtain a compound of formula: 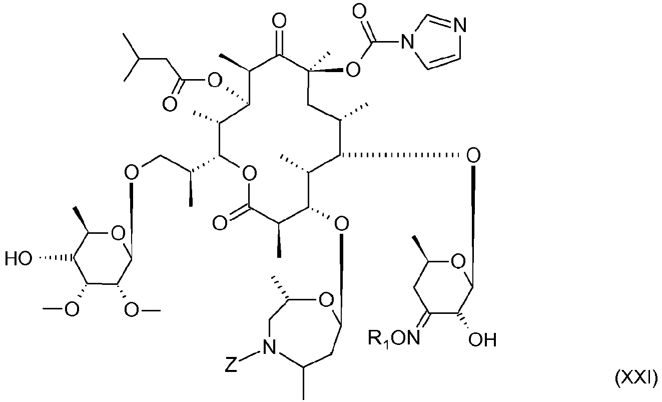 in which Z and R₁ are as defined for a compound of formula (I);
a'-3), the compound of formula (XXI) is reacted with a compound of formula (II) HNR₂R₃ in which R₂ and R₃ are as defined for the compounds of formula (I).

Step a'-3) is typically performed in a polar solvent, for instance dimethylformamide (DMF), in the presence of a base, for instance 1,8-diazabicyclo[5.4.0]undec-7-ene, generally for 10 to 48 hours and at room temperature.

In accordance with the invention, the compounds of formula (I) in which Y represents a group -(C=O)-NR₂R₃ may also be prepared according to the process characterized in that:
b-1) a compound of formula (V): 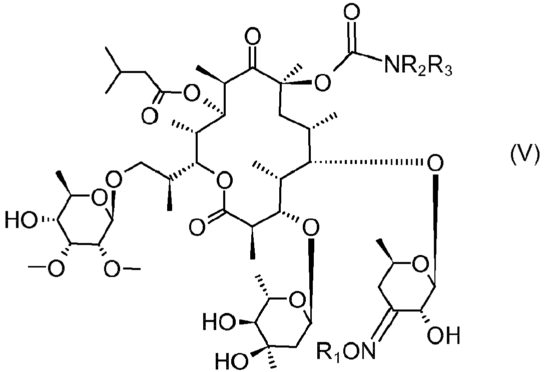 in which:
   - R₁, R₂ and R₃ are as defined for the compounds of formula (I); is reacted with an oxidizing agent to obtain a compound of formula (VI): 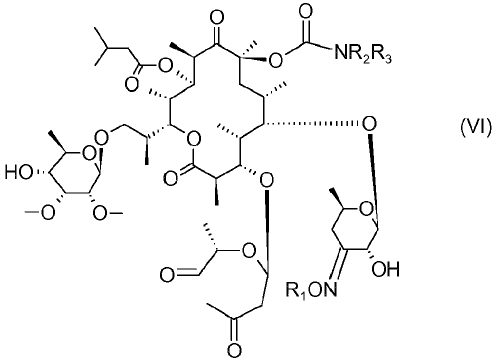 in which:
      R₁, R₂ and R₃ are as defined for the compounds of formula (I);
b-2) the compound of formula (VI) thus obtained is reacted with a compound of formula (VII):

   ZNH₂ (VII)

   in which Z is as defined for compound (I), in the presence of a reducing agent, to obtain the expected compound of formula (I).

In step b-1), the oxidation of the compound of formula (V) is performed via the action of an oxidizing agent, for instance sodium periodate, in a polar solvent, for instance MeOH, and at a temperature of between 0 and 10°C.

In step b-2), the reaction of the compound of formula (VI) with a compound of formula (VII) takes place in the presence of a reducing agent, for instance sodium cyanoborohydride, in a slightly acidic medium, in a solvent such as MeOH.

In accordance with the invention, the compounds of formula (I) in which Y represents a hydrogen atom may be prepared according to the process characterized in that:
c-1) a compound of formula (VIII): 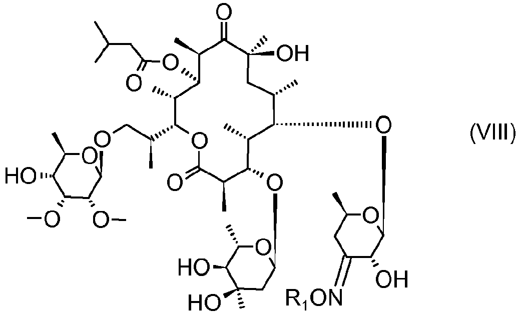 in which:
   - R₁ is as defined for the compounds of formula (I);
      is reacted with an oxidizing agent to obtain a compound of formula (IX): 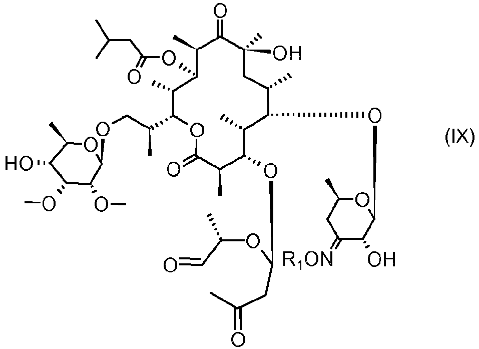 in which R₁ is as defined for the compounds of formula (I);
c-2) the compound of formula (IX) thus obtained is reacted with a compound of formula (VII):

   ZNH₂ (VII)

   in which Z is as defined for compound (I), in the presence of a reducing agent, to obtain the expected compound of formula (I).

Steps c-1) and c-2) are performed under the same operating conditions as those described in steps b-1) and b-2) above.

In accordance with the invention, the compounds of formula (I) in which Y represents a group -(C=O)-O-R₁₈ may be prepared according to the process characterized in that:
a compound of formula (XXI): 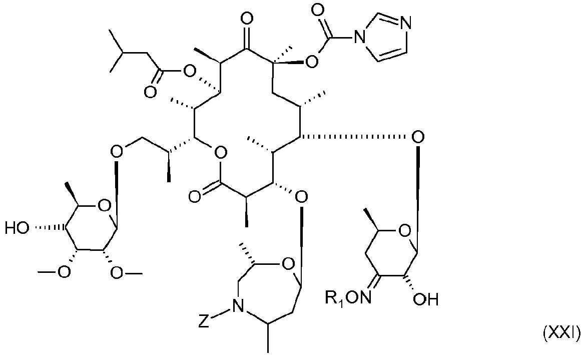 in which Z and R₁ are as defined for a compound of formula (I), is reacted with an alcohol of formula HO-R₁₈ (XXII), in the presence of a base.

The reaction is performed in the presence of a mineral base, for instance potassium carbonate, at room temperature.

In particular, certain compounds of formula (I) may be prepared from other compounds of formula (I). Thus, for example, a compound of formula (I) in which Z = Me may be prepared from a compound of formula (I) in which Z = H, by reaction with formaldehyde in the presence of formic acid and in a solvent, for instance chloroform.

The compounds of formula (I) thus obtained may be subsequently separated from the reaction medium and purified according to standard methods, for example by crystallization or chromatography.

The compounds of formula (I) thus obtained are isolated in the form of the free base or of a salt, according to the standard techniques.

The compounds of formula (II) are commercial, known or prepared according to methods known to those skilled in the art.

The compounds of formula (V) in which Y represents a group -(C=O)NR₂R₃ are prepared by reacting a compound of formula (VIII): 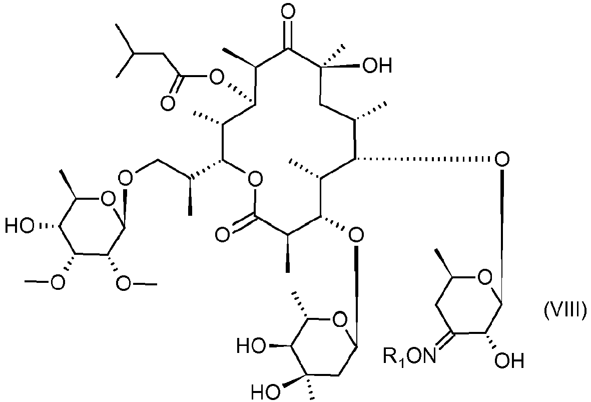 in which R₁ is as defined for the compounds of formula (I);
with a compound of formula (II) HNR₂R₃ in which R₂ and R₃ are as defined for the compounds of formula (I), in the presence of a carbonyl derivative, according to the four steps below:
- d-1) protection of the hydroxyl functions of the compound of formula (VIII),
- d-2) formation of an activated intermediate by activation of the hydroxyl function in position 7 of the macrocycle,
- d-3) reaction of the activated intermediate with a compound of formula (II) HNR₂R₃,
- d-4) optional deprotection of the hydroxyl functions.

In step d-1), the hydroxyl functions of the compound of formula (VIII) are protected to form a compound of formula (X) below (the hydroxyl function in position 7 of the macrocycle onto which the group Y will be introduced remaining free): 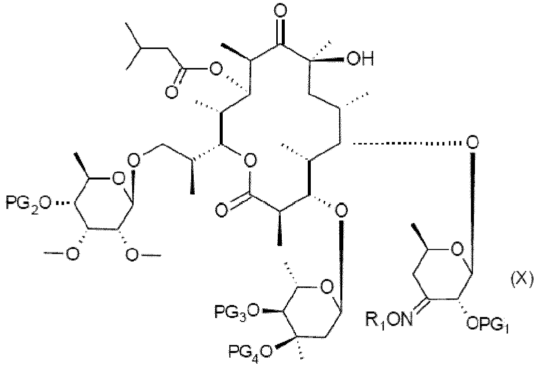 in which:
- R₁ is as defined for the compounds of formula (I);
- PG₁, PG₂, PG₃ and PG₄ independently represent a hydroxyl-function protecting group.

In step d-2), a carbonyl intermediate is formed from the hydroxyl function in position 7 of the macrocycle of the compound of formula (X), especially one or more of the carbonyl intermediates of formula (XI) below: 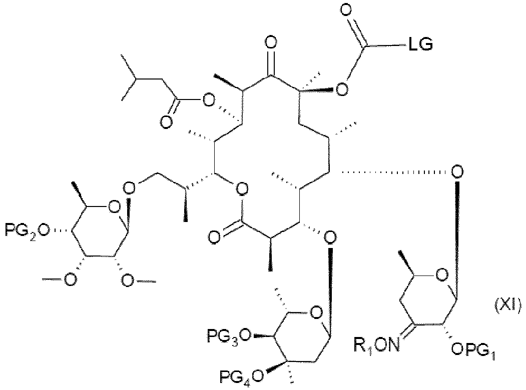 in which:
- R₁ is as defined for the compounds of formula (I);
- LG represents a leaving group;
- PG₁, PG₂PG₃ and PG₄ independently represent a hydroxyl-function protecting group.

In step d-3), the carbonyl intermediate obtained in step d-2) is reacted with a compound of formula (II) HNR₂R₃ in which R₂ and R₃ are as defined for the compounds of formula (I).

Step d-3) is typically performed in a polar solvent, for instance dimethylformamide (DMF), generally for 10 to 48 hours and at room temperature.

In step d-4), the hydroxyl functions of the compound obtained in step d-3) are deprotected.

Step d-4) is typically performed according to the deprotection processes described in Protective Groups in Organic Chemistry, J.F.W. McOmie, Plenum Press, 1973 or in Greene's Protective Groups in Organic Synthesis, by Theodora W. Greene published by John Wiley & Sons Inc., 2006.

In a first embodiment of step d-1), the hydroxyl functions of compound (VIII) are protected, for example, with acetate functions. This protection reaction may be performed by placing the compound of formula (VIII) in contact with acetic anhydride in the presence of a base, especially a nitrogenous base, for example pyridine, at a temperature typically ranging from room temperature to 160°C, the hydroxyl function in position 7 of the macrocycle onto which the group Y will be introduced remaining free, to form a compound of formula (Xα) below: 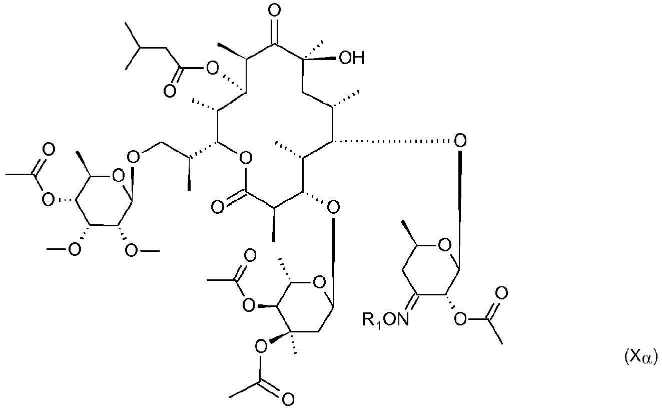 in which R₁ is as defined for the compounds of formula (I).

In a second embodiment, step d-1) typically comprises the following three successive steps d-1-1), d-1-2) and d-1-3):
- step d-1-1) the hydroxyl functions of the compound of formula (VIII) are protected with acyl imidazole functions to form a compound of formula (XII) by placing compound (VIII) in contact with 1,1'-carbonyldiimidazole in an apolar aprotic solvent, for example toluene, for a time from 10 minutes to 3 hours and at a temperature between room temperature and 80°C: 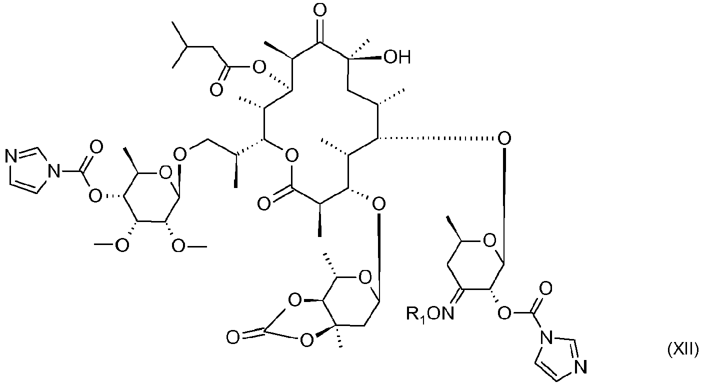 in which R₁ is as defined for the compounds of formula (I).

The tertiary alcohol of mycarose reacts with the acylimidazole of the secondary alcohol at α to form the carbonate.
- step d-1-2) the hydroxyl functions are deprotected by placing the compound of formula (XII) in contact with an acid, generally hydrochloric acid, in a polar aprotic solvent, for example tetrahydrofuran (THF), typically at room temperature and for a time from 2 to 24 hours, for example, to form the compound of formula (XIII) below: 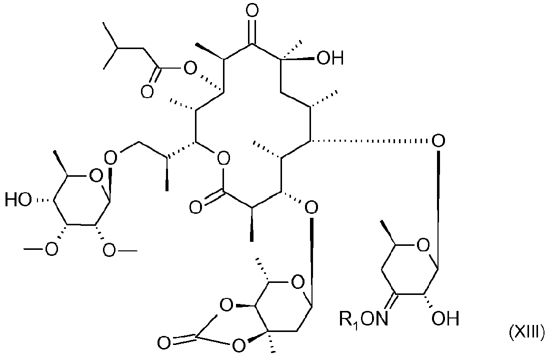 in which R₁ is as defined for the compounds of formula (I).
- step d-1-3) the secondary hydroxyl functions of the compound of formula (XIII) are protected with acetate functions by placing the said compound in contact with acetic anhydride in the presence of a base, especially a nitrogenous base, for example pyridine, typically at room temperature and for a time from 5 to 48 hours, for example: 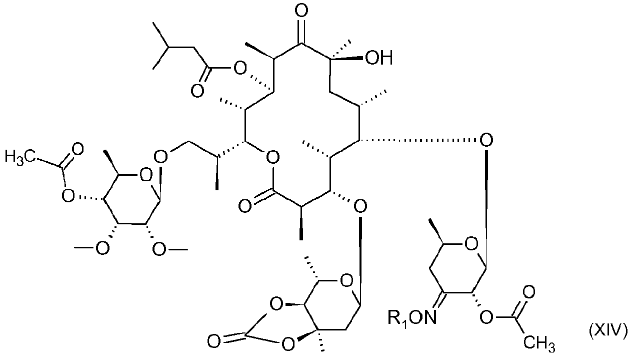 in which R₁ is as defined for the compounds of formula (I).

In a first embodiment of step d-2), a compound of formula (Xα) as defined above is reacted with 4-N,N-dimethylaminopyridine (DMAP) and trichloromethyl chloroformate, generally in the presence of a base, especially a nitrogenous base, for example pyridine, in an apolar aprotic solvent, for example dichloromethane, at a temperature between -20°C and 5°C for a time of between 30 minutes and 10 hours, and then at room temperature for a time of between 5 and 30 hours, to form two carbonyl intermediates of formulae (XIα) and (XIβ) below: 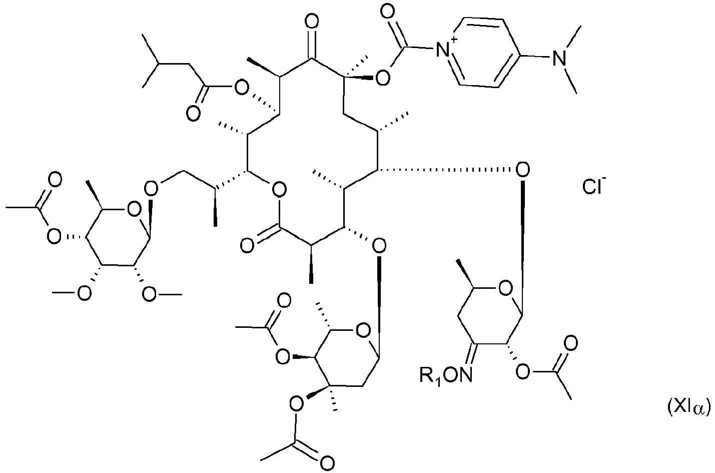 and 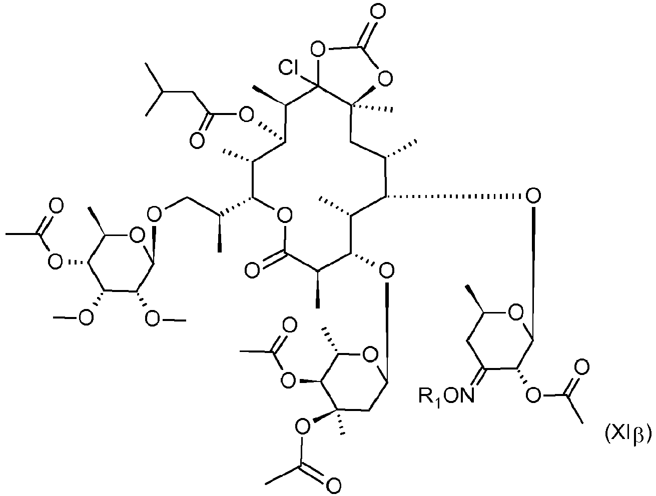 in which R₁ is as defined for the compounds of formula (I).

In a second embodiment of step d-2), the compound of formula (XIV) is reacted, for example, with DMAP and trichloromethyl chloroformate, generally in the presence of a base, especially a nitrogenous base, for example pyridine, to form two carbonyl intermediates of formulae (XV) and (XVI) below: 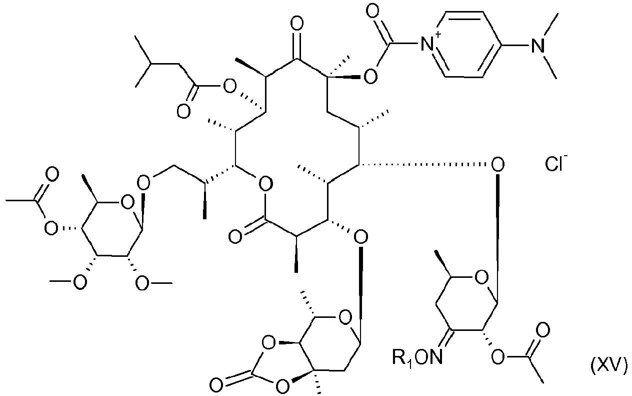 and 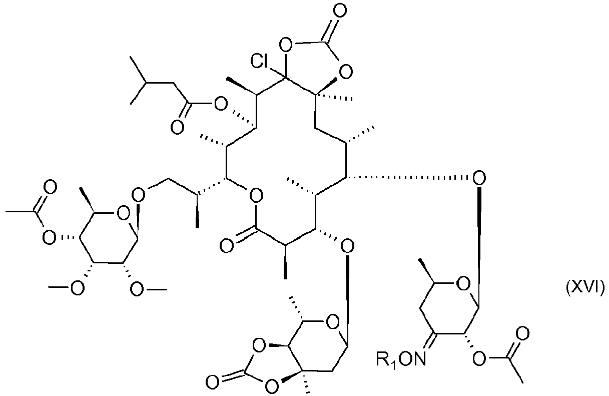 in which R₁ is as defined for the compounds of formula (I).

In a third embodiment of step d-2), a compound of formula (XIV) is reacted, for example, with 1,1-carbonyldiimidazole to form a carbonyl intermediate of formula (XVII) below: 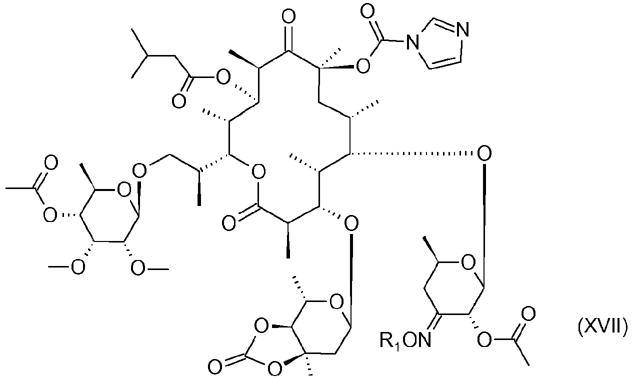 in which R₁ is as defined for the compounds of formula (I).

In a fourth embodiment of step d-2), a compound of formula (XIV) is reacted, for example, with diphosgene to form a carbonyl intermediate of formula (XVIII) below: 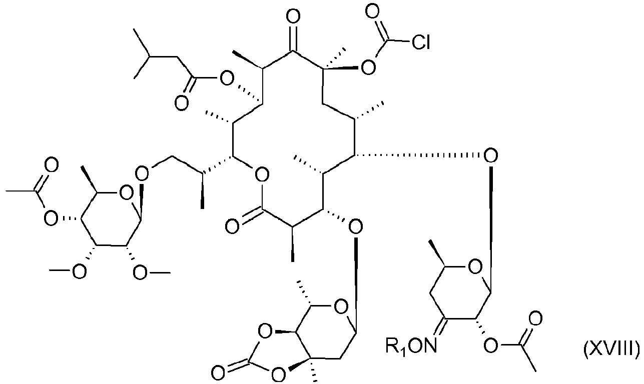 in which R₁ is as defined for the compounds of formula (I).

The compounds of formula (VII) are commercially available, known or prepared according to methods known to those skilled in the art, and may be in salt form, such as the hydrochloride.

The compounds of formula (VIII) are prepared by reacting the sequanamycins (A) with a compound of formula (XIX) H₂NOR₁ in which R₁ is as defined for the compounds of formula (I), in the presence of a base, for instance triethylamine, if necessary. The reaction is performed in a solvent, for instance methanol.

The compounds of formula (XIX) are commercially available, known or prepared according to methods known to those skilled in the art, and may be in salt form, such as the hydrochloride.

The compounds of formula (XXII) are commercially available, known or prepared according to methods known to those skilled in the art.

According to another of its aspects, a subject of the present invention is also the compounds of formulae (V) and (VIII). These compounds are useful as intermediates for synthesizing the compounds of formula (I).

Thus, a subject of the invention is compounds of formula (V): 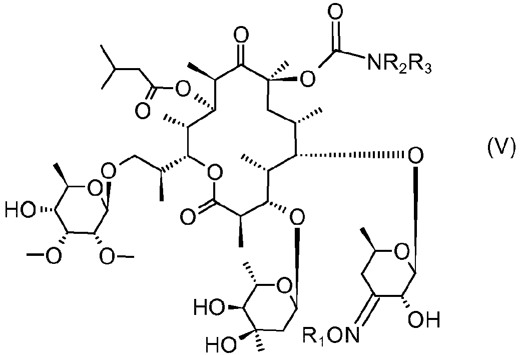 in which:
- R₁, R₂ and R₃ are as defined for the compounds of formula (I).
A subject of the invention is also compounds of formula (VIII): 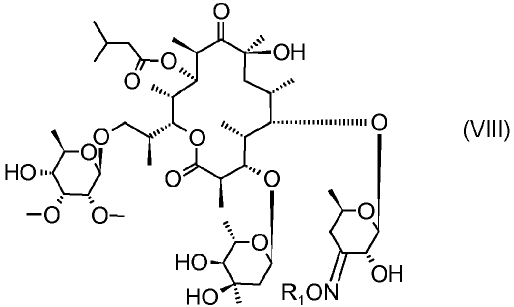 in which:
- R₁ is as defined for the compounds of formula (I).

### [Examples of preparation of the compounds of formula (I) from sequanamycin of formula (A)]

The following Examples describe the preparation of certain compounds in accordance with the invention. These examples are not limiting and merely illustrate the present invention.

In the Preparations and in the Examples, the following abbreviations are used:
EtOAc: ethyl acetate
TLC: thin-layer chromatography
CHCl₃: chloroform
DCM: dichloromethane
DMF: N,N-dimethylformamide
TEA: triethylamine
NalO₄: sodium metaperiodate, sodium periodate
K₂CO₃: potassium carbonate
MeOH: methanol
MgSO₄: magnesium sulfate
NaBH₃CN: sodium cyanoborohydride
NaCl: sodium chloride
NaHCO₃: sodium bicarbonate
Na₂SO₄: sodium sulfate
NH₄Cl: ammonium chloride
NH₄Ac: ammonium acetate
THF: tetrahydrofuran
RT: room temperature

### MATERIALS AND METHODS

The progress of the synthetic reactions is monitored by TLC. The plates are made of glass and are coated with Merck 60 F₂₅₄ silica gel. After elution, the plates are observed under ultraviolet light at 254 nm and then revealed by spraying with a 5M sulfuric acid/water solution followed by heating.

The microwave reactions were performed using a Biotage Initiator 8 EXP microwave machine.

The products were purified, when necessary, on a Biotage SP-1 chromatograph or a Spot 2 chromatograph from Merck. The columns used are Merck 15-40 µm silica columns (2.5 g to 400 g).

### Analyses

### Mass Spectrometry (MS):

### Method a:

- The spectra were acquired on a **Waters UPLC-SQD** machine;
- Ionization: electrospray in positive and/or negative mode (ES+/-);
- Chromatographic conditions:
   - Column: Acquity BEH C18 - 1.7 µm - 2.1 x 50 mm,
   - Solvents: A: H₂O (0.1% formic acid) B: CH₃CN (0.1% formic acid),
   - Column temperature: 50°C,
   - Flow rate: 1 ml/min,
   - Gradient (2 min): from 5% to 50% B over 0.8 min; 1.2 min: 100% B; 1.85 min: 100% B; 1.95: 5% B.

### Method b:

- The spectra were acquired on a **Waters UPLC-SQD** machine;
- Ionization: electrospray in positive and/or negative mode (ES+/-);
- Chromatographic conditions:
   - Column: Acquity BEH C18 - 1.7 µm - 2.1 x 50 mm,
   - Solvents: A: H₂O (0.1% formic acid) B: CH₃CN (0.1% formic acid),
   - Column temperature: 50°C,
   - Flow rate: 0.8 ml/min,
   - Gradient (2.5 min): from 5% to 100% B over 1.8 min; 2.40 min: 100% B; 2.45 min: 100% B; from 100% to 5% B over 0.05 min.

### Method c:

- The spectra were acquired on a **Waters ZQ** machine;
- Ionization: electrospray in positive and/or negative mode (ES+/-);
- Chromatographic conditions:
   - Column: XBridge C18- 2.5 µm - 3 x 50 mm,
   - Solvents: A: H₂O (0.1% formic acid) B: CH₃CN (0.1% formic acid),
   - Column temperature: 70°C,
   - Flow rate: 0.9 ml/min,
   - Gradient (7 min): from 5% to 100% B over 5.3 min; 5.5 min: 100% B; 6.3 min: 5% B.

### Method d:

- The spectra were acquired on a **Waters UPLC-SQD** machine;
- Ionization: electrospray in positive and/or negative mode (ES+/-);
- Chromatographic conditions:
   - Column: Acquity BEH C18 - 1.7 µm - 2.1 x 50 mm,
   - Solvents: A: H₂O (0.1% formic acid) B: CH₃CN (0.1% formic acid),
   - Column temperature: 50°C,
   - Flow rate: 1 ml/min,
   - Gradient (5 min): from 5% to 100% B over 4.2 min; 4.6 min: 100% B; 4.8 min: 5% B.

### Method e:

- The spectra were acquired on a **Waters ZQ** machine;
- Ionization: electrospray in positive and/or negative mode (ES+/-);
- Chromatographic conditions:
   - Xselect C18 column 3.5 µm - 3 x 50 mm,
   - Solvents: A: H₂O (0.1% formic acid) B: CH₃CN (0.1% formic acid),
   - Column temperature: 60°C,
   - Flow rate: 1 ml/min,
   - Gradient (7 min): from 10% to 100% B over 4.5 min; 4.85 min: 100% B; 6.5 min: 10% B.

### Method f:

- The spectra were acquired on an **Agilent 6110 or Shimadzu 2010** machine;
- Ionization: electrospray in positive and/or negative mode (ES+/-);
   - Xtimate C18 column 2.1 X 30 mm, 3 µm,
   - Solvents: A: H₂O (4L) + TFA (1.5 mL) B: CH₃CN (4L) + TFA (0.75 mL),
   - Column temperature: 50°C,
   - Flow rate: 1.2 ml/min,
   - Gradient (2 min): from 10% to 80% B over 0.9 min; 1.5 min: 80% B; 1.51 min: 10% B; 2 min: 10% B.

### Method g:

- The spectra were acquired on a **Shimadzu 2010** machine;
- Ionization: electrospray in positive and/or negative mode (ES+/-);
   - Merck RP-18e 2 X 25 mm column,
   - Solvents: A: H₂O (4L) + TFA (1.5 mL) B: CH₃CN (4L) + TFA (0.75 mL),
   - Column temperature: 50°C,
   - Flow rate: 1.0 ml/min from 0 to 0.08 min; 1.5 ml from 0.08 to 1.50 min,
   - Gradient (1.50 min): from 0 to 0.08 min 5% B; from 5% to 95% B from 0.08 to 0.7 min; 1.10 min: 95% B; 1.11: 5% B; 1.5 min: 5% B.

### Method h:

- The spectra were acquired on an **Agilent 6110** or **Shimadzu 2010** machine;
- Ionization: electrospray in positive and/or negative mode (ES+/-);
   - Xtimate C18 column 2.1 X 30 mm, 3 µm,
   - Solvents: A: H₂O (4L) + TFA (1.5 mL) B: CH₃CN (4L) + TFA (0.75 mL),
   - Column temperature: 50°C,
   - Flow rate: 1.2 ml/min,
   - Gradient (2 min): from 30% to 90% B over 0.9 min; 1.5 min: 90% B; 1.51 min: 30% B; 2 min: 30% B.

### Method i:

- The spectra were acquired on an **Agilent 6110** machine;
- Ionization: electrospray in positive and/or negative mode (ES+/-);
   - Columns A: Durashell C18 2.1 X 30 mm, 3 µm; B: Xbrige RP18 2.1 X 50 mm, 5 µm,
   - Solvents: A: H₂O (4L) + TFA (1.5 mL) B: CH₃CN (4L) + TFA (0.75 mL),
   - Column temperature: 50°C,
   - Flow rate: 1.2 ml/min,
   - Gradient (2 min): from 10% to 80% B over 0.9 min; 1.5 min: 80% B; 1.51 min: 10% B; 2 min: 10% B.

### 1H Nuclear magnetic resonance (NMR)

The 1 H NMR spectra were recorded on a Bruker Avance spectrometer (300 MHz, 400 MHz, 500 MHz or 600 MHz) in deuterated DMSO. The chemical shifts are expressed in units δ (ppm) using tetramethylsilane (TMS) as internal reference. For the interpretation of the spectra, the following abbreviations were used: s = singlet, d = doublet, t = triplet, q = quartet, quint = quintet, sext = sextet, dd = doubled doublet, ddd = doublet of doubled doublets, m = multiplet, ax. = axial, equat. = equatorial.

### PREPARATION

### Preparation of the intermediates for the examples described below:

### Preparation 1:

(2R,3S,4R,5R,7S,9S,10S,11R,12S,13R)-12-{[(2R,4R,5S,6S)-4,5-dihydroxy-4,6-dimethyltetrahydro-2*H*-pyran-2-yl]oxy}-7-hydroxy-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate.

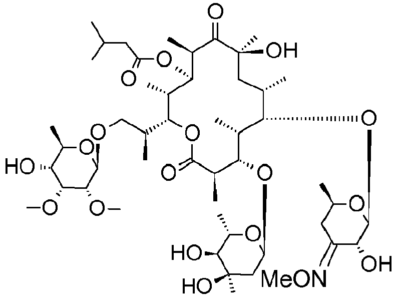

12 g of sequanamycin (A) are placed in 175 ml of MeOH with stirring, and 5.3 ml of TEA, and 3 g of methylhydroxylamine hydrochloride are then added, in this order. The stirring is continued at RT for 20 hours and the MeOH is then evaporated off under vacuum. The crude reaction product is taken up in 150 ml of DCM and washed with 100 ml of water and then with 100 ml of saturated aqueous NaCl solution. The aqueous phases are extracted with 150 ml of DCM. The organic phases are combined, dried over MgSO₄, filtered and concentrated under vacuum. 12.7 g of the product obtained are suspended in 70 ml of a petroleum ether (40-60°C)/isopropanol mixture (2/1). The mixture is heated to 70°C, the insoluble matter is filtered off while hot and the product is then left to precipitate out at RT over 20 hours. It is filtered off by suction and rinsed with 20 ml of a petroleum ether (40-60°C)/isopropanol mixture (2/1). The precipitate is dried under vacuum at 35°C to give 10.62 g of expected product.

### MS: method c

Retention time Tr (min) = 4.87; [M+Na]+: m/z 1014; [M-H+HCO₂H]- m/z 1036. **1 H NMR spectrum (500 MHz, in ppm, DMSO-d₆):** 0.81 (d, J=6.8 Hz, 3 H); 0.93 to 1.01 (m, 15 H); 1.07 (d, J=7.0 Hz, 3 H); 1.09 to 1.13 (m, 9 H); 1.17 (d, J=6.0 Hz, 3 H); 1.18 (d, J=6.0 Hz, 3 H); 1.24 (s, 3H); 1.44 (dd, J=10.8 and 14.4 Hz, 1 H); 1.68 to 1.76 (m, 2 H); 1.81 (d, J=14.4 Hz, 1 H); 1.88 (dd, J=11.5 and 15.9 Hz, 1 H); 1.96 to 2.06 (m, 3 H); 2.07 to 2.20 (m, 4 H); 2.73 (quint, J=7.0 Hz, 1 H); 2.81 (t, J=9.0 Hz, 1 H); 2.89 to 2.97 (m, 2 H); 3.03 (ddd, J=2.5 and 7.3 and 9.5 Hz, 1 H); 3.18 (q, J=6.8 Hz, 1 H); 3.34 to 3.36 (m, 2 H); 3.37 (s, 3 H); 3.45 (s, 3 H); 3.52 (dq, J=6.2 and 9.4 Hz, 1 H); 3.60 (s, 1 H); 3.62 to 3.65 (m, 1 H); 3.66 (t, J=2.5 Hz, 1 H); 3.71 to 3.77 (m, 1 H); 3.78 (m, 1 H); 3.80 (s, 3 H); 3.81 to 3.84 (m, 1 H); 3.87 (m, 1 H); 4.39 to 4.46 (m, 3 H); 4.50 (s, 1 H); 4.72 (d, J = 8.3 Hz, 1 H); 4.78 (d, J=8.3 Hz, 1 H); 4.84 (d, J=7.3 Hz, 1 H); 4.87 (d, J=3.8 Hz, 1 H); 5.19 (d, J=4.4 Hz, 1 H).

### Preparation 2: benzyl carbamate

(2R,3S,4R,5R,7S,9S,10S,11R,12S,13R)-7-[(benzylcarbamoyl)oxy]-12-{[(2R,4R,5S,6S)-4,5-dihydroxy-4,6-dimethyltetrahydro-2*H*-pyran-2-yl]oxy}-2-(1-{[(2R,3R,4R,5R,6R)-5-hydroxy-3,4-dimethoxy-6-methyltetrahydro-2*H*-pyran-2-yl]oxy}propan-2-yl)-10-{[(2S,3R,6R)-3-hydroxy-4-(methoxyimino)-6-methyltetrahydro-2*H-*pyran-2-yl]oxy}-3,5,7,9,11,13-hexamethyl-6,14-dioxooxacyclotetradecan-4-yl 3-methylbutanoate.

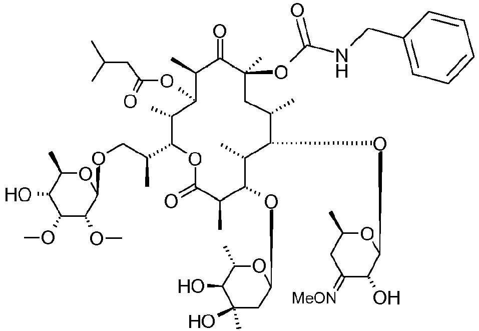

### Preparation 2.1:

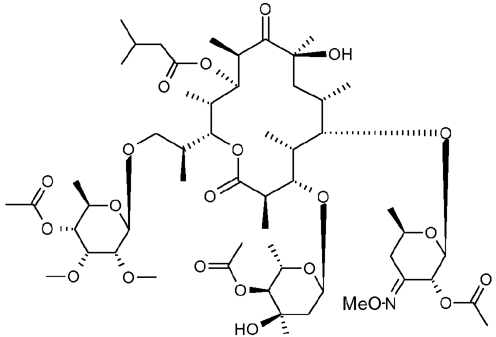

8.6 g of the compound obtained in Preparation 1 are placed in 86 ml of pyridine, and 8.27 ml of acetic anhydride are added. The mixture is stirred for 24 hours at room temperature, and the pyridine is then concentrated under vacuum. 150 ml of DCM are added and the resulting mixture is washed with 120 ml of 1 N HCl solution and then with 100 ml of saturated aqueous NaCl solution. The aqueous phases are extracted with 150 ml of DCM. The organic phases are combined, dried over Na₂SO₄, filtered and then evaporated to dryness. 9.75 g of the expected product are obtained.

### MS: method a

Retention time Tr (min) = 1.27; [M+Na]+: m/z 1140.

**1H NMR spectrum (400 MHz, in ppm, DMSO-d₆):** 0.81 (d, J=6.8 Hz, 3 H); 0.91 to 1.01 (m, 15 H); 1.02 (s, 3 H); 1.03 to 1.12 (m, 12 H); 1.21 (d, J=6.1 Hz, 3 H); 1.24 (s, 3 H); 1.50 (dd, J=10.5 and 14.5 Hz, 1H); 1.72 to 1.94 (m, 6 H); 1.96 to 2.10 (m, 8 H); 2.12 to 2.18 (m, 3 H); 2.22 (s, 3 H); 2.77 (m, 1 H); 3.02 (dd, J=2.7 and 8.1 Hz, 1 H); 3.08 (m, 1 H); 3.16 (q, J=7.1 Hz, 1 H); 3.36 (m, 1 H); 3.39 (s, 3 H); 3.41 (s, 3H); 3.45 (m, 1 H); 3.63 to 3.72 (m, 2 H); 3.76 (broad s, 4 H); 3.80 (m, 1 H); 3.85 (t, J=2.7 Hz, 1 H); 4.11 (m, 1 H); 4.17 (s, 1 H); 4.35 (dd, J=2.7 and 10.3 Hz, 1 H); 4.42 (m, 2 H); 4.52 (d, J=8.1 Hz, 1 H); 4.63 (d, J=7.3 Hz, 1 H); 4.69 (d, J=9.0 Hz, 1 H); 4.75 (d, J=9.0 Hz, 1 H); 4.93 (d, J = 4.0 Hz, 1 H); 5.00 (d, J=7.3 Hz, 1 H).

### Preparation 2.2:

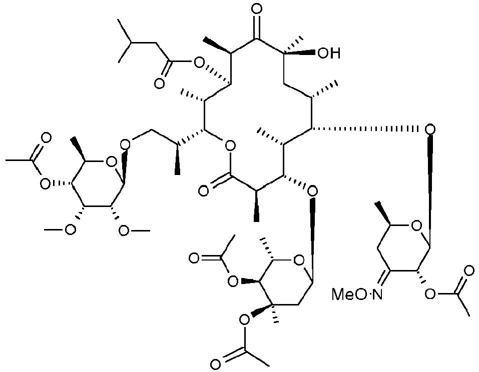

1 g of the compound obtained in Preparation 2.1 are placed in 20 ml of pyridine in a microwave reactor, and 1 ml of acetic anhydride and 50 mg of 4-dimethylaminopyridine are added. The solution is heated for 2 hours at 155°C by microwave. The reaction medium is poured into 50 ml of DCM and washed with 30 ml of 1 N HCl solution and then with 30 ml of water. The aqueous phases are extracted with twice 50 ml of DCM. The organic phases are combined, dried over Na₂SO₄, filtered and then evaporated to dryness under vacuum. The residue, 1.1 g of a brown foam, is purified by chromatography on a Merck cartridge (50 g of 15-40 µm silica), eluting with a 40/60 EtOAc/heptane mixture. 0.69 g of the expected compound is obtained.

### MS: method a

Retention time Tr (min) = 1.29; [M+Na]+: m/z 1182.

**1 H NMR spectrum (500 MHz, in ppm, DMSO-d₆):** 0.81 (d, J=6.9 Hz, 3 H); 0.91 to 1.02 (m, 18 H); 1.06 (m, 6 H); 1.10 (d, J=6.9 Hz, 3 H); 1.19 to 1.26 (m, 6 H); 1.35 (s, 3 H); 1.44 (dd, J=10.5 and 14.5 Hz, 1H); 1.79 (m, 1 H); 1.84 to 2.09 (m, 15 H); 2.11 to 2.18 (m, 6 H); 2.73 (m, 1 H); 3.02 (dd, J=2.7 and 7.8 Hz,1 H); 3.06 to 3.20 (m, 3 H); 3.35 to 3.42 (m, 8 H); 3.60 to 3.69 (m, 2 H); 3.77 (broad s, 4 H); 3.80 (m, 1 H); 3.85 (t, J=2.7 Hz, 1 H); 4.16 (m, 1 H); 4.36 (dd, J=2.7 and 9.9 Hz, 1 H); 4.48 to 4.53 (m, 3 H); 4.59 (d, J = 7.1 Hz, 1 H); 4.73 to 4.80 (m, 2 H); 4.87 (d, J=4.3 Hz, 1 H); 5.00 (d, J=7.1 Hz, 1 H).

### Preparation 2.3:

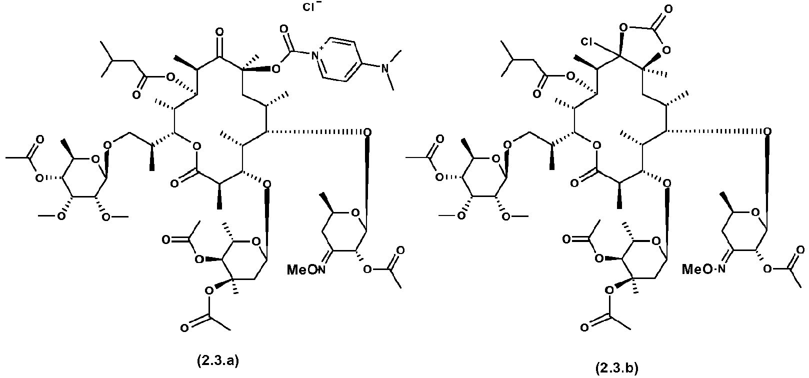

3.5 g of the compound obtained in Preparation 2.2 dissolved in 140 ml of DCM and 3.6 ml of pyridine are placed under argon with stirring. The yellow solution obtained is cooled to -10°C, followed by rapid addition of trichloromethyl chloroformate (diphosgene), and stirring is continued at -10°C for 3 hours. 0.368 g of 4-dimethylaminopyridine dissolved in 10 ml of DCM is then added. The reaction medium is maintained at -5°C for a further 30 minutes and is then allowed to warm to room temperature, and stirring is continued for 20 hours. The solvent is evaporated off and 150 ml of EtOAc are added to the crude reaction product. The mixture is stirred for 15 minutes at room temperature and the precipitate formed is then filtered off. It is rinsed with 70 ml of EtOAc and the filtrate is evaporated to dryness under vacuum. 3.92 g of a mixture of the expected compounds (structures **2.3.a** and **2.3.b**) are obtained. The mixture is used as obtained for the following stage.

### Preparation 2.4:

### a) Condensation of the amine (benzylamine)

1.5 g of the compound obtained in Preparation 2.3 are placed in 30 ml of DMF in a 100 ml round-bottomed flask, and 0.61 ml of benzylamine are then added. The mixture is stirred for 24 hours at room temperature, followed by addition of 100 g of ice and 100 ml of water. The precipitate formed is filtered off by suction and washed with a minimum amount of water. After drying in an oven under vacuum at 35°C, 1.18 g of the expected compound are obtained.

### b) Deprotection

1.18 g of the compound obtained in the preceding step are placed in 20 ml of MeOH, and 0.63 g of K₂CO₃ is added. The heterogeneous medium is stirred at room temperature for 3 hours and then filtered through a No. 4 sinter funnel. The filtrate is taken up in 100 ml of EtOAc and washed with saturated aqueous NaCl solution. The organic phase is dried over MgSO₄, filtered and then evaporated to dryness. 1.05 g of crude compound are obtained, which product is purified by chromatography on a Merck column (30 g of 15-40 µm silica) with a 30/70 to 60/40 EtOAc/heptane elution gradient. 0.466 g of the expected product is obtained.

### MS: method c

Retention time Tr (min) = 5.21; [M+H]+: m/z 1125; base peak: m/z 981 [M-H+HCO₂H]-: m/z 1169.

**1H NMR spectrum (500 MHz, in ppm, DMSO-d₆):** 0.80 (d, *J*=6.8 Hz, 3 H); 0.92 (d, *J*=6.8 Hz, 3 H); 0.96 to 1.02 (m, 9 H); 1.05 (m, 6 H); 1.08 to 1.15 (m, 9 H); 1.18 (m, 6 H); 1.67 to 2.18 (m, 10 H); 1.73 (s, 3 H); 2.18 (d, *J*=6.8 Hz, 2 H); 2.59 to 2.67 (m, 1 H); 2.80 (t, *J*=8.8 Hz, 1 H); 2.92 (dd, *J*=2.7 and 8.1 Hz, 1 H); 2.94 to 3.06 (m, 3 H); 3.27 to 3.35 (partially masked m, 1 H); 3.38 (s, 3 H); 3.41 (d, *J*=9.8 Hz, 1 H); 3.45 (s, 3 H); 3.49 to 3.56 (m, 1 H); 3.60 to 3.72 (m, 4 H); 3.80 (s, 3 H); 3.82 (m, 1 H); 3.87 (broad d, *J*=5.4 Hz, 1 H); 4.01 to 4.17 (m, 3 H); 4.34 to 4.39 (m, 2 H); 4.45 (d, *J*=7.8 Hz, 1 H); 4.50 to 4.57 (m, 2 H); 4.85 (d, *J*=7.3 Hz, 1 H); 4.93 (d, *J*=2.4 Hz, 1 H); 5.13 (broad s, 1 H); 7.18 to 7.36 (m, 6 H).

### Preparation 3:

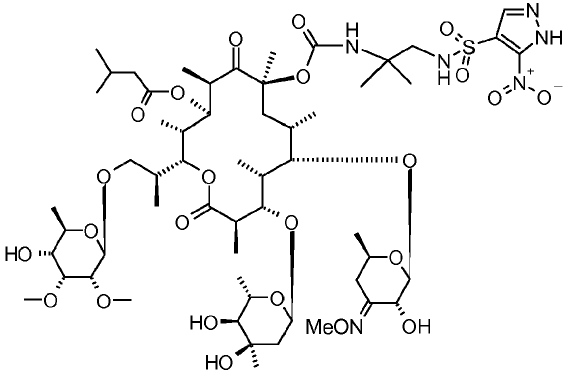

### Preparation 3.1:

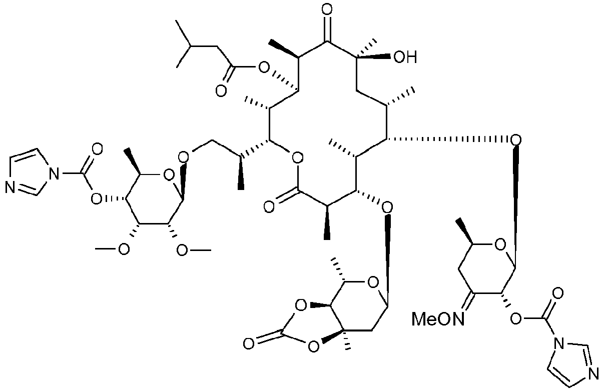

11.1 g of the compound prepared in Preparation 1 are placed in 220 ml of toluene. 9.07 g of 1,1'-carbonyldiimidazole are added and the reaction medium is then heated at 60°C for 45 minutes. It is allowed to cool to room temperature and the precipitate is filtered off and washed with toluene. The toluene phase is washed with 100 ml of water and then dried over MgSO₄. After filtration, the solvent is evaporated off to dryness and 14.26 g of the expected product are recovered.

### MS: method a

Retention time Tr (min) = 1.22; [M+H]+: m/z 1206; [M-H+HCOOH]-: m/z 1250.

**1H NMR spectrum (500 MHz, in ppm, DMSO-d₆):** 0.80 (d, J=6.8 Hz, 3 H); 0.83 (d, J=7.3 Hz, 3 H); 0.90 (d, J=6.8 Hz, 3 H); 0.98 (dt, J=3.2 and 6.4 Hz, 9 H); 1.03 (d, J = 7.3 Hz, 3 H); 1.13 (d, J=6.8 Hz, 3 H); 1.16 (d, J=6.4 Hz, 3 H); 1.24 (t, J=2.9 Hz, 6 H); 1.29 (d, J=5.9 Hz, 3 H); 1.53 (m, 4 H); 1.78 (m, 1 H); 1.84 to 1.91 (m, 1 H); 1.98 (m, 1 H); 2.02 to 2.11 (m, 3 H); 2.16 (m, 3 H); 2.21 (m, 1 H); 2.34 (dd, J=5.9 and 14.2 Hz,1 H); 2.73 (dq, J=7.2 and 7.3 Hz, 1 H); 3.08 to 3.17 (m, 3 H); 3.38 (m, 1 H); 3.41 (s, 3 H); 3.44 (s, 3 H); 3.56 (d, J=5.9 Hz, 1 H); 3.66 to 3.74 (m, 5 H); 3.78 (ddd, J=2.9 and 6.0 and 11.6 Hz, 1 H); 3.96 (m, 1 H); 4.07 to 4.20 (m, 3 H); 4.39 (s, 1 H); 4.58 (m, 2 H); 4.63 (d, J=9.3 Hz, 1 H); 4.76 (d, J=9.8 Hz, 1 H); 4.91 (s, 2 H); 5.22 (d, J=6.8 Hz, 1 H); 7.12 (d, J=10.3 Hz, 2 H); 7.61 (d, J=1.5 Hz, 2 H); 8.29 (d, J=8.8 Hz, 2 H).

### Preparation 3.2:

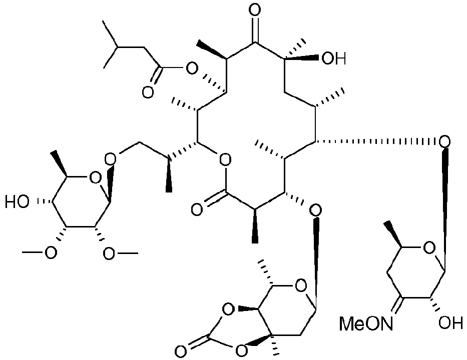

14.01 g of the compound obtained in Preparation 3.1 are placed in 140 ml of THF, 34.8 ml of 1N HCl solution are added and stirring is continued for 24 hours at room temperature. The reaction medium is poured into 200 ml of DCM and washed with 100 ml of water and then with 100 ml of saturated aqueous sodium bicarbonate solution. The aqueous phases are extracted with 200 ml of DCM, and the organic phases are combined, dried over Na₂SO₄, filtered and then evaporated to dryness under vacuum. 11.48 g of the expected product are obtained.

### MS: method a

Retention time Tr (min) = 1.19; [M+Na]+: m/z 1040; [M-H + HCOOH]-: m/z 1062.

**1H NMR spectrum (400 MHz, in ppm, DMSO-d₆):** 0.80 (d, J = 6.8 Hz, 3 H); 0.95 (m, 15 H); 1.03 to 1.17 (m, 12 H); 1.23 (s, 3 H); 1.30 (d, J = 5.9 Hz, 3 H); 1.45 (m, 1 H); 1.52 (s, 3 H); 1.78 to 1.89 (m, 2 H); 1.90 to 2.24 (m, 8 H); 2.37 (dd, J = 5.1 and 13.9 Hz, 1 H); 2.79 (m, 2 H); 2.91 (m, 1 H); 3.03 (ddd, J = 2.7 and 6.8 and 9.5 Hz, 1 H); 3.13 (q, J = 6.7 Hz, 1 H); 3.32 (masked m, 1 H); 3.37 (s, 3 H); 3.45 (s, 3 H); 3.52 (m, 2 H); 3.63 to 3.72 (m, 3 H); 3.80 (s, 3 H); 3.92 (t, J = 3.9 Hz, 1 H); 4.00 (ddd, J = 3.2 and 6.1 and 11.7 Hz, 1 H); 4.08 (m, 1 H); 4.15 (m, 1 H); 4.34 (s, 1 H); 4.45 (d, J = 8.1 Hz, 1 H); 4.63 (d, J = 9.8 Hz, 1 H); 4.68 (d, J=4.2 Hz, 1 H); 4.76 (d, J=9.5 Hz, 1 H); 4.83 (d, J = 7.1 Hz, 1 H); 4.98 (dd, J = 5.1 and 9.0 Hz, 1 H); 5.43 (d, J = 3.9 Hz, 1 H).

### Preparation 3.3:

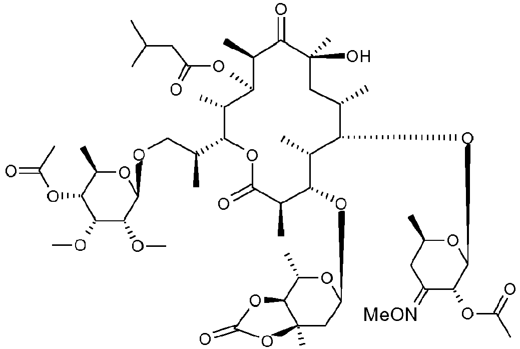

11.4 g of the compound obtained in Preparation 3.2 are placed in 114 ml of pyridine, and 7.4 ml of acetic anhydride are then added. The reaction medium is stirred at room temperature for 24 hours. The pyridine is evaporated off under vacuum and 200 ml of DCM are added to the crude reaction mixture. The resulting mixture is washed with 150 ml of 1 N HCl solution and then with 150 ml of water. The aqueous phases are extracted with twice 200 ml of DCM, and the organic phases are combined, dried over Na₂SO₄, filtered and then evaporated to dryness under vacuum. 12.2 g of compound are obtained, which product is purified by chromatography on a Merck column (400 g of 15-40 µm silica), eluting with a 45/55 EtOAc/heptane mixture. 8.6 g of the expected product are recovered.

### MS: method a

Retention time Tr (min) = 1.29;

[M+H]+: m/z 1102; [M-H+HCOOH]-: m/z 1146.

**1H NMR spectrum (400 MHz, in ppm, DMSO-d₆):** 0.81 (d, J=7.3 Hz, 3 H); 0.95 (m, 15 H); 1.06 (m, 6 H); 1.11 (d, J=6.6 Hz, 3 H); 1.20 (d, J=6.1 Hz, 3 H); 1.24 (s, 3 H); 1.29 (d, J=3.9 Hz, 3 H); 1.41 to 1.54 (m, 4H); 1.77 to 1.91 (m, 2 H); 1.96 to 2.09 (m, 10 H); 2.11 to 2.23 (m, 4 H); 2.36 (m, 1 H); 2.79 (m, 1 H); 3.02 (m, 2 H); 3.13 (m, 1 H); 3.35 (m, 1 H); 3.38 (s, 3 H); 3.41 (s, 3 H); 3.52 (d, J=7.1 Hz, 1 H); 3.64 to 3.73 (m, 2 H); 3.74 to 3.83 (m, 5 H); 3.86 (broad s, 1 H); 4.06 to 4.14 (m, 2 H); 4.33 to 4.40 (m, 2 H); 4.52 (d, J=8.1 Hz, 1 H); 4.65 (d, J=9.8 Hz, 1 H); 4.76 (m, 2 H); 4.97 (dd, J=5.3 and 8.9 Hz, 1 H); 5.02 (d, J=6.1 Hz, 1 H).

### Preparation 3.4:

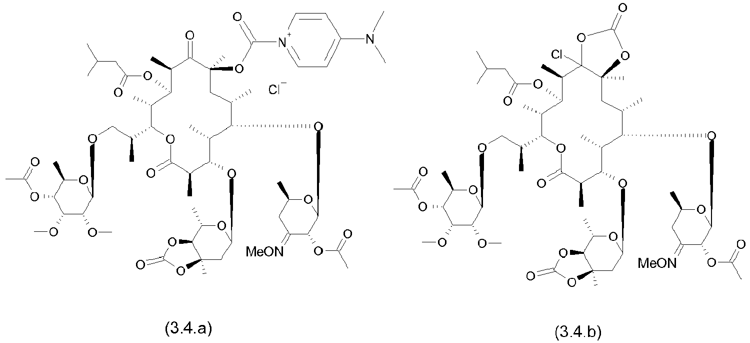

4 g of the compound obtained in Preparation 3.3 are placed, under argon, in 190 ml of DCM, and 4.12 ml of pyridine are added. The solution is cooled to -20°C, followed by addition, in a single portion, of 0.8 ml diphosgene, and stirring is continued at -20°C for 3 hours. 0.443 g of 4-dimethylaminopyridine is added, while still at -20°C, the mixture is then allowed to warm to room temperature and stirring is continued for 20 hours. The DCM is evaporated off under vacuum and the crude reaction product is taken up in 150 ml of EtOAc and stirred for 1 hour at room temperature. The precipitate formed is filtered off and rinsed with 80 ml of EtOAc. The filtrate is evaporated to dryness under vacuum, 4.7 g of the expected compounds (structures 3.4.a and 3.4.b) are obtained as a mixture, and the mixture is used as obtained for the following stage.

### MS: method a

Retention time 3.4.a: Tr (min) = 1.1 [M+H]+: 1251; 3.4.b: Tr (min) = 1.3 [M+Na]+: 1187.

### Preparation 3.5:

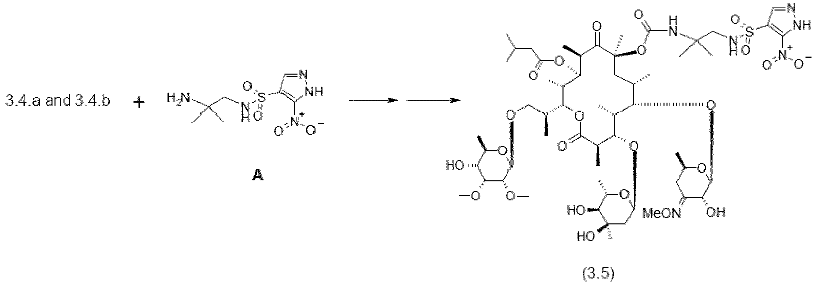

### a) Condensation of the amine A.

0.64 g of the mixture of compounds obtained in Preparation 3.4 is dissolved in 16 ml of DMF, followed by addition of 401 mg of N-(2-amino-2-methylpropyl)-5-nitro-1H-pyrazole-4-sulfonamide dihydrochloride (prepared according to the process described in international patent application WO 2009/29439 A1) and 374 µl of TEA. The mixture is stirred for 4 days at room temperature, and 75 ml of EtOAc are then added. The mixture is washed with 50 ml of water and then with 50 ml of saturated aqueous NaCl solution. The organic phase is dried over MgSO₄, filtered and concentrated under reduced pressure. 0.7 g of compound is obtained, which product is purified by chromatography on a silica column (Merck, 30 g of 15-40 µm silica), eluting with a 60/40 heptane/EtOAc mixture. 147 mg of the expected compound are obtained.

### b) Deprotection

140 mg of the compound obtained in the preceding step are placed in 3 ml of MeOH. 70 mg of K₂CO₃ are added to the solution obtained. The mixture is stirred for 24 hours at room temperature. 15 ml of EtOAc are then added. The mixture is washed with 10 ml of water and then with 10 ml of saturated aqueous NaCl solution. The organic phase is dried over MgSO₄, filtered and then evaporated to dryness under vacuum. 0.114 g of the expected product is obtained.

### MS: method b

ES⁺: m/z 1137 (base peak)

**1H NMR spectrum (500 MHz, in ppm, DMSO-d₆):** 0.79 (d, J=6.8 Hz, 3 H); 0.93 (m, 9 H); 1.01 (d, J=6.8 Hz, 3 H); 1.10 (m, 18 H); 1.17 (m, 9 H); 1.64 (s, 3 H); 1.80 (m, 5 H); 1.99 (m, 4 H); 2.13 (m, 3 H); 2.65 (m.1 H); 2.82 (m, 2 H); 2.96 (m, 5 H); 3.30 (masked m, 1 H); 3.37 (s, 3 H); 3.40 (d, J=9.8 Hz, 1 H); 3.46 (s, 3H); 3.51 (m, 1 H); 3.60 (m, 2 H); 3.67 (m, 2 H); 3.78 (m, 1 H); 3.80 (s, 3 H); 3.85 (m, 1 H); 4.08 (broad s, 1 H); 4.36 (m, 2 H); 4.44 (d, J=8.3 Hz, 1 H); 4.56 (m, 2 H); 4.80 (d, J=6.4 Hz, 1 H); 4.99 (broad d, J=2.0 Hz, 1 H); 5.09 (d, J=3.9 Hz, 1 H); 6.33 (s, 1 H); 6.86 (m, 1 H); 8.07 (broad s, 1 H); 14.52 (m, 1 H).

### Preparation 4:

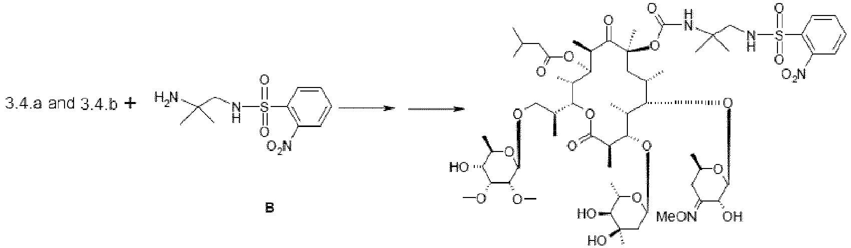

### a) Condensation of the amine B

0.64 g of the mixture of compounds obtained in Preparation 3.4 is placed in 15 ml of DMF. 462 mg of N-(2-amino-2-propylmethyl)-2-nitrobenzenesulfonamide hydrochloride (prepared according to the process described in Tetrahedron Letters, 2009, vol. 50, 28, pp. 4050 - 4053) and then 249 µl of TEA are added to the solution obtained. The mixture is stirred for 3 days at room temperature, and 75 ml of EtOAc are then added. The mixture is washed with 50 ml of water and then with 50 ml of saturated aqueous NaCl solution. The organic phase is dried over MgSO₄, filtered and concentrated under reduced pressure. 0.57 g of compound is obtained, which product is purified by chromatography on silica (30 g of 15-40 µm silica), eluting with a 55/45 heptane/EtOAc mixture.

0.25 g of the expected compound is obtained.

### b) Deprotection

0.24 g of the compound obtained in the preceding step is placed in 4 ml of MeOH. 119 mg of potassium carbonate are added to the solution obtained. The suspension is stirred at room temperature for 24 hours. 20 ml of EtOAc are added and the mixture is washed with 10 ml of water and then with 20 ml of saturated aqueous NaCl solution. The organic phase is dried over MgSO₄, filtered and concentrated under reduced pressure. 0.197 g of the expected product is obtained.

### MS: method b

ES⁺: m/z 1147 (base peak)

**1H NMR spectrum (500 MHz, in ppm, DMSO-d₆):** 0.79 (d, J=6.8 Hz, 3 H); 0.90 to 0.95 (m, 9 H); 1.01 (d, J=6.8 Hz, 3 H); 1.04 to 1.14 (m, 18 H); 1.15 to 1.20 (m, 9 H); 1.64 (s, 3 H); 1.67 to 1.88 (m, 5 H); 1.92 to 2.05 (m, 4 H); 2.09 to 2.18 (m, 3 H); 2.65 (m, 1 H); 2.81 (t, J=9.0 Hz, 1 H); 2.89 to 3.12 (m, 6 H); 3.31 (partially masked m, 1 H); 3.37 (s, 3 H); 3.40 (d, J=9.5 Hz, 1 H); 3.45 (s, 3 H); 3.51 (m, 1 H); 3.59 to 3.63 (m, 2 H); 3.65 to 3.71 (m, 2 H); 3.78 (m, 1 H); 3.80 (s, 3 H); 3.85 (dd, J=4.7 and 6.5 Hz, 1 H); 4.07 (broad s, 1 H); 4.36 (d, J=6.5 Hz, 1 H); 4.38 (d, J=8.9 Hz, 1 H); 4.43 (d, J=7.9 Hz, 1 H); 4.52 to 4.61 (m, 2 H); 4.83 (d, J=7.1 Hz, 1 H); 4.99 (broad d, J=2.9 Hz, 1 H); 5.11 (broad d, J=4.7 Hz, 1 H); 6.32 (broad s, 1 H); 7.77 to 8.05 (m, 5 H).

### Preparation 5:

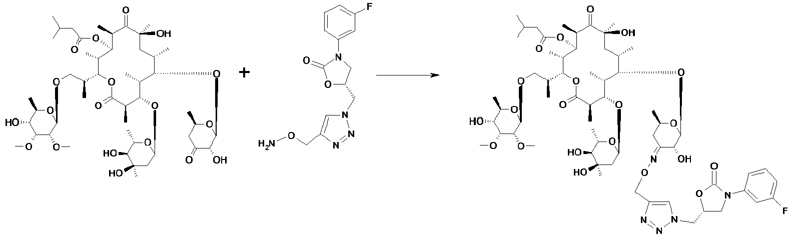

400 mg of sequanamycin (A), 180 mg of (R)-5-((4-((aminooxy)methyl)-1H-1,2,3-triazol-1-yl)methyl)-3-(3-fluorophenyl)oxazolidin-2-one (prepared according to the process described in Journal of Carbohydrate Chemistry, 2006, vol. 25, pp. 407 - 425) and 10 ml of MeOH are added. The solution obtained is stirred for 5 hours at room temperature. 20 ml of DCM are added. The mixture is washed with 20 ml of 1 M HCl and then with 20 ml of water. The organic phase is dried over MgSO₄, filtered through a sinter funnel and then evaporated to dryness. The product is purified by chromatography on a column of silica (Merck, 15-40 µm, 20 g), eluting with a 95/5 DCM/MeOH mixture. 205 mg of the expected compound are recovered.

### MS: method b

ES-: [M-H+HCO2H]-: m/z 1296.

**1H NMR spectrum (400 MHz, in ppm, DMSO-d₆):** 0.81 (d, J=6.8 Hz, 3 H); 0.96 (d, J=6.4 Hz, 9 H); 1.00 (m, 6 H); 1.07 (d, J=7.3 Hz, 3 H); 1.08 to 1.13 (m, 9 H); 1.17 (m, 6 H); 1.24 (s, 3 H); 1.46 (m, 1 H); 1.69 to 1.75 (m, 2 H); 1.79 to 1.91 (m, 2 H); 1.95 to 2.20 (m, 7 H); 2.73 (m, 1 H); 2.81 (m, 1 H); 2.89 to 2.97 (m, 2 H); 3.03 (m, 1 H); 3.19 (broad q, J=6.8 Hz, 1 H); 3.27 to 3.38 (partially masked m, 2 H); 3.37 (s, 3 H); 3.45 (s, 3 H); 3.52 (m, 1 H); 3.60 (broad s, 1 H); 3.62 to 3.68 (m, 2 H); 3.72 (m, 1 H); 3.76 to 3.85 (m, 2 H); 3.86 to 3.94 (m, 2 H); 4.26 (t, J=9.3 Hz, 1 H); 4.38 to 4.46 (m, 3 H); 4.50 (s, 1 H); 4.72 (d, J=8.3 Hz, 1 H); 4.78 (d, J=8.6 Hz, 1 H); 4.82 (m, 3 H); 4.89 (broad d, J=3.8 Hz, 1 H); 5.10 (s, 2 H); 5.12 to 5.20 (m, 2 H); 6.97 (dt, J=2.2 and 8.4 Hz, 1 H); 7.27 (broad d, J=8.4 Hz, 1 H); 7.37 to 7.51 (m, 2 H); 8.22 (s, 1 H).

### EXAMPLE 1: Compound 1

**Compound 1-a:** (2R,3S,4R,5R,7S,9S,10S,11R,12S,13R)-7-hydroxy-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2*H-*pyran-2-yl]oxy}-3,5,7,9,11,13-hexamethyl-6,14-dioxo-12-{[(2S,5R,7R)-2,4,5-trimethyl-1,4-oxazepan-7-yl]oxy}oxacyclotetradecan-4-yl 3-methylbutanoate.

**Compound 1-b:** (2R,3S,4R,5R,7S,9S,10S,11R,12S,13R)-7-hydroxy-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5S,7R)-2,4,5-trimethyl-1,4-oxazepan-7-yl]oxy}oxacyclotetradecan-4-yl 3-methylbutanoate.

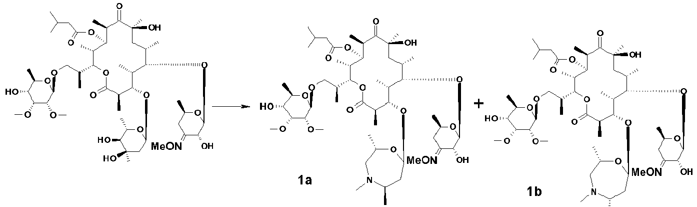

3 g of the compound of Preparation 1 are placed in 68 ml of MeOH. The reaction medium is cooled in an ice bath to a temperature of +4°C, followed by dropwise addition of a solution of 3.23 g of sodium periodate in 68 ml of water. The mixture is stirred for 6 hours at room temperature.

The medium is saturated with NaCl and filtered, and the filtrate is extracted with DCM (3×200 ml). The organic phases are combined, washed with saturated aqueous NaCl solution, dried over MgSO₄, filtered and finally concentrated under reduced pressure. The oily residue obtained is dissolved, under argon, in 680 ml of MeOH. The pH is adjusted to 7 by addition of acetic acid, followed by addition of 2 M methylamine disolved in 12.1 ml of THF. The pH is maintained at 7 with acetic acid. After stirring for 30 minutes at room temperature, 0.95 g of NaBH₃CN is added in a single portion, and the mixture is stirred for a further 16 hours at room temperature. The reaction medium is filtered and rinsed with MeOH. The filtrate is concentrated under reduced pressure and then taken up in 600 ml of DCM. The resulting mixture is washed with saturated aqueous NaCl solution (3 × 60 ml). The organic phase is dried over MgSO₄, filtered and then evaporated to dryness under vacuum. 3.5 g of product are purified by chromatography on a Merck cartridge (150 g of 15-40 µm silica) with a 100/0 to 90/10 DCM/MeOH elution gradient. 530 mg of diastereoisomer **1-a,** 380 mg of diastereoisomer **1-b** and 661 mg of a mixture of the two isomers are obtained.

### Compound 1-a:

### MS: method b

Retention time Tr (min) = 1.26; [M+H]+: m/z 989; [M-H+HCO₂H]- m/z 1033 (base peak).

**1H NMR spectrum (500 MHz, in ppm, DMSO-d₆):** 0.79 (d, *J*=6.8 Hz, 3 H); 0.89 to 1.01 (m, 15 H);1.03 (d, *J*=6.8 Hz, 3 H); 1.05 to 1.10 (m, 9 H); 1.11 (d, *J*=6.1 Hz, 3 H); 1.13 (d, *J*=6.1 Hz, 3 H); 1.24 (s, 3H); 1.48 (dd, *J*=11.4 and 14.7 Hz, 1 H); 1.70 to 2.08 (m, 8 H); 2.10 to 2.22 (m, 3 H); 2.18 (broad s, 3 H); 2.36 (m, 1 H); 2.57 (m, 1 H); 2.70 (d, *J*=13.6 Hz, 1 H); 2.75 (m, 1 H); 2.83 (dd, *J*=2.9 and 16.6 Hz, 1 H);2.92 (dd, *J*=2.7 and 8.0 Hz, 1 H); 3.03 (m, 1 H); 3.12 (q, *J*=6.8 Hz, 1 H); 3.30 (partially masked m, 1 H); 3.38 (s, 3 H); 3.45 (s, 3 H); 3.52 (m, 1 H); 3.58 to 3.72 (m, 4 H); 3.80 (s, 3 H); 3.89 (m, 2 H); 4.26 (m, 1 H); 4.31 (s, 1 H); 4.45 (d, *J*=8.0 Hz, 1 H); 4.65 (broad d, *J*=9.8 Hz, 1 H); 4.70 (d, *J*=4.6 Hz, 1 H); 4.74 (d, *J*=9.6 Hz, 1 H); 4.86 (d, *J*=7.1 Hz, 1 H); 4.93 (dd, *J*=3.1 and 9.5 Hz, 1 H); 5.33 (d, *J*=4.6 Hz, 1 H).

### Compound 1-b:

### MS: method b

Retention time Tr (min) = 1.26; [M-H+HCO₂H]⁻: m/z 1033 (base peak).

**1H NMR spectrum (500 MHz, in ppm, DMSO-d₆):** 0.82 (d, J=6.8 Hz, 3 H); 0.91 to 1.32 (m, 36 H); 1.44 to 1.51 (m, 1 H); 1.80 to 1.87 (m, 1 H); 1.96 to 2.37 (m, 10 H); 2.76 to 2.80 (m, 2 H); 2.77 (s, 3 H); 2.85 (dd, J=3.1 and 16.8 Hz, 1 H); 2.93 (dd, J=2.7 and 8.0 Hz, 1 H); 3.00 to 3.07 (m, 2 H); 3.12 to 3.18 (m, 1 H); 3.28 (d, J=13.7 Hz, 1 H); 3.31 to 3.36 (m, 1 H); 3.40 (s, 3 H); 3.48 (s, 3 H); 3.51 to 3.58 (m, 2 H); 3.64 to 3.70 (m, 2H); 3.82 (s, 3 H); 3.86 to 3.90 (m, 1 H); 3.94 to 4.02 (m, 2 H); 4.31 to 4.37 (m, 1 H); 4.47 (d, J=7.9 Hz, 1 H);4.66 to 4.77 (m, 3 H); 5.12 (dd, J=5.8 and 8.9 Hz, 1 H).

### EXAMPLE 2: Compound 2

(2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-7-({[2-(pyridin-4-yl)ethyl]carbamoyl}oxy)-12-{[(2S,5S,7R)-2,4,5-trimethyl-1,4-oxazepan-7-yl]oxy}oxacyclotetradecan-4-yl 3-methylbutanoate;

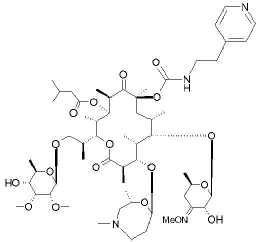

### Step 2.1:

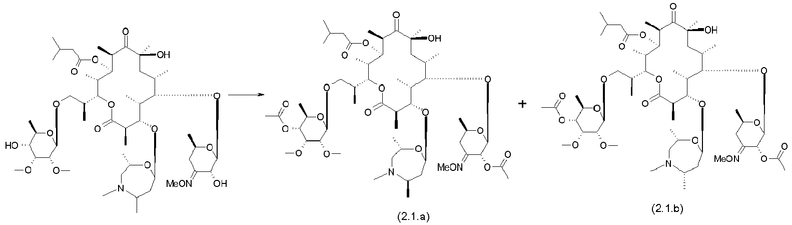

660 mg of the mixture of diastereoisomers prepared in Example 1 are placed in 14.7 ml of pyridine. 631 µl of acetic anhydride are added. After stirring for 24 hours at room temperature, 189 µl of acetic anhydride are added and stirring is continued at room temperature for 24 hours. The solution is concentrated under vacuum and the residue is taken up in 200 ml of DCM. The resulting mixture is washed with 1N HCl solution (3 × 5 ml) and then with saturated aqueous sodium bicarbonate solution (2 × 5 ml) and finally with saturated aqueous NaCl solution (3 × 5 ml). The organic phase is dried over MgSO₄, filtered and then evaporated to dryness under vacuum. 756 mg of crude product are obtained, which product is purified by chromatography on a Merck cartridge (15-40 µm silica) with a 100/0 to 95/5 DCM/MeOH elution gradient. 287 mg of diastereoisomer **2.1.a** and 266 mg of diastereoisomer **2.1.b** are obtained.

### Compound 2.1.a:

### MS: method b

Retention time Tr (min) = 1.46; [M+H]⁺: 1073

**1 H NMR spectrum (500 MHz, in ppm, DMSO-d₆):** 0.79 (s, 3 H); 0.91 (m, 6 H); 0.97 (m, 9 H); 1.02 (d, J=6.6 Hz, 3 H); 1.07 (m, 12 H); 1.19 (d, J=6.1 Hz, 3 H); 1.24 (s, 3 H); 1.49 (dd, J=11.7 and 14.5 Hz, 1 H); 1.82 (m, 4 H); 1.98 (m, 4 H); 2.05 (s, 3 H); 2.08 (s, 3 H); 2.16 (m, 3 H); 2.20 (s, 3 H); 2.44 (dd, J=9.9 and 14.4 Hz, 1 H); 2.64 (m, 1 H); 2.74 (m, 2 H); 3.03 (m, 2 H); 3.12 (q, J=6.7 Hz, 1 H); 3.35 (dd, J=4.9 and 10.1 Hz, 1 H); 3.39 (s, 3 H); 3.41 (s, 3 H); 3.55 (d, J=8.0 Hz, 1 H); 3.67 (m, 2 H); 3.79 (m, 2 H); 3.78 (s, 3 H); 3.86 (t, J=2.7 Hz, 1 H); 4.14 (m, 1 H); 4.31 (s, 1 H); 4.37 (dd, J=2.5 and 9.9 Hz, 1 H); 4.52 (d, J=8.0 Hz, 1 H); 4.65 (d, J=9.8 Hz, 1 H); 4.76 (d, J=9.5 Hz, 1 H); 4.79 (d, J=6.8 Hz, 1 H); 4.88 (dd, J=3.4 and 8.6 Hz, 1 H); 5.01 (d, J=6.8 Hz, 1 H).

### Compound 2.1.b:

### MS: method b

Retention time Tr (min) = 1.46; [M+H]⁺: 1073

**1H NMR spectrum (500 MHz, in ppm, in DMSO-d₆+ TFA + AcOD)** : 0.82 (d, J=6.8 Hz, 3 H); 0.95 (m, 15 H); 1.06 (m, 9 H); 1.20 (m, 6 H); 1.25 (s, 3 H); 1.30 (d, J=6.9 Hz, 3 H); 1.48 (m, 1 H); 1.84 (m, 1 H); 1.94 to 2.36 (m, 10 H); 2.08 (s, 6 H); 2.75 (m, 1 H); 2.76 (s, 3 H); 3.06 (m, 3 H); 3.15 (q, J=6.5 Hz, 1 H); 3.20 (d, J=13.7 Hz, 1 H); 3.37 (dd, J=4.7 and 9.7 Hz, 1 H); 3.40 (s, 3 H); 3.43 (s, 3 H); 3.54 (m, 1 H); 3.60 (m, 1 H); 3.68 to 3.77 (m, 2 H); 3.81 (m, 1 H); 3.79 (s, 3 H); 3.87 (t, J=2.4 Hz, 1 H); 3.91 (m, 1 H); 4.26 (m, 1 H); 4.38 (dd, J=2.4 and 9.9 Hz, 1 H); 4.54 (d, J=8.0 Hz, 1 H); 4.67 (d, J=9.5 Hz, 1 H); 4.72 (m, 2 H); 5.04 (d, J=6.6 Hz, 1 H); 5.09 (dd, J=5.1 and 8.9 Hz, 1 H).

### Step 2.2:

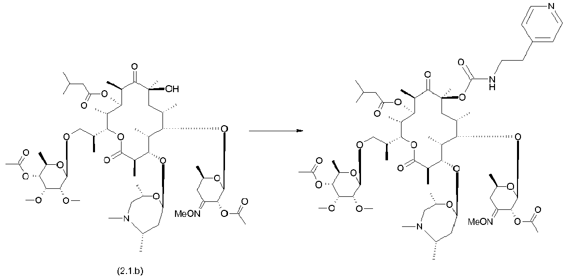

50 mg of diastereoisomer 2.1.b of the preceding step are placed, under argon, in 2 ml of DCM and 65 µl of pyridine. The colourless solution is cooled to -10°C. 10 µl of trichloromethyl chloroformate are then added, stirring is continued at -10°C for 3 hours, and 5.7 mg (46.67 µmol) of 4-dimethylaminopyridine are then added. The mixture is allowed to warm to room temperature, and stirring is then continued for 20 hours. The resulting mixture is concentrated under vacuum, the residue is taken up in 5 ml of EtOAc and the insoluble matter is filtered off and rinsed with 2 ml of EtOAc. The filtrate is concentrated under vacuum. 55 mg of the compound obtained are dissolved in 2 ml of DMF. 53.4 mg of 4-(2-aminoethyl)pyridine are then added and the mixture is stirred for 48 hours at room temperature. The medium is poured onto an ice/water mixture. The resulting mixture is filtered by suction and the precipitate formed is then dried under vacuum. 34 mg of the expected product are obtained.

### MS: method b

Retention time Tr (min) = 1.16 ES+: [M+2H]2+ m/z 611 (base peak); ES-: [M-H+HCO₂H]-: m/z 1265 (base peak)

**1H NMR spectrum (500 MHz, in ppm, DMSO-d₆):** 0.79 (d, J=6.9 Hz, 3 H); 0.89 to 1.11 (m, 30 H); 1.17 (m, 3 H); 1.64 to 2.18 (m, 12 H); 1.67 (s, 3 H); 2.07 (s, 3 H); 2.08 (s, 3 H); 2.27 (s, 3 H); 2.44 (d, J=13.2 Hz, 1 H); 2.55 (masked m, 1 H); 2.70 (m, 3 H); 2.85 (m, 1 H); 3.02 (m, 5 H); 3.35 (masked m, 1 H); 3.38 (s, 3H); 3.41 (s, 3 H); 3.67 (m, 3 H); 3.80 (m, 2 H); 3.78 (s, 3 H); 3.86 (s, 1 H); 4.14 (m, 1 H); 4.37 (dd, J=2.2 and 9.9 Hz, 1 H); 4.52 (d, J=8.0 Hz, 1 H); 4.57 (d, J=9.6 Hz, 1 H); 4.64 (d, J=9.3 Hz, 1 H); 4.75 (d, J=6.6 Hz, 1H); 4.96 (m, 1 H); 5.01 (d, J=6.6 Hz, 1 H); 6.89 (broad t, J=5.4 Hz, 1 H); 7.18 to 7.23 (m, 2 H); 8.43 (d, J=5.5 Hz, 2 H)

### Step 2.3:

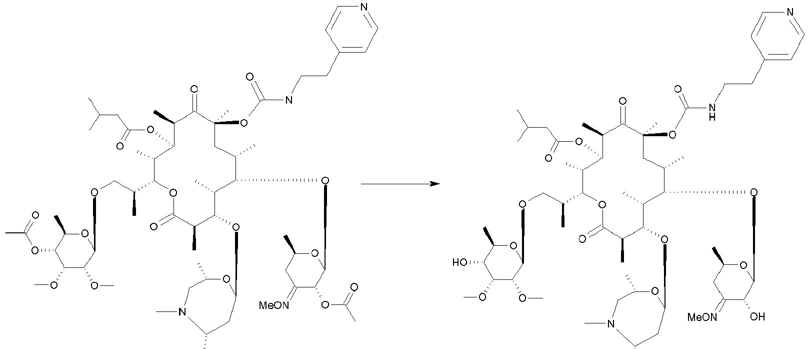

32.5 mg of the product prepared in the preceding step are placed in 2 ml of MeOH with 11 mg of K₂CO₃. After stirring for 1 hour 30 minutes at room temperature, 20 ml of EtOAc are added to the reaction medium. The resulting mixture is washed with 20 ml of saturated aqueous NaCl solution. After separation of the phases by settling, the organic phase is dried over MgSO₄, filtered and then evaporated to dryness under vacuum. 24 mg of the expected compound are obtained.

### MS: method b

Retention time Tr (min) = 0.97; [M+₂H]2+: 569 (base peak); [M-H+HCO₂H]-: m/z 1181.

**1H NMR spectrum (500 MHz, in ppm, DMSO-d₆):** 0.78 (d, J=6.9 Hz, 3 H); 0.96 to 1.15 (m, 33 H); 1.64 to 1.87 (m, 6 H); 1.92 to 2.19 (m, 9 H); 2.25 (s, 3 H); 2.42 (d, J=13.4 Hz, 1 H); 2.55 (masked m, 1 H); 2.70 (m, 3 H); 2.85 (m, 2 H); 2.92 (dd, J=2.3 and 8.1 Hz, 1 H); 3.01 (m, 2 H); 3.12 (m, 2 H); 3.30 (masked m, 1 H); 3.37 (s, 3 H); 3.45 (s, 3 H); 3.52 (m, 1 H); 3.61 (m, 2 H); 3.68 (broad s, 1 H); 3.80 (m, 4 H); 3.97 (m, 2 H); 4.28 (m, 1 H); 4.44 (d, J=8.0 Hz, 1 H); 4.53 to 4.65 (m, 2 H); 4.69 (d, J=4.7 Hz, 1 H); 4.86 (d, J=7.1 Hz, 1 H); 4.99 (m, 1 H); 5.38 (d, J=4.7 Hz, 1 H); 6.89 (m, 1 H); 7.23 (d, J=5.2 Hz, 2 H); 8.43 (d, J=5.5 Hz, 2 H).

### EXAMPLE 3: Compound 3

(2R,3S,4R,5R,7S,9S,10S,11R,12S,13R)-7-[(dimethylcarbamoyl)oxy]-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5R,7R)-2,4,5-trimethyl-1,4-oxazepan-7-yl]oxy}oxacyclotetradecan-4-yl 3-methylbutanoate.

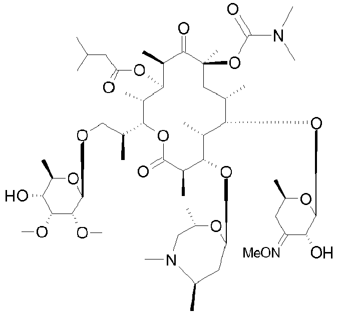

### Step 3.1:

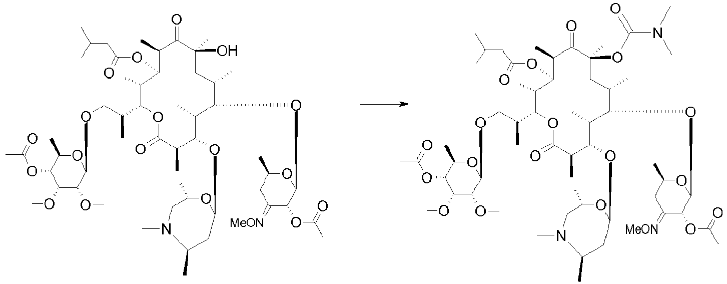

150 mg of compound **2.1.a** obtained in step 2.1 of Example 2 are placed in 9 ml of DCM and 210 µl of pyridine in a 30 ml round-bottomed flask, under argon. The colourless solution is cooled to -10°C. 30 µl of trichloromethyl chloroformate are then added and stirring is continued for 3 hours at -10°C, followed by addition of 17 mg of 4-dimethylaminopyridine dissolved in 2 ml of DCM. The mixture is allowed to warm to room temperature, and stirring is continued for 20 hours. The resulting mixture is concentrated under vacuum, the residue is taken up in 4 ml of DMF, and dimethylamine hydrochloride (173 mg) dissolved in DMF (2 ml) and TEA (0.3 ml) is then added. The reaction medium is stirred for 3 hours at room temperature. The resulting mixture is poured into an ice/water mixture, and the precipitate formed is filtered off by suction. 146 mg of product are recovered, which product is purified by chromatography on a Merck cartridge (5 g of 15-40 µm silica), eluting with a 95/5 DCM/MeOH mixture. 93 mg of the expected compound is obtained.

### MS: method b

Retention time Tr (min) = 1.33; [M+H]⁺: 1144

**1H NMR spectrum (500 MHz, in ppm, DMSO-d₆):** 0.79 (d, J=6.9 Hz, 3 H); 0.92 (m, 6 H); 0.96 (d, J=6.6Hz, 6 H); 1.05 (m, 18 H); 1.17 to 1.19 (m, 3 H); 1.69 to 2.03 (m, 9 H); 1.71 (s, 3 H); 2.05 (s, 3 H); 2.08 (s, 3H); 2.13 (m, 3 H); 2.21 (s, 3 H); 2.44 to 2.49 (masked m, 1 H); 2.72 (m, 6 H); 2.84 (broad s, 3 H); 3.03 (m, 3H); 3.33 (masked, 1 H); 3.39 (s, 3 H); 3.42 (s, 3 H); 3.65 (m, 3 H); 3.77 (s, 3 H); 3.80 (m, 1 H); 3.86 (t.J=2.6 Hz, 1 H); 3.89 (d, J=4.3 Hz, 1 H); 4.10 (m, 1 H); 4.37 (dd, J=2.5 and 9.9 Hz, 1 H); 4.52 (d, J=8.0 Hz, 1H); 4.59 (d, J=10.1 Hz, 1 H); 4.69 (d, J=9.6 Hz, 1 H); 4.73 (d, J=7.3 Hz, 1 H); 4.83 (dd, J=3.8 and 7.9 Hz, 1 H); 5.00 (d, J=7.3 Hz, 1 H).

### Step 3.2:

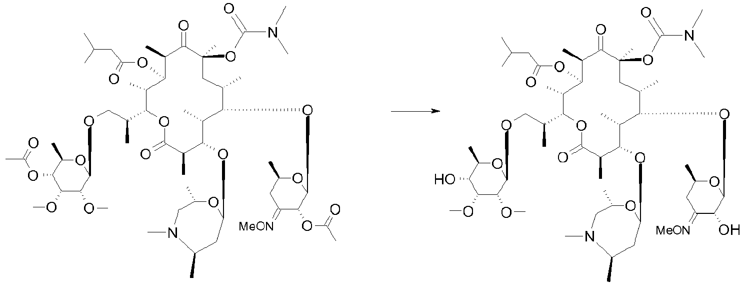

90 mg of the product obtained in the preceding step are placed in 5.5 ml of MeOH, and 32.6 mg of K₂CO₃. After stirring for 1 hour 30 minutes at room temperature, 50 ml of EtOAc are added to the reaction medium. The resulting mixture is washed with 20 ml of saturated aqueous NaCl solution. After separation of the phases by settling, the organic phase is dried over MgSO₄, filtered and then evaporated to dryness under vacuum. 50 mg of the expected compound is obtained.

### MS: method b

Retention time Tr (min) = 1.12; [M+H]⁺: 1060.

**1H NMR spectrum (500 MHz, in ppm, DMSO-d₆):** 0.78 (d, J=6.9 Hz, 3 H); 0.92 (m, 6 H); 0.96 (d, J=6.6 Hz, 6 H); 1.01 to 1.14 (m, 21 H); 1.70 to 2.06 (m, 9 H); 1.71 (s, 3 H); 2.09 to 2.17 (m, 3 H); 2.19 (s, 3 H); 2.38 (dd, J=9.4 and 14.0 Hz, 1 H); 2.59 (m, 1 H); 2.79 (m, 9 H); 2.92 (dd, J=2.6 and 8.0 Hz, 1 H); 3.02 (m, 2 H); 3.30 (masked m, 1 H); 3.38 (s, 3 H); 3.46 (s, 3 H); 3.51 (m, 1 H); 3.63 (dd, J=4.4 and 9.6 Hz, 1 H); 3.67 (broad s, 1 H); 3.71 (d, J=6.9 Hz, 1 H); 3.77 (d, J=5.4 Hz, 1 H); 3.83 (m, 4 H); 3.89 (t, J=4.9 Hz, 1 H); 4.21 (m, 1 H); 4.45 (d, J=7.9 Hz, 1 H); 4.62 (m, 3 H); 4.88 (m, 2 H); 5.24 (d, J=4.6 Hz, 1 H).

### EXAMPLE 4: Compound 4

(2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(5-nitro-1H-pyrazol-4-yl)sulfonyl]amino}propan-2-yl)carbamoyl]oxy}-6,14-dioxo-12-{[(2S,7R)-2,4,5-trimethyl-1,4-oxazepan-7-yl]oxy}oxacyclotetradecan-4-yl 3-methylbutanoate.

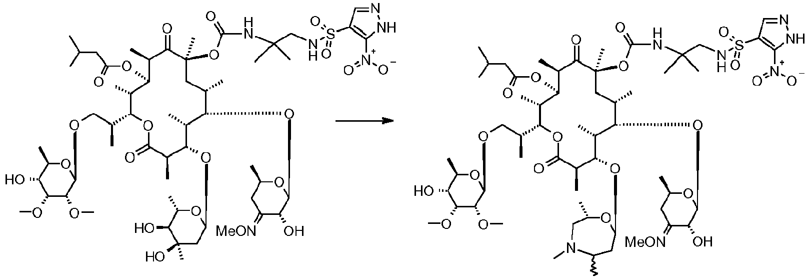

102 mg of the product obtained in Preparation 3 are placed in 2 ml of MeOH. The solution is cooled to 0°C, and a solution of 86 mg of sodium metaperiodate in water (2 ml) is added. The reaction mixture is stirred magnetically at room temperature for 5 hours. 15 ml of DCM are added and the mixture is washed with 10 ml of water and then with 10 ml of saturated aqueous NaCl solution. The organic phase is dried over MgSO₄, filtered and concentrated under reduced pressure. 86 mg of product obtained are dissolved in MeOH (10 ml), the solution is cooled to 0°C and methylamine (84 µl of a 2M solution in THF) and then acetic acid (9.7 µl) are added. After stirring for 10 minutes at 0°C, 13.34 mg of NaBH₃CN are added. The reaction medium is stirred for 1 hour at 0°C and is then allowed to warm to room temperature, and stirring is continued for 20 hours. 20 ml of DCM are added and the mixture is washed with 15 ml of saturated aqueous NaHCO₃ solution and then with 15 ml of saturated aqueous NaCl solution. The organic phase is dried over MgSO₄, filtered and concentrated under reduced pressure. 0.12 g of product obtained is purified by preparative LC/MS, eluting with a 15/85 to 95/5 gradient of acetonitrile/water containing 0.1% TFA. The fractions of mass 1278 to 1281 are recovered. The recovered phases are brought to pH 8 with saturated aqueous NaHCO₃ solution and then extracted with 50 ml of EtOAc. The organic phase is dried over MgSO₄, filtered and then evaporated to dryness under vacuum. 38 mg of the expected product are obtained.

### MS: method b

Retention time Tr (min) = 1.06; [M+Na]⁺: 1278

**1H NMR spectrum (500 MHz, in ppm, DMSO-d₆):** 70/30 mixture of the diastereoisomers, 0.78 (d, J=6.8 Hz, 3 H); 0.91 (d, J=6.8 Hz, 3 H); 0.95 (m, 9 H); 1.02 to 1.16 (m, 27 H); 1.68 (broad s, 3 H); 1.71 to 1.90 (m, 4 H); 1.93 to 2.21 (m, 8 H); 2.36 (broad s, 2.1 H); 2.46 (broad s, 0.9 H); 2.61 (m, 1 H); 2.69 to 3.06 (m, 9 H); 3.30 (masked m, 1 H); 3.38 (s, 3 H); 3.46 (s, 3 H); 3.49 to 3.64 (m, 2.3 H); 3.67 (t, J=2.6 Hz, 1 H); 3.72 (m, 1 H); 3.78 to 3.91 (m, 2.7 H); 3.80 (s, 3 H); 4.30 (m, 1 H); 4.45 (d, J=7.9 Hz, 1 H); 4.57 to 4.67 (m, 3 H); 4.83 (d, J=6.8 Hz, 1 H); 4.96 (dd, J=3.0 and 9.2 Hz, 0.7 H); 5.04 (dd, J=4.8 and 8.6 Hz, 0.3 H); 5.26 (broad s, 0.7 H); 5.36 (broad s, 0.3 H); 6.32 (broad s, 1 H); 6.70 m, 1 H); 7.89 to 7.93 (m, 1 H).

### EXAMPLE 5: Compound 5

**Compound 5-a:** (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(2-nitrophenyl)sulfonyl]amino}propan-2-yl)carbamoyl]oxy}-6,14-dioxo-12-{[(2S,5R,7R)-2,4,5-trimethyl-1,4-oxazepan-7-yl]oxy}oxacyclotetradecan-4-yl 3-methylbutanoate.

**Compound 5-b:** (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(2-nitrophenyl)sulfonyl]amino}propan-2-yl)carbamoyl]oxy}-6,14-dioxo-12-{[(2S,5S,7R)-2,4,5-trimethyl-1,4-oxazepan-7-yl]oxy}oxacyclotetradecan-4-yl 3-methylbutanoate.

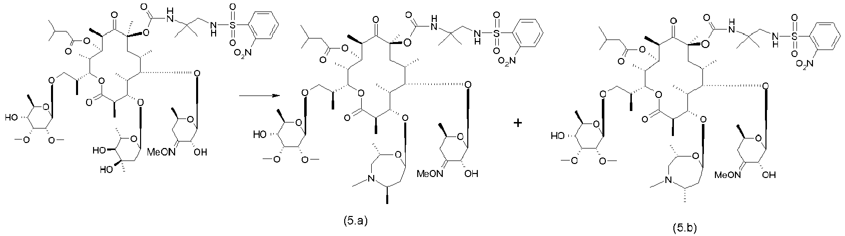

A solution of 99 mg of the product obtained in Preparation 4 in 2 ml of MeOH is cooled to 0°C. A solution of 83 mg of NaIO₄ in 2 ml of water is then added dropwise. After 15 minutes at 0°C, the mixture is allowed to warm to room temperature, and stirring is continued for 3 hours. The reaction medium is poured into 20 ml of DCM. The resulting mixture is washed with 10 ml of water, the phases are separated by settling and then washed again with 10 ml of saturated aqueous NaCl solution. The organic phase is dried over MgSO₄, filtered and then evaporated to dryness. 110 µl of a 2M solution of methylamine in THF, 13.9 µl of AcOH and finally 16.67 mg of NaBH₃CN are added, in this order, to a solution of 95 mg of the compound thus obtained in 8 ml of MeOH. The mixture is stirred for 20 hours at room temperature. 20 ml of DCM are added and the resulting mixture is washed with saturated aqueous NaHCO₃ solution and then with aqueous NaCl solution. The aqueous phases are extracted with DCM. The organic phases are combined, dried over MgSO₄, filtered and then evaporated to dryness. The 85 mg of product obtained are combined with another batch of 68 mg prepared in a preceding reaction under the conditions described above. This mixture is purified by chromatography on silica (10 g of 15-40 µm silica) with a 94.5/5.5 DCM/MeOH elution solvent. 49 mg of diastereoisomer **5-a** and 30 mg of isomer **5-b** are obtained.

### Compound 5-a:

### MS: method b

Retention time Tr (min) = 1.16; [M+H]⁺: 1288

**1H NMR spectrum (500 MHz, in ppm, DMSO-d₆):** 0.78 (d, J=6.8 Hz, 3 H); 0.90 (d, J=6.8 Hz, 3 H); 0.92 to 0.97 (m, 9 H); 1.00 to 1.17 (m, 27 H); 1.62 to 1.70 (m, 4 H); 1.71 to 1.85 (m, 4 H); 1.89 to 2.08 (m, 4 H); 2.13 (s, 3 H); 2.19 (s, 3 H); 2.39 (dd, J=9.7 and 14.2 Hz, 1 H); 2.59 (m, 1 H); 2.74 (m, 2 H); 2.83 to 3.11 (m, 6 H); 3.28 (masked m, 1 H); 3.38 (s, 3 H); 3.46 (s, 3 H); 3.52 (quint, J=6.2 Hz, 1 H); 3.62 (dd, J=4.4 and 9.8 Hz, 1 H); 3.68 (m, 2 H); 3.76 (d, J=4.5 Hz, 1 H); 3.82 (m, 5 H); 4.26 (quint, J=7.2 Hz, 1 H); 4.45 (d, J=7.9 Hz, 1 H); 4.59 (d, J=9.9 Hz, 1 H); 4.68 (m, 2 H); 4.83 (d, J=7.1 Hz, 1 H); 4.91 (dd, J=2.8 and 9.2 Hz, 1 H); 5.23 (d, J=4.4 Hz, 1 H); 6.28 (s, 1 H); 7.91 (m, 5 H).

### Compound 5-b:

### MS: method b

Retention time Tr (min) = 1.16; [M+H]⁺: 1288

**1H NMR spectrum (500 MHz, in ppm, DMSO-d₆):** 0.78 (d, J=6.8 Hz, 3 H); 0.90 (d, J=7.0 Hz, 3 H); 0.95 (d, J=6.7 Hz, 9 H); 1.00 (d, J=6.7 Hz, 3 H); 1.05 (m, 9 H); 1.09 (d, J=6.2 Hz, 3 H); 1.13 (m, 9 H); 1.24 (s, 3 H); 1.66 (s, 3 H); 1.78 (m, 4 H); 1.99 (m, 4 H); 2.13 (m, 4 H); 2.25 (s, 3 H); 2.42 (d, J=13.0 Hz, 1 H); 2.53 (m, 1 H); 2.74 (quint, J=7.3 Hz, 1 H); 2.87 (m, 4 H); 2.98 (q, J=6.4 Hz, 1 H); 3.07 (m, 2 H); 3.32 (masked m, 1 H); 3.37 (s, 3 H); 3.46 (s, 3 H); 3.52 (m, 1 H); 3.62 (m, 2 H); 3.67 (t, J=2.5 Hz, 1 H); 3.80 (m, 4 H); 3.88 (m, 2 H); 4.29 (quint, J=7.8 Hz, 1 H); 4.44 (d, J=7.9 Hz, 1 H); 4.58 (d, J=9.3 Hz, 1 H); 4.63 (d, J=9.4 Hz, 1 H); 4.72 (d, J=4.5 Hz, 1 H); 4.83 (d, J=7.2 Hz, 1 H); 5.00 (dd, J=4.5 and 9.1 Hz, 1 H); 5.35 (d, J=4.5 Hz, 1 H); 6.27 (broad s, 1 H); 7.74 to 8.05 (m, 5 H).

### EXAMPLE 6: Compound 6

(2R,3S,4R,5R,7S,9S,10S,11R,12S,13R)-7-[(benzylcarbamoyl)oxy]-12-{[(2S,7R)-4-(2-fluoroethyl)-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate.

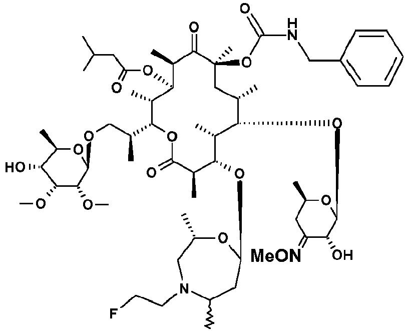

0.5 g of the compound obtained in Preparation 2 is placed in 10 ml of MeOH.

The solution obtained is cooled to 0°C. A solution of 0.475 mg of sodium metaperiodate in 10 ml of water is then rapidly added dropwise. After 15 minutes at 0°C, the mixture is allowed to warm to room temperature, and stirring is continued for 5 hours. The medium is saturated with NaCl (∼3 g) and taken up in DCM (40 ml). The precipitate is filtered off and washed with saturated aqueous NaCl solution. The aqueous phase is extracted with DCM. The organic phases are combined, dried over MgSO₄, filtered and then evaporated to dryness under vacuum. 443 mg of the expected product are obtained.

100 mg of this compound are dissolved in 2.2 ml of MeOH, followed by addition, in the following order, of 25 µl of TEA, 22 mg of 2-fluoroethylamine hydrochloride, 12.7 µl of acetic acid and finally 16.8 mg of NaBH₃CN. The medium is stirred for 20 hours at room temperature. 20 ml of DCM are added and the resulting mixture is washed with saturated aqueous sodium bicarbonate solution and then with aqueous NaCl solution. The aqueous phases are extracted with DCM. The organic phases are combined, dried over Na₂SO₄, filtered and then evaporated to dryness. 92 g of the product obtained are purified by chromatography on silica (5 g of 15-40 µm silica) with a 97/3 DCM/MeOH eluent mixture. The expected product is obtained in the form of a mixture of diastereoisomers.

### MS: method b

Retention time Tr (min) = 1.28; [M-H+HCO₂H]-: m/z 1198.

**1H NMR spectrum (500 MHz, in ppm, DMSO-d₆):** 70/30 mixture of the diastereoisomers 0.78 (d, J=6.8 Hz, 3 H); 0.91 (d, J=6.9 Hz, 3 H); 0.94 (d, J=7.3 Hz, 3 H); 0.98 (dd, J=1.6 and 6.6 Hz, 6 H); 1.01 to 1.16 (m, 21 H); 1.60 to 2.22 (m, 15 H); 2.52 (masked m, 1 H); 2.59 (d, J=5.0 Hz, 1 H); 2.68 to 3.08 (m, 9 H); 3.28 (masked m, 1 H); 3.38 (s, 3 H); 3.46 (s, 3 H); 3.52 (m, 1 H); 3.64 (m, 2 H); 3.72 to 3.93 (m, 7 H); 4.10 (d, J=6.0 Hz, 2 H); 4.24 (m, 1 H); 4.41 (m, 3 H); 4.56 to 4.72 (m, 3 H); 4.83 (d, J=7.1 Hz, 1 H); 4.88 (m, 0.7 H); 4.99 (dd, J=4.0 and 9.1 Hz, 0.3 H); 5.13 (d, J=4.6 Hz, 0.7 H); 5.31 (d, J=4.6 Hz, 0.3 H); 7.28 (m, 5 H).

### EXAMPLE 7: Compound 7

**Compound 7-a:** (2R,3S,4R,5R,7S,9S,10S,11R,12S,13R)-7-[(benzylcarbamoyl)oxy]-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5R,7R)-2,4,5-trimethyl-1,4-oxazepan-7-yl]oxy}oxacyclotetradecan-4-yl 3-methylbutanoate.

**Compound 7-b:** (2R,3S,4R,5R,7S,9S,10S,11R,12S,13R)-7-[(benzylcarbamoyl)oxy]-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5S,7R)-2,4,5-trimethyl-1,4-oxazepan-7-yl]oxy}oxacyclotetradecan-4-yl 3-methylbutanoate.

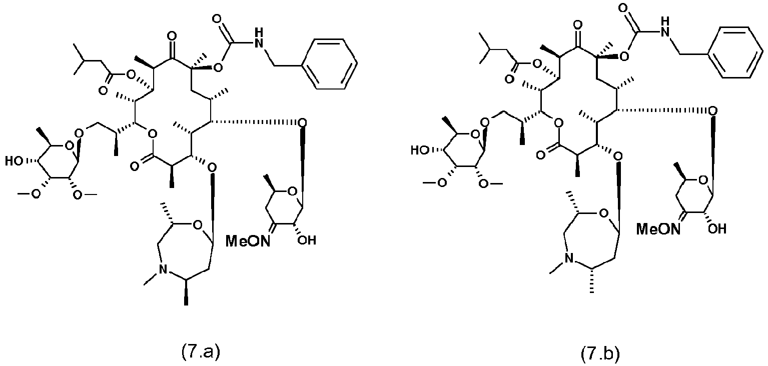

0.68 g of the compound obtained in Preparation 2 is placed in THF (7 ml). The solution obtained is cooled to 0°C. A solution of sodium metaperiodate in 7ml of water is then rapidly added. After 15 minutes at 0°C, the mixture is allowed to warm to room temperature, and stirring is continued for 6 hours. The precipitate formed is filtered off and rinsed with 7 ml of THF. A 2N solution of methylamine in 1.21 ml of THF and then 139.04 µl of acetic acid are added. After stirring for 5 minutes at room temperature, 199.63 mg of NaBH₃CN are added. The suspension obtained is stirred at room temperature for 20 hours. The precipitate formed is filtered off and rinsed with 50 ml of DCM. The filtrate is washed with 30 ml of saturated aqueous NaHCO₃ solution and then with 30 ml of saturated aqueous NaCl solution. The aqueous phases are extracted with 50 ml of DCM. The organic phases are combined, dried over MgSO₄, filtered and then evaporated to dryness under vacuum. The product obtained is purified by chromatography on a Merck cartridge (50 g of 15-40 µm silica), eluting with a 94/6 CHCl₃/MeOH mixture. 320 mg of the expected compound **7-a,** 77 mg of the other diastereoisomer **7-b** and 147 mg of a mixture of diastereoisomers are obtained.

### Compound 7-a:

### MS: method b

Retention time Tr (min) = 1.31; [M-H+HCO₂H]⁻: m/z 1166 (base peak).

**1H NMR spectrum (500 MHz, in ppm, DMSO-d₆ + CD₃COOD):** 0.81 (d, J=6.9 Hz, 3 H); 0.93 (d, J=6.8 Hz, 3 H); 0.95 to 1.02 (m, 9 H); 1.05 to 1.17 (m, 15 H); 1.24 (d, J=6.0 Hz, 3 H); 1.30 (d, J=6.6 Hz, 3 H); 1.74 (m, 7 H); 2.04 (m, 3 H); 2.18 (m, 5 H); 2.78 (s, 3 H); 2.79 (m, 1 H); 2.89 (d, J=14.8 Hz, 1 H); 2.93 (dd, J=2.5 and 8.0 Hz, 1 H); 3.03 (m, 2 H); 3.13 (m, 1 H); 3.35 (m, 2 H); 3.40 (s, 3 H); 3.45 (m, 1 H); 3.48 (s, 3 H); 3.51 (m, 2 H); 3.68 (m, 2 H); 3.82 (s, 3 H); 3.87 (m, 2 H); 3.94 (d, J=4.9 Hz, 1 H); 4.13 (d, J=5.7 Hz, 2 H); 4.45 (m, 2 H); 4.62 (m, 3 H); 5.12 (dd, J=4.5 and 8.4 Hz, 1 H); 7.28 (m, 6 H).

### Compound 7-b:

### MS: method b

Retention time Tr (min) = 1.32; [M-H+HCO₂H]⁻: m/z 1166 (base peak).

**1H NMR spectrum (500 MHz, in ppm, DMSO-d₆ + CD₃COOD):** 0.82 (d, J=6.9 Hz, 3 H); 0.94 (d, J=6.8 Hz, 3 H); 1.02 (m, 9 H); 1.08 (m, 6 H); 1.14 (m, 9 H); 1.22 (d, J=6.1 Hz, 3 H); 1.34 (d, J=6.8 Hz, 3 H); 1.75 (s, 3 H); 1.82 (m, 3 H); 1.90 to 1.95 (masked m, 1 H); 1.99 (m, 2 H); 2.09 (m, 1 H); 2.19 (m, 3 H); 2.35 (m, 2 H); 2.69 (m, 1 H); 2.79 (s, 3 H); 2.96 (m, 2 H); 3.04 (m, 2 H); 3.15 (dd, J=9.6 and 13.6 Hz, 1 H); 3.23 (d, J=13.6 Hz, 1 H); 3.32 (dd, J=4.8 and 9.7 Hz, 1 H); 3.40 (s, 3 H); 3.48 (s, 3 H); 3.53 (m, 2 H); 3.66 (m, 2 H); 3.70 (t, J=1.0 Hz, 1 H); 3.80 (m, 4 H); 3.94 (d, J=5.2 Hz, 1 H); 4.06 (broad s, 1 H); 4.14 (m, 2 H); 4.38 (m, 1 H); 4.47 (d, J=7.9 Hz, 1 H); 4.56 (m, 3 H); 5.15 (m, 1 H); 7.21 to 7.33 (m, 6 H).

### Alternative for the preparation of compound 7-a

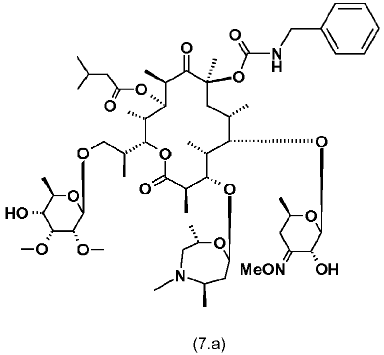

0.2 ml of CHCl₃, 12 mg of compound **8-a** of Example 8,108 µl of 0.1 M formic acid and 3 µl of formaldehyde are added together with stirring, under argon, and the mixture is heated for 30 minutes at 50°C. The reaction medium is neutralized with saturated aqueous sodium bicarbonate solution and extracted with DCM. The organic phase is dried over MgSO₄, filtered and then evaporated to dryness under vacuum. The residue obtained is purified by chromatography on silica (2.5 g of 15-40 µm SiOH) with a 95/5 to 90/10 CHCl₃/MeOH elution gradient. 6.2 mg of the expected product are obtained.

### Other alternative for the preparation of compound 7-a

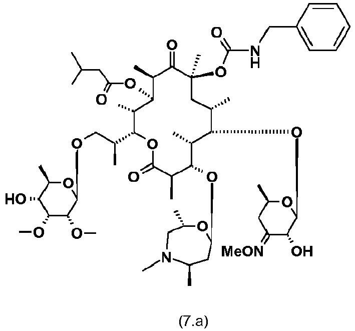

### Step 1

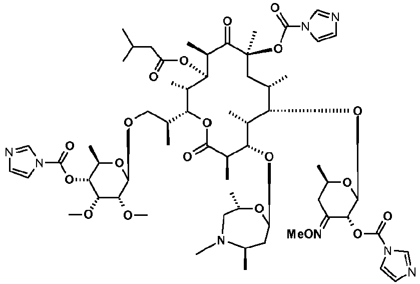

1.35 g of compound **1-a** of Example 1 and 1.11 g of N,N'-carbonyldiimidazole are placed in 8 ml of cyclohexane. The mixture is heated at 100°C for 35 minutes by microwave. The heterogeneous medium is taken up in 60 ml of DCM and washed with 40 ml of water and then with 40 ml of saturated NaCl solution. The aqueous phases are re-extracted with 60 ml of DCM. The organic phases are combined, dried over MgSO₄, filtered and then evaporated to dryness under vacuum. 1.7 g of the expected compound is obtained.

### MS: method e

Retention time Tr (min) = 3.67; [M+H]⁺: 1271

### Step 2

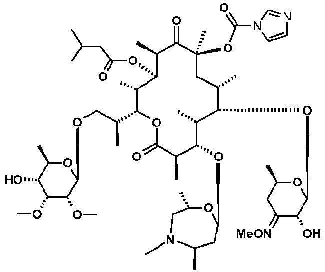

1.7 g of the compound prepared in step 1 are placed in 17 ml of THF. 6.84 ml of 1 M HCl are added. The mixture is stirred for 3 hours at room temperature. 50 ml of DCM are added and the resulting mixture is washed with saturated NaHCO₃ solution (20 ml) and then with saturated NaCl solution (20 ml). The aqueous phases are re-extracted with 50 ml of DCM. The organic phases are combined, dried over MgSO₄, filtered and then evaporated to dryness under vacuum. 1.48 g of the expected product are recovered.

### MS: method e

Retention time Tr (min) = 3.41; [M+H]⁺: 1083

### Step 3

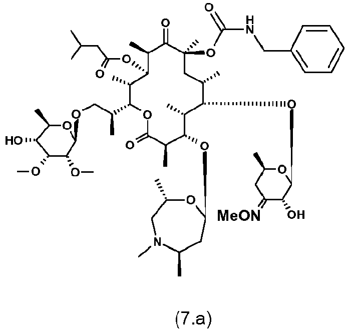

1 g of the product prepared above is placed in DMF (10 ml). Benzylamine (305.54 µl) and 1,8-diazabicyclo[5.4.0]undec-7-ene (168.87 µl) are added. The mixture is stirred at room temperature for 24 hours. The resulting mixture is extracted with 60 ml of EtOAc and washed with 30 ml of water and then with 30 ml of saturated NaCl solution. The aqueous phases are re-extracted with 60 ml of EtOAc. The organic phases are combined, dried over MgSO₄, filtered and then evaporated to dryness under vacuum. 1.1 g of a yellow oil are obtained. The product is purified by chromatography on a Merck cartridge (50 g of 15-40 µm SiOH), eluting with a 98/2 EtOAc/TEA mixture. 400 mg of the expected compound is obtained.

### EXAMPLE 8: Compound 8

**Compound 8-a:** (2R,3S,4R,5R,7S,9S,10S,11R,12S,13R)-7-[(benzylcarbamoyl)oxy]-12-{[(2S,5R,7R)-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate.

**Compound 8-b:** (2R,3S,4R,5R,7S,9S,10S,11R,12S,13R)-7-[(benzylcarbamoyl)oxy]-12-{[(2S,5S,7R)-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate.

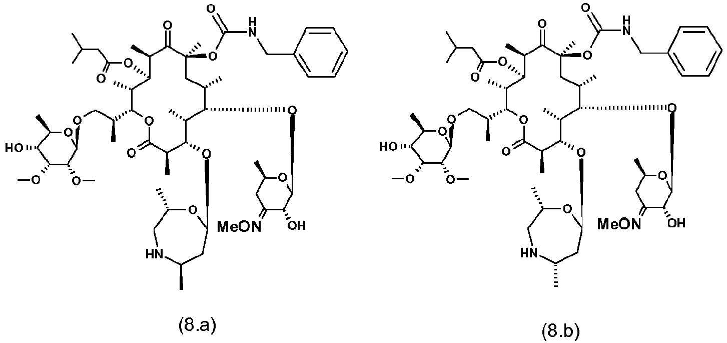

### Step 8.1:

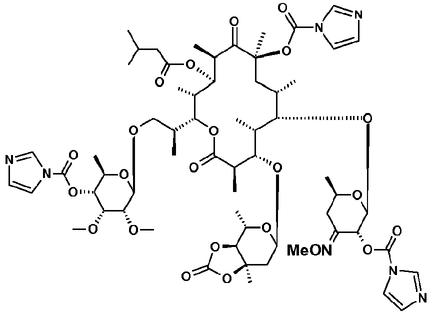

1 ml of toluene, 0.2 g of the product obtained in Preparation 1 and 196 mg of N,N'-carbonyldiimidazole are placed under argon. The reaction medium is heated for 3 hours at 80°C and then concentrated under vacuum. DCM is added and the resulting mixture is then washed with saturated aqueous NaCl solution. The organic phase is dried over MgSO₄, filtered and then evaporated to dryness under vacuum. The residue is purified by chromatography (10 g of 15-40 µm silica) with a 98/2 to 95/5 DCM/MeOH elution gradient. 112 mg of the expected compound is obtained.

### MS: method b

Retention time Tr (min) = 1.63; [M+H]⁺: 1300

### Step 8.2:

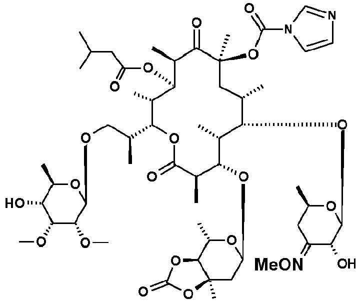

3 ml of THF, 240 mg of the macrolide prepared in step 8.1 and 369 µl of 1 M HCl are stirred together under argon. The pale yellow homogeneous medium is stirred overnight at room temperature. A further 369 µl of 1 M HCl are added and stirring is continued for 24 hours. The reaction medium is neutralized with saturated aqueous sodium bicarbonate solution. The resulting mixture is extracted with EtOAc. The organic phase is dried over MgSO₄, filtered and then evaporated to dryness under vacuum. 181 mg of the residue obtained are purified by chromatography (10 g of 15-40 µm silica) with a 50/50 to 70/30 EtOAc/heptane elution gradient. 87 mg of the expected compound is obtained.

### MS: method b

Retention time Tr (min) = 1.56; [M-H+HCO₂H]⁻: m/z 1156 (base peak).

### Step 8.3:

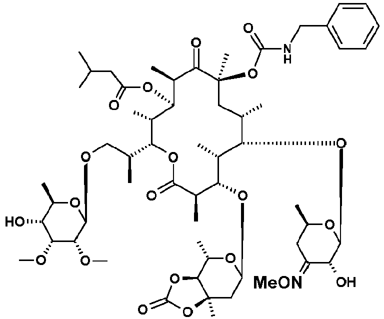

10 ml of DMF, 1 g of the compound obtained in step 8.2, 188 µl of 1,8-diazabicyclo [5.4.0]undec-7-ene and 138 µl of benzylamine are stirred together under argon. The homogeneous medium is stirred overnight at room temperature. 20 g of ice are added to the reaction medium and the resulting mixture is then extracted with 3 × 20 ml of EtOAc. The organic phases are combined and washed with 20 ml of saturated aqueous NaCl solution, and the organic phase is dried over MgSO₄, filtered and then evaporated to dryness under vacuum. 975 mg of residue are purified by chromatography (50 g of 15-40 µm silica), eluting with a 7/3 EtOAc/heptane mixture. 490 mg of the expected compound is obtained.

### MS: method b

Retention time Tr (min) = 1.68; [M-H+HCO₂H]⁻: m/z 1195 (base peak).

**1H NMR spectrum (500 MHz, in ppm, DMSO-d₆):** 0.79 (d, J=6.9 Hz, 3 H); 0.88 to 1.01 (m, 12 H); 1.05 (m, 6 H); 1.11 (m, 6 H); 1.14 (d, J=6.0 Hz, 3 H); 1.30 (d, J=5.5 Hz, 3 H); 1.52 (s, 3 H); 1.67 to 1.86 (m, 4 H); 1.71 (s, 3 H); 1.94 to 2.10 (m, 4 H); 2.15 (m, 4 H); 2.36 (dd, J=5.1 and 13.6 Hz, 1 H); 2.77 (m, 2 H); 2.92 (dd, J=2.5 and 8.0 Hz, 1 H); 3.01 (m, 2 H); 3.27 to 3.32 (masked m, 1 H); 3.37 (s, 3 H); 3.45 (s, 3 H); 3.52 (m, 2 H); 3.61 to 3.68 (m, 2 H); 3.80 (s, 3 H); 3.84 (d, J=6.9 Hz, 1 H); 3.95 (m, 2 H); 4.13 (m, 3 H); 4.45 (d, J=8.0 Hz, 1 H); 4.55 (d, J=9.9 Hz, 1 H); 4.65 (m, 2 H); 4.87 (d, J=7.1 Hz, 1 H); 4.99 (dd, J=5.4 and 8.4 Hz, 1H); 5.43 (m, 1 H); 7.26 (m, 5 H); 7.38 (broad t, J=6.0 Hz, 1 H).

### Step 8.4:

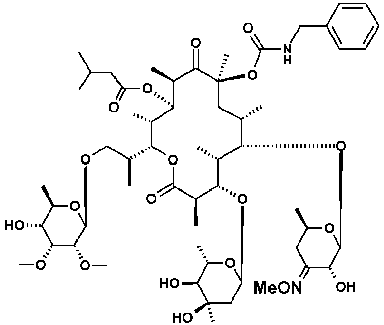

55 ml of MeOH, 300 mg of the macrolide prepared in the preceding step and 108 mg of K₂CO₃ are stirred together under argon. The reaction medium is stirred for 24 hours at room temperature, followed by addition of a further 72 mg of K₂CO₃. The reaction medium is stirred for a further 24 hours, and 30 ml of saturated aqueous NaCl solution are then added. The resulting mixture is extracted with EtOAc (3 × 100 ml). The organic phase is separated out after settling of the phases, dried over MgSO₄, filtered and then evaporated to dryness. 300 mg of the residue obtained are purified by chromatography (30 g of 15-40 µm silica), eluting with a 7/3 EtOAc/heptane mixture. 265 mg of the expected product are obtained.

### Step 8.5:

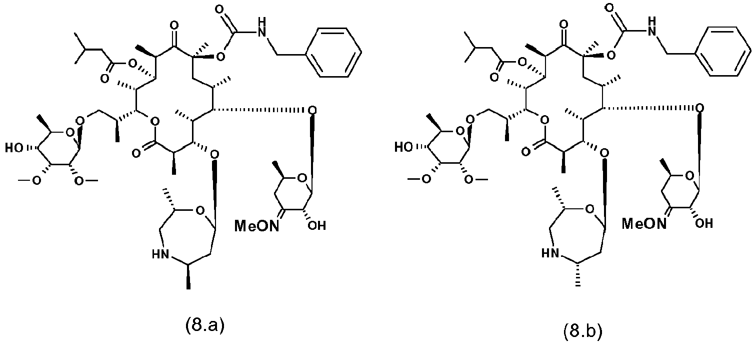

3.5 ml of THF and 340 mg of the compound obtained in the preceding step are stirred together under argon. The solution obtained is cooled to 0°C, followed by dropwise addition of an aqueous solution of 325 mg of sodium metaperiodate in 3.5 ml of water. Stirring is continued at 0°C for 10 minutes, and the mixture is then allowed to warm to room temperature. After stirring for 5 hours 30 minutes, the precipitate formed is filtered off and rinsed with 4 ml of THF. 64.8 mg of NH₄Cl are added to the filtrate obtained, followed, after stirring for 5 minutes at room temperature, by 95 mg of NaBH₃CN. The reaction medium is stirred at room temperature for 20 hours. The precipitate formed is filtered off and rinsed with DCM. The filtrate is washed with saturated sodium bicarbonate solution and then with aqueous NaCl solution. The aqueous phases are extracted with DCM. The organic phases are combined, dried over MgSO₄ filtered and then evaporated to dryness under vacuum. The 278 mg of residue are purified by chromatography on silica (20 g of 15-40 µ SiOH) with a 98/2 to 95/5 CHCl₃/MeOH elution gradient. 120 mg of diastereoisomer **8-a** and 14 mg of diastereoisomer **8-b** are obtained.

### Compound 8-a:

### MS: method b

Retention time Tr (min) = 1.3; [M-H+HCO₂H]⁻: m/z 1152 (base peak).

**1H NMR spectrum (500 MHz, in ppm, DMSO-d₆):** 0.78 (d, J=6.6 Hz, 3 H); 0.91 (d, J=6.9 Hz, 3 H); 0.95 (d, J=7.1 Hz, 3 H); 0.99 (d, J=6.6 Hz, 6 H); 1.03 to 1.12 (m, 21 H); 1.75 (m, 8 H); 1.99 (m, 4 H); 2.15 (m, 3 H); 2.50 (masked m, 1 H); 2.68 to 2.94 (m, 5 H); 3.01 (m, 2 H); 3.29 (masked m, 1 H); 3.38 (s, 3 H); 3.45 (s, 3 H); 3.52 (m, 1 H); 3.61 to 3.90 (m, 6 H); 3.80 (s, 3 H); 4.10 (m, 3 H); 4.45 (d, J=7.7 Hz, 1 H); 4.62 (m, 3 H); 4.86 (d, J=7.1 Hz, 1 H); 4.95 (m, 1 H); 5.26 (d, J=4.4 Hz, 2 H); 7.20 to 7.32 (m, 5 H); 7.36 (t, J=6.0 Hz, 1 H).

### Compound 8-b:

### MS: method b

Retention time Tr (min) = 1.12; [M-H+HCO₂H]⁻: m/z 1152 (base peak).

**1H NMR spectrum (500 MHz, in ppm, DMSO-d₆):** 0.78 (d, J=6.9 Hz, 3 H); 0.91 (d, J=6.6 Hz, 3 H); 0.98 (m, 9 H); 1.03 to 1.16 (m, 21 H); 1.69 to 2.20 (m, 12 H); 1.72 (s, 3 H); 2.71 (m, 3 H); 2.92 (m, 2 H); 3.01 (m, 3 H); 3.10 (m, 1 H); 3.34 (masked m, 1 H); 3.38 (s, 3 H); 3.45 (s, 3 H); 3.52 (m, 1 H); 3.63 (dd, J=4.5 and 9.7 Hz, 1 H); 3.67 (broad s, 1 H); 3.71 to 3.81 (m, 3 H); 3.79 (s, 3 H); 3.87 (t, J=4.7 Hz, 1 H); 4.09 (m, 3 H); 4.45 (d, J=8.0 Hz, 1 H); 4.60 (m, 3 H); 4.83 (d, J=6.9 Hz, 1 H); 5.01 (m, 1 H); 5.14 (d, J=3.6 Hz, 1 H); 7.20 to 7.35 (m, 6 H).

**Table 1: Structures and analyses of compounds prepared according to one of the processes described in Examples 1 to 8 above and 9 to 11 below.**

| Compound | CHEMISTRY | LCSM | | |
|---|---|---|---|---|
| | | Méth | Tr | MS |
| 9 | 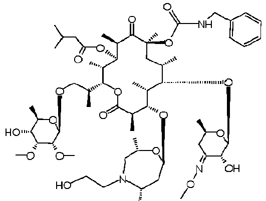 | a | 1,02 | [M-H+HCO2H]- : m/z 1196 (base peak) |
| 10 | 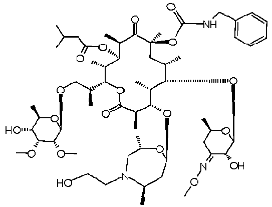 | a | 1,01 | [M-H+HCO2H]- : m/z 1196 (base peak) |
| 11 | 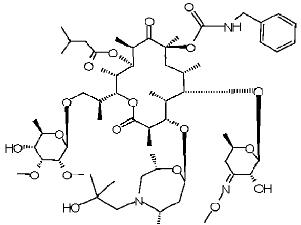 | a | 1,04 | [M-H+HCO2H]- : m/z 1224 (base peak) |
| 12 | 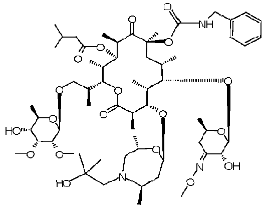 | a | 1,03 | [M-H+HCO2H]- m/z 1224 (base peak) |
| 13 | 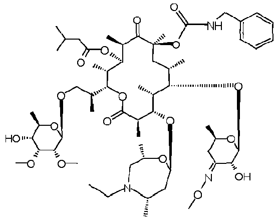 | b | 1,04 | [M-H+HCO2H]- : m/z 1180 (base peak) |
| 14 | 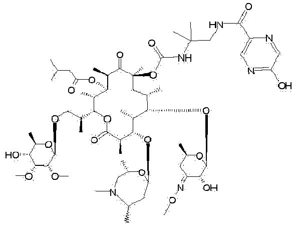 | b | 1,19 | [M+H]+ : 1225 |
| 15 | 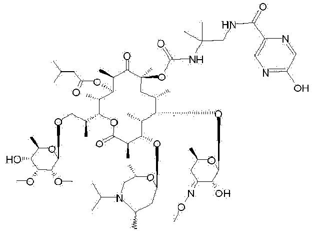 | b | 1,2 1,25 | [M+H]+ : 1253, mixture of isomers |
| 16 | 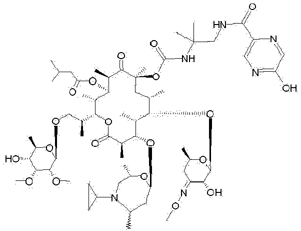 | b | 1,2 | [M+H]+ : 1251 |
| 17 | 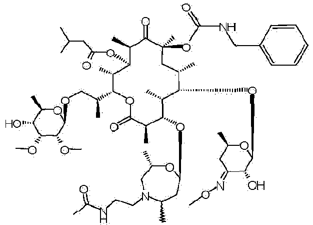 | b | 1,31 | [M-H+HCO2H]- : m/z 1237 (base peak) |
| 18 | 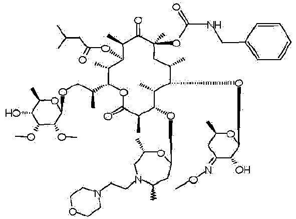 | b | 1,32 | [M+H]+ : 1221 |
| 19 | 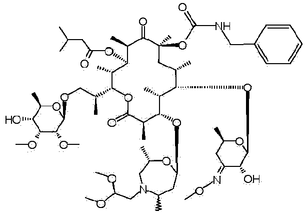 | b | 1,36 | [M-H+HCO2H]- : m/z 1240 (base peak) |
| 20 | 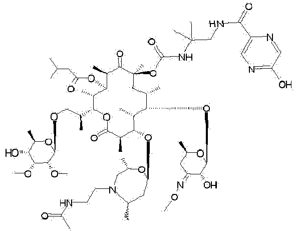 | b | 1,16 | [M+H]+ ; 1296 |
| 21 | 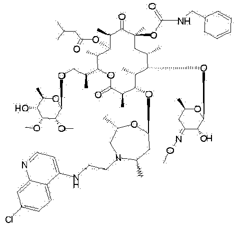 | b | 1,17-1,19 | m/z 306 (base peak) - mixture of isomers |
| 22 | 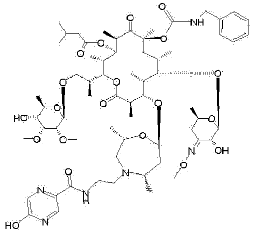 | b | 1,26 | [M+H]+ : 1273 |
| 23 | 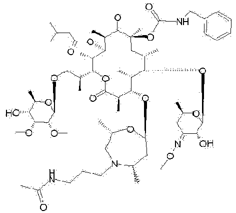 | b | 1,29 | m/z 1251 (base peak)-mixture of isomers |
| 24 | 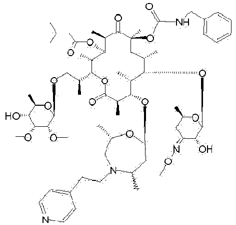 | b | 1,2 | m/z : 207 (base peak) ; [M-H+HCO2H]- : m/z 1257 |
| 25 | 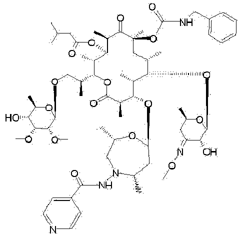 | b | 1,53 | [M-H+HCO2H]- : m/z 1272 (base peak) |
| 26 | 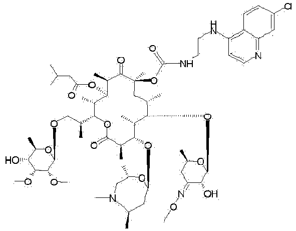 | b | 1,04 | [M+2H]2+: 619 (base peak); [M-H+HCO2H]-: m/z 1280 |
| 27 | 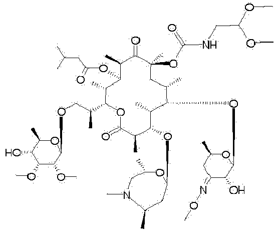 | b | 1,21 | [M-H+HCO2H]- : m/z 1164 (base peak) |
| 28 | 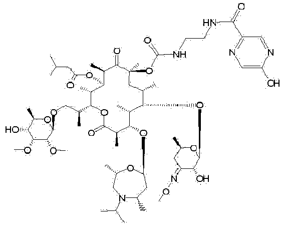 | b | 1,1 | [M+H]+ : 1225 |
| 29 | 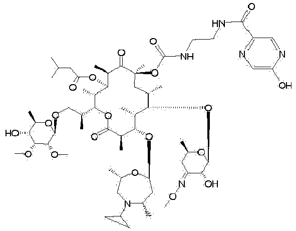 | b | 1,1 - 1,21 | [M+H]+ : 1223 mixture of isomers |
| 30 | 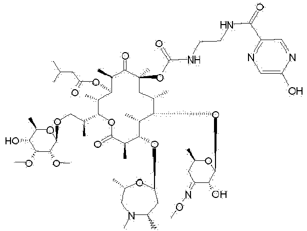 | b | 1,09 | [M+H]+ : 1197 |
| 31 | 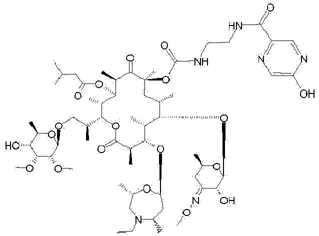 | b | 1,11 | [M+H]+ : 1211 |
| 32 | 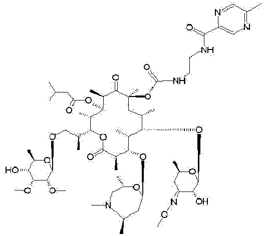 | b | 1,17 | [M-H+HC02H]- : m/z 1240 |
| 33 | 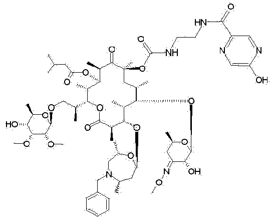 | b | 1,18 | [M+H]+ : 1273 |
| 34 | 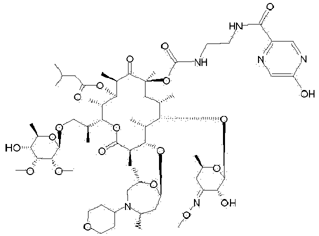 | b | 1,1 | [M+H]+: 1267 |
| 35 | 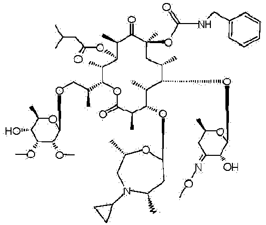 | b | 1,35 | [M-H+HC02H]-: m/z 1192 |
| 36 | 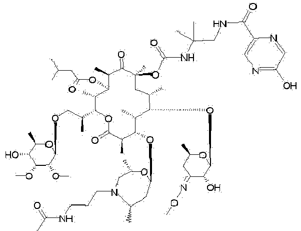 | b | 1,16-1,17 | [M+H]+:1311 mixture of isomers |
| 37 | 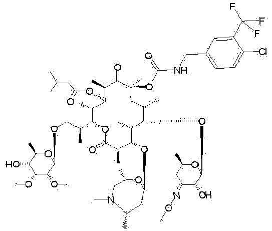 | b | 1,38 | [M-H+HCO2H]-: m/z 1268 (base peak) |
| 38 | 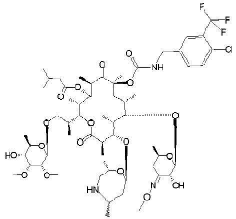 | b | 1,35 | [M-H+HCO2H]-: m/z 1254 (base peak) |
| 39 | 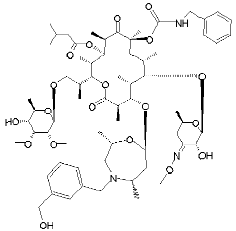 | b | 1,3 | [M-H+HCO2H]-: m/z 1272 (base peak) |
| 40 | 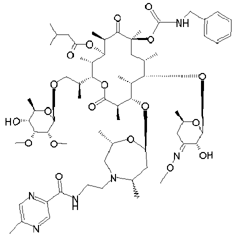 | b | 1,33 (d) | m/z : 265 (base peak) ; [M-H+HCO2H]- : m/z 1315 - mixture of isomers |
| 41 | 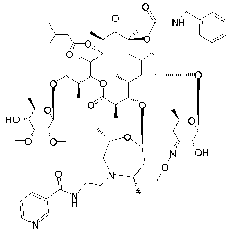 | b | 1,29 (d) | m/z : 250 (base peak) ; [M-H+HCO2H]- : m/z 1300 - mixture of isomers |
| 42 | 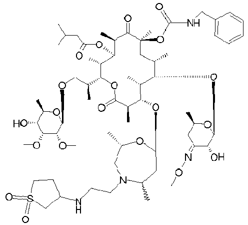 | b | 1,25 | m/z : 263 (base peak) ; [M-H+HCO2H]- : m/z 1313 |
| 43 | 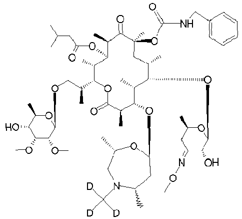 | b | 1,24 | [M-H+HCO2H]- : m/z 1169 (base peak) |
| 44 | 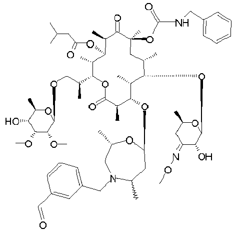 | b | 1,30-1,32 | [M+H]+ : 1226 mixture of isomers |
| 45 | 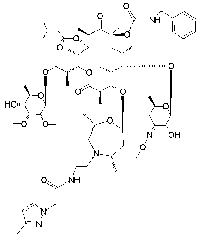 | b | 1,23 | m/z : 267 (base peak) ; [M-H+HCO2H]- : m/z 1317 |
| 46 | 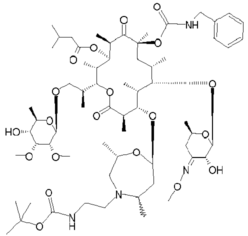 | b | 1,29 | [M-H+HCO2H]- : m/z 1295 (base peak) |
| 47 | 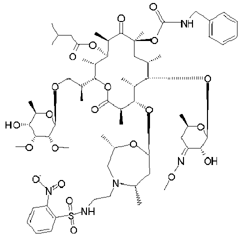 | b | 1,33 | m/z : 330 (base peak) |
| 48 | 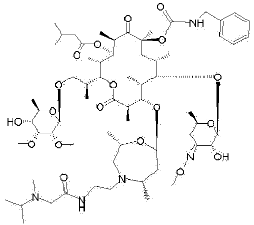 | b | 0,99 | m/z : 258 (base peak) ; [M-H+HCO2H]- : m/z 1308 |
| 49 | 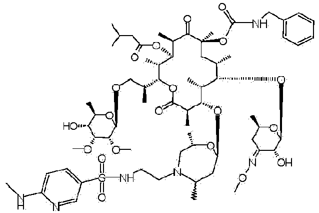 | b | 1,28 | [M-H+HC02H]- : m/z 1365 |
| 50 | 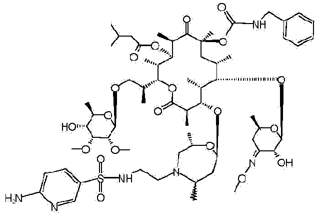 | b | 1,15 (d) | [M-H+HCO2H]- : m/z 1351 - mixture of isomers |
| 51 | 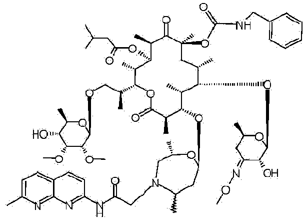 | b | 1,25-1,29 | m/z : 327 (base peak) ; m/z : 301 (base peak) |
| 52 | 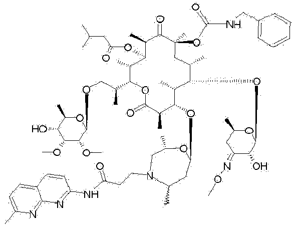 | b | 1,14 | m/z : 315 (base peak) |
| 53 | 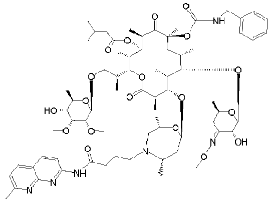 | b | 1,12 | m/z : 315 (base peak) |
| 54 | 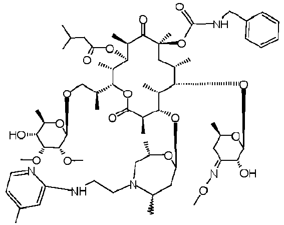 | d | 2,20-2,22 | [M-H+HCO2H]- : m/z 1286 (base peak) - mixture of isomers |
| 55 | 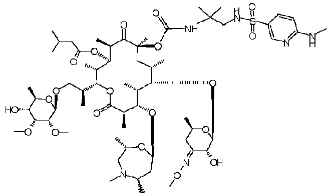 | b | 1,06 | [M+2H]2+: 637 (base peak) |
| 56 | 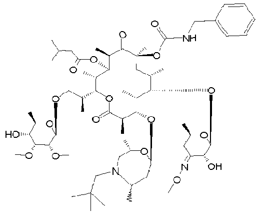 | d | 2,36-2,39 | [M-H+HCO2H]- : m/z 1222 (base peak) - mixture of isomers |
| 57 | 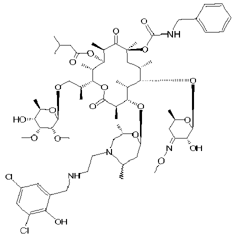 | b | 2,38-2,41 | m/z : 345 (base peak) - mixture of isomers |
| 58 | 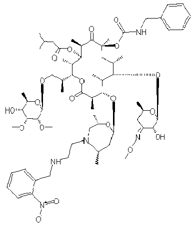 | b | 1,21-1,23 | m/z 306 (base peak); [M-H+HCO2H]- : m/z 1330 - mixture of isomers |
| 59 | 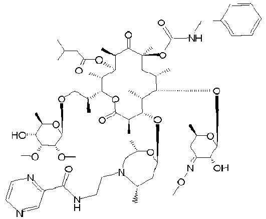 | b | 1,16 | [M-H+HCO2H]- : m/z 1301 (base peak) |
| 60 | 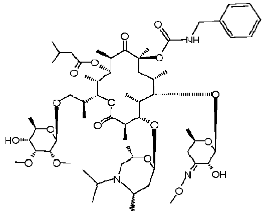 | b | 1,18 | [M-H+HCO2H]- : m/z 1194 (base peak) |
| 61 | 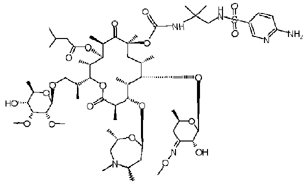 | b | 1 | [M+2H]2+: 630 (base peak) |
| 62 | 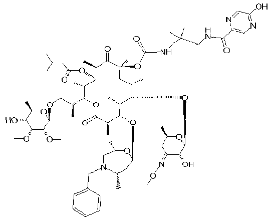 | b | 1,12 | [M+H]+ : 1301 |
| 63 | 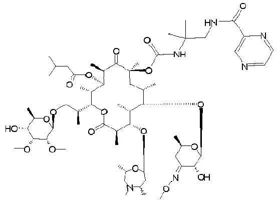 | b | 1,08 | m/z : 1035 (base peak) |
| 64 | 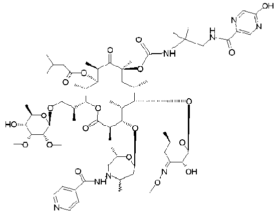 | b | 1,33-1,34 | [M+2H]2+: 666 - m/z :248 (base peak) - mixture of isomers |
| 65 | 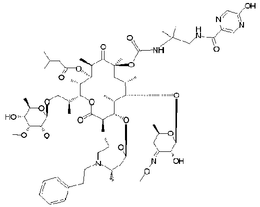 | b | 1,13 | [M+H]+ : 1315 |
| 66 | 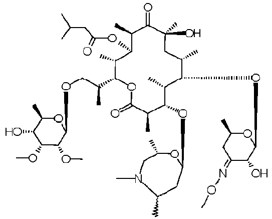 | b | 1,16 | [M-H+HCO2H]- : m/z 1033 (base peak) |
| 67 | 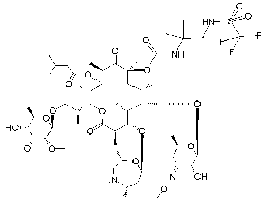 | b | 1,19 | [M+H]+ : 1235 |
| 68 | 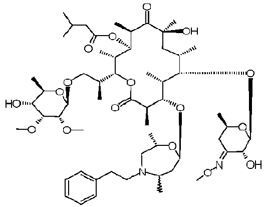 | b | 1,19-1,20 | [M-H+HCO2H]- : m/z 1123 (base peak) - mixture of isomers |
| 69 | 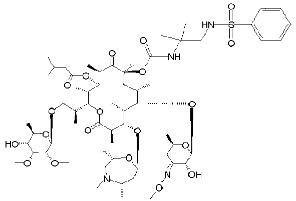 | a | 1,15 | [M+H]+ : 1243 |
| 70 | 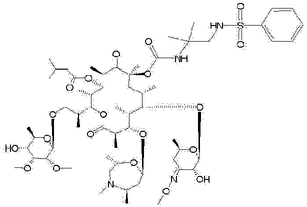 | a | 1,15 | [M+H]+ : 1243 |
| 71 | 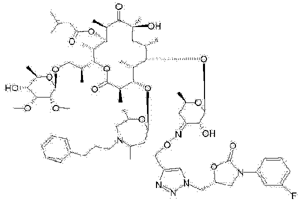 | a | 1,23-1,24 | [M+H]+ : 1354 mixture of isomers |

### EXAMPLE 9: Compound 70

(2R,3S,4R,5R,7S,9S,10S,11R,12S,13R)-2-((S)-1-(((2R,3R,4R,5R,6R)-5-hydroxy-3,4-dimethoxy-6-methyltetrahydro-2H-pyran-2-yl)oxy)propan-2-yl)-10-(((2S,3R,6R)-3-hydroxy-4-(methoxyimino)-6-methyltetrahydro-2H-pyran-2-yl)oxy)-3,5,7,9,11,13-hexamethyl-7-(((2-methyl-1-((phenylsulfonamido)propan-2-yl)carbamoyl)oxy)-6,14-dioxo-12-(((2S,5R,7R)-2,4,5-trimethyl-1,4-oxazepan-7-yl)oxy)oxacyclotetradecan-4-yl 3-methylbutanoate.

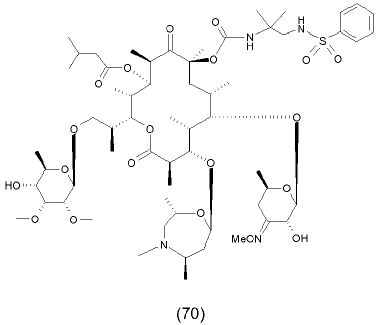

### Step 9.1

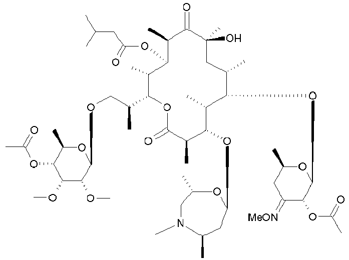

3 g of compound **1-a** obtained in Example 1 are placed in pyridine (30 ml). Acetic anhydride (2.88 ml) is added. The mixture is stirred at room temperature for 40 hours. The resulting mixture is concentrated under vacuum, and extracted with 3 × 60 ml of DCM, washed with 40 ml of 1 M HCl, then with saturated aqueous NaHCO₃ solution and finally with saturated NaCl solution. The organic phases are combined, dried over MgSO₄, filtered and then evaporated to dryness. 3.5 g of the expected product are obtained in the form of a white powder.

### MS: method e

Retention time Tr (min) = 4.01; [M+H]⁺: 1073

### Step 9.2

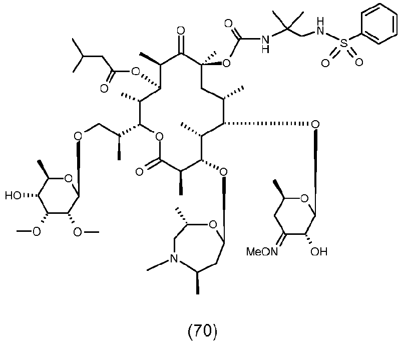

### Step 9.2.a:

4.2 g of the compound prepared in step 9.1 are placed in DCM (150 ml). Pyridine (5.43 ml) is added and the mixture is cooled to 0°C. Trichloromethyl chloroformate (diphosgene) (842.40 µl) is added and stirring is continued for 3 hours at 0°C. 4-Dimethylaminopyridine (507.03 mg) is added, the mixture is then allowed to warm to room temperature and stirring is continued overnight. The resulting mixture is evaporated to dryness under vacuum. The crude reaction product is used as obtained for the following stage.

### Step 9.2.b: condensation of the amine

DMF (70 ml) is added to the crude reaction medium obtained in the above step. A dark brown suspension is obtained. TEA (4.91 ml) is added in a single portion, followed by N-(2-amino-2-methylpropyl)benzenesulfonamide hydrochloride (2.80 g) in a single portion. The reaction mixture is stirred magnetically at room temperature for 24 hours. 400 ml of EtOAc are added. The mixture is washed with 200 ml of water and then with 200 ml of saturated aqueous NaCl solution. The aqueous phases are re-extracted with 400 ml of EtOAc. The organic phases are combined, dried over MgSO₄, filtered through a sinter funnel and concentrated under reduced pressure. 8 g of a brown oil are recovered.

### Step 9.2.c: deprotection of the alcohols

MeOH (40 ml) is added to the 8 g of brown oil obtained above. An orange solution is obtained. Potassium carbonate (1.30 g) is added in a single portion. The reaction mixture is stirred magnetically at room temperature for 2 hours 30 minutes. 150 ml of DCM are added. The mixture is washed with 75 ml of water and then with 75 ml of saturated aqueous NaCl solution. The aqueous phases are re-extracted with 150 ml of DCM. The organic phases are combined and then dried over MgSO₃ and finally filtered through a sinter funnel. The filtrate is evaporated to dryness, and 3.5 g of an orange foam are recovered. The product is purified by preparative HPLC under the following conditions:
▪ Apparatus: Waters 4000
▪ Stationary phase: Kromasil C18 10 µm 300 x 50 mm
▪ Mobile phase: B: 70/30 v/v acetonitrile/H₂O + 10 mM of ammonium acetate
▪ Flow rate: 120 ml/min
▪ UV detection: 210 nm · Cell length: 2.5 mm

After evaporation and lyophilization, the following is obtained:
800 mg in the form of a yellow powder corresponding to the expected product.

### MS: method b

Retention time Tr (min) = 1.15; [M+H]⁺: 1243

**1 H NMR spectrum (500MHz, in ppm, DMSO-d₆):** 0.77 (d, J=6.6 Hz, 3 H); 0.88 to 0.97 (m, 12 H); 1.00 to 1.15 (m, 27 H); 1.62 to 1.82 (m, 4 H); 1.67 (s, 3 H); 1.90 to 2.05 (m, 4 H); 2.09 to 2.20 (m, 4 H); 2.19 (s, 3 H); 2.39 (dd, J=9.7 and 13.9 Hz, 1 H); 2.59 (m, 1 H); 2.68 to 2.79 (m, 3 H); 2.83 to 2.93 (m, 3 H); 2.98 (broad q, J=6.6 Hz, 1 H); 3.03 (m, 1 H); 3.30 (m, 1 H); 3.37 (s, 3 H); 3.45 (s, 3 H); 3.52 (m, 1 H); 3.59 to 3.69 (m, 3 H); 3.76 (broad d, J=4.1 Hz, 1 H); 3.80 (s, 3 H); 3.83 (m, 1 H); 3.88 (t, J=4.8 Hz, 1 H); 4.28 (m, 1 H); 4.45 (d, J=8.0 Hz, 1 H); 4.59 (d, J=9.9 Hz, 1 H); 4.68 (m, 2 H); 4.87 (d, J=7.1 Hz, 1 H); 4.91 (dd, J=2.6 and 9.2 Hz, 1 H); 5.27 (d, J=4.8 Hz, 1 H); 6.22 (s, 1 H); 7.51 (broad t, J=6.6 Hz, 1 H); 7.58 (t, J=7.5 Hz, 2 H); 7.63 (t, J=7.5 Hz, 1 H); 7.78 (d, J=7.5 Hz, 2 H).

### EXAMPLE 10: Compound 72

(2*R*,3*S*,4*R*,5*R*,7*S*,9*S*,10*S*,11*R*,12*S*,13*R*)-10-{[(2*S*,3*R*,6*R*)-4-{[(1-{[(5*R*)-3-(3-fluorophenyl)-2-oxo-1,3-oxazolidin-5-yl]methyl}-1*H*-1,2,3-triazol-4-yl)methoxy]imino}-3-hydroxy-6-methyltetrahydro-2*H*-pyran-2-yl]oxy}-7-hydroxy-2-(1-{[(2*R*,3*R*,4*R*,5*R*,6*R*)-5-hydroxy-3,4-dimethoxy-6-methyltetrahydro-2*H*-pyran-2-yl]oxy}propan-2-yl)-3,5,7,9,11,13-hexamethyl-6,14-dioxo-12-{[(2*S*,5*R*,7*R*)-2,4,5-trimethyl-1,4-oxazepan-7-yl]oxy}oxacyclotetradecan-4-yl 3-methylbutanoate.

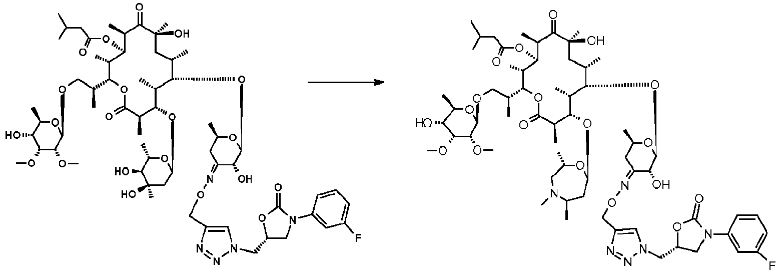

200 mg of the compound obtained in Preparation 5 are placed in 2 ml of THF, the solution is cooled to 0°C and 172.51 mg of sodium metaperiodate dissolved in 2 ml of water are then added. The mixture is allowed to warm to room temperature, and stirring is continued for 4 hours. The precipitate is filtered off and rinsed with 0.5 ml of THF.

400 µl of a 2 M solution of methylamine in THF, then 46 µl of acetic acid and finally 63.5 mg of NaBH₃CN are added to the filtrate. The suspension obtained is stirred at room temperature for 18 hours. The suspension is taken up in 40 ml of DCM. The mixture is washed with 20 ml of saturated aqueous NaHCO₃ solution and then with 20 ml of saturated aqueous NaCl solution. The organic phase is dried over MgSO₄, filtered and finally concentrated under reduced pressure. The crude mixture is purified by chromatography on a Merck cartridge (10 g of 15-40 µm silica), eluting with a 92/8 CHCl₃/MeOH mixture.

41 mg of the expected product and 5 mg of the other diastereoisomer are recovered.

### MS: method b

ES⁻: [M-H+HCO₂H]⁻: m/z 1293

**1H NMR spectrum (500 MHz, in ppm, DMSO-d₆):** 0.79 (d, J=6.8 Hz, 3 H); 0.93 (d, J=6.8 Hz, 6 H); 0.95 to 1.00 (m, 9 H); 1.02 (d, J=6.6 Hz, 3 H); 1.05 to 1.14 (m, 15 H); 1.24 (s, 3 H); 1.48 (m, 1 H); 1.69 to 1.80 (m, 2 H); 1.83 to 2.07 (m, 6 H); 2.11 to 2.21 (m, 3 H); 2.17 (s, 3 H); 2.35 (dd, J=9.4 and 14.1 Hz, 1 H); 2.56 (m, 1 H); 2.69 (d, J=12.6 Hz, 1 H); 2.75 (m, 1 H); 2.83 (dd, J=3.0 and 16.5 H, 1 H); 2.92 (dd, J=2.6 and 7.9 Hz, 1 H); 3.03 (m, 1 H); 3.12 (broad q, J=6.8 Hz, 1 H); 3.30 (partially masked m, 1 H); 3.38 (s, 3 H); 3.45 (s, 3 H); 3.52 (m, 1 H); 3.59 to 3.73 (m, 4 H); 3.82 to 3.94 (m, 3 H); 4.22 to 4.28 (m, 2 H); 4.31 (s, 1 H); 4.45 (d, J=7.9 Hz, 1 H); 4.66 (d, J=9.7 Hz, 1 H); 4.68 (d, J=4.7 Hz, 1 H); 4.74 (d, J=9.4 Hz, 1 H); 4.82 (d, J=5.3 Hz, 2 H); 4.86 (d, J=7.2 Hz, 1 H); 4.91 (dd, J=3.1 and 9.4 Hz, 1 H); 5.11 (s, 2 H); 5.15 (m, 1 H); 5.30 (d, J=4.7 Hz, 1 H); 6.97 (dt, J=2.3 and 8.4 Hz, 1 H); 7.27 (dd, J=1.5 and 8.4 Hz, 1 H); 7.40 to 7.47 (m, 2 H); 8.21 (s, 1 H).

### EXAMPLE 11: Compound 88

(2R,3S,4R,5R,7S,9S,10S,11R,12S,13R)-2-((S)-1-(((2R,3R,4R,5R,6R)-5-hydroxy-3,4dimethoxy-6-methyltetrahydro-2H-pyran-2-yl)oxy)propan-2-yl)-10-(((2S,3R,6R)-3-hydroxy-4-(methoxyimino)-6-methyltetrahydro-2H-pyran-2-yl)oxy)-7-((methoxycarbonyl)oxy)-3,5,7,9,11,13-hexamethyl-6,14-dioxo-12-(((2S,7R)-2,4,5-trimethyl-1,4-oxazepan-7yl)oxy)oxacyclotetradecan-4-yl 3-methylbutanoate.

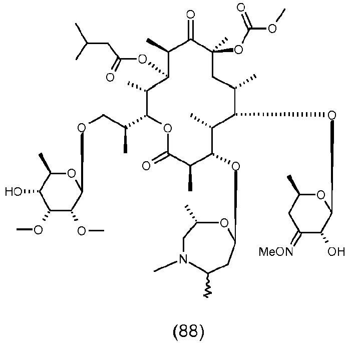

### Step 11.1

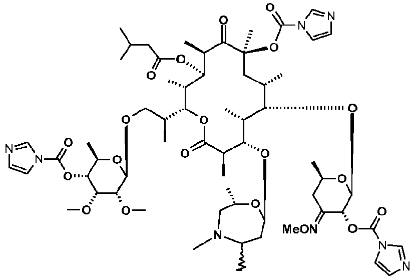

556 mg of the mixture of diastereoisomers obtained in Example 1 and 456 mg of N,N'-carbonyldiimidazole are placed in cyclohexane (3.3 ml). The mixture is heated at 100°C for 35 minutes by microwave. The heterogeneous medium is taken up in 30 ml of DCM and washed with 20 ml of water and then with 20 ml of saturated NaCl solution. The aqueous phases are re-extracted with 30 ml of DCM. The organic phases are combined, dried over MgSO₄ filtered and then evaporated to dryness under vacuum. 710 mg of the expected product are obtained.

### MS: method a

Retention time Tr (min) = 1.19; [M+H]⁺: 1271

**1H NMR** (in ppm, DMSO-d₆) - Brüker spectrometer: 0.79 (d, J=6.9 Hz, 3 H); 0.85 (d, J=7.1 Hz, 3 H); 0.90 to 0.95 (m, 12 H); 1.01 (d, J=6.9 Hz, 3 H); 1.06 to 1.14 (m, 9 H); 1.17 (d, J=6.3 Hz, 3 H); 1.25 (d, J=6.3 Hz, 3 H); 1.75 to 1.87 (m, 3 H); 1.92 (s, 3 H); 1.94 to 2.18 (m, 9 H); 2.20 (s, 3 H); 2.47 (m, 1 H); 2.65 to 2.81 (m, 3 H); 3.07 to 3.19 (m, 3 H); 3.37 (m, 1 H); 3.41 (s, 3 H); 3.44 (s, 3 H); 3.63 to 3.78 (m, 3 H); 3.72 (s, 3 H); 3.93 to 4.00 (m, 2 H); 4.10 to 4.17 (m, 2 H); 4.56 to 4.64 (m, 3 H); 4.69 (d, J=9.6 Hz, 1 H); 4.84 (m, 1 H); 5.00 (d, J=7.4 Hz, 1 H); 5.24 (d, J=7.4 Hz, 1 H); 7.10 (dd, J=0.8 and 1.6 Hz, 1 H); 7.12 (dd, J=0.8 and 1.6 Hz, 1 H); 7.15 (broad s, 1 H); 7.46 to 7.47 (t, J=1.6 Hz, 1 H); 7.62 (t, J=1.6 Hz, 1 H); 7.64 (t, J=1.6 Hz, 1 H); 8.12 (broad s, 1 H); 8.28 (broad s, 1 H); 8.30 (broad s, 1 H).

### Step 11.2

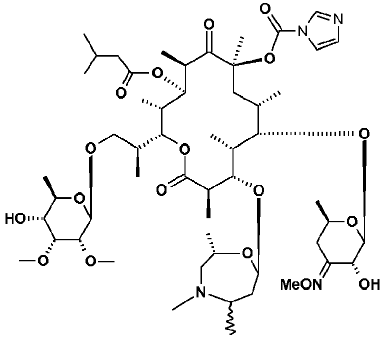

500 mg of the compound isolated in step 11.1 are placed in THF (5 ml). 1 M hydrochloric acid (1.97 ml) is added. The mixture is stirred for 3 hours at room temperature. 50 ml of DCM are added and the resulting mixture is washed with saturated NaHCO₃ solution (20 ml) and then with saturated NaCl solution (20 ml). The aqueous phases are re-extracted with 50 ml of DCM. The organic phases are combined, dried over MgSO₄, filtered and then evaporated to dryness under vacuum. 420 mg of the expected product are recovered.

### MS: method a

Retention time Tr (min) = 1.02; [M+H]⁺: 1083

### Step 11.3

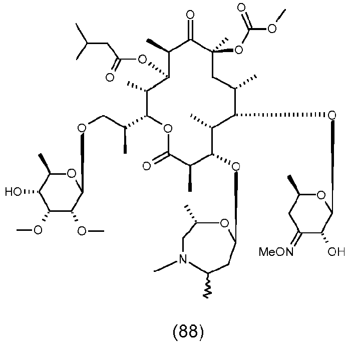

50 mg of the compound prepared in step 11.2 are placed in MeOH (1 ml). Potassium carbonate (32.22 mg) is added and the mixture is stirred at room temperature for 4 hours. 15 ml of DCM are added and the mixture is washed with water and then with saturated NaCl solution. The aqueous phases are extracted with 15 ml of DCM. The organic phases are combined, dried over MgSO₄, filtered and then evaporated to dryness under vacuum. 57 mg of a lacquer are obtained, which product is purified by chromatography on a Merck cartridge (2.5 g of 15-40 µm SiOH), eluting with a 97/3 EtOAc/TEA mixture. The fraction of Rf 0.35/0.45 (core fraction) is recovered, i.e. 14 mg of the expected compound.

### MS: method a

Retention time Tr (min) = 1.07; [M+H]⁺: 1047

**1H NMR spectrum (500 MHz, in ppm, DMSO-d₆):** 0.76 - 0.81 (m, 3 H); 0.89 - 0.94 (m, 6 H); 0.96 (d, J=6.6 Hz, 6 H); 0.99 - 1.17 (m, 21 H); 1.73 - 1.82 (m, 6 H); 1.85 (m, 1 H); 1.91 - 2.06 (m, 5 H); 2.11 - 2.21 (m, 6 H); 2.38 (dd, J=14.0, 9.6 Hz, 1 H); 2.53 - 2.66 (m, 1 H); 2.66 - 2.77 (m, 2 H); 2.81 - 2.89 (m, 1 H); 2.92 (dd, J=7.8, 2.6 Hz, 1 H); 2.98 - 3.06 (m, 2 H); 3.27 - 3.31 (masked m, 1 H); 3.38 (s, 3 H); 3.45 (s, 3 H); 3.48 - 3.56 (m, 1 H); 3.58 - 3.71 (m, 6 H); 3.74 - 3.78 (m, 1 H); 3.80 (s, 3 H); 3.82 - 3.92 (m, 2 H); 4.26 (d, J=6.3 Hz, 1 H); 4.45 (d, J=8.0 Hz, 1 H); 4.59 (d, J=10.2 Hz, 1 H); 4.63 - 4.75 (m, 2 H); 4.81 - 4.94 (m, 2 H); 5.29 - 5.34 (m, 1 H).

**Table 2: Structures and analyses of compounds prepared according to one of the processes described in Examples 1 to 8 and 9 to 11 above.**

| Compound | CHEMISTRY | LCMS | | |
|---|---|---|---|---|
| | | Meth. | Tr | MS |
| 73 | 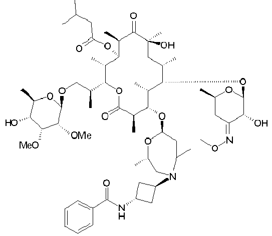 | f | 1.237 | [M+H]+: 1148.5 mixture of isomers |
| 74 | 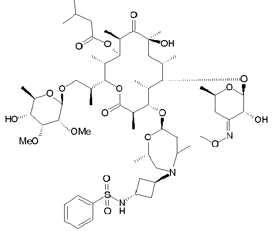 | f | 1.12 | [M+H]+: 1184.5 mixture of isomers |
| 75 | 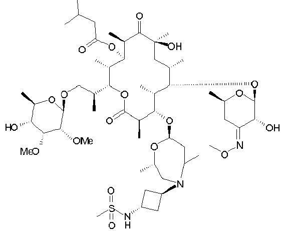 | h | 0.812 | [M+H]+: 1122.6 mixture of isomers |
| 76 | 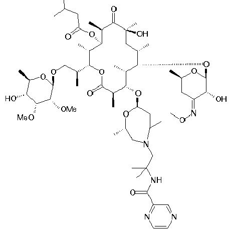 | f | 1.209 | [M+H]+: 1152.6 mixture of isomers |
| 77 | 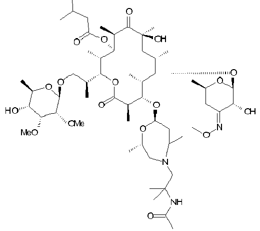 | g | 0.881 | [M+H]+: 1083.3 mixture of isomers |
| 78 | 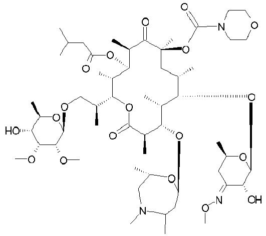 | b | 1.04 | [M+H]+: 1102 mixture of isomers |
| 79 | 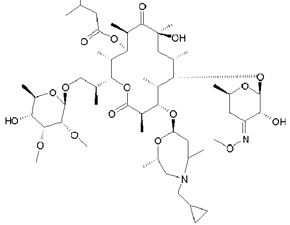 | f | 1.087 | [M+H]+: 1029.6 mixture of isomers |
| 80 | 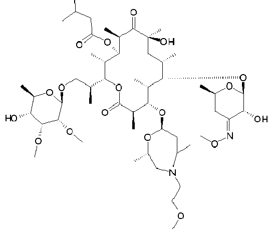 | f | 1.078 | [M+H]+: 1033.5 mixture of isomers |
| 81 | 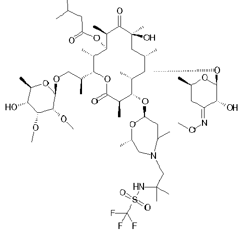 | f | 1.26 | [M+H]+: 1178.6 mixture of isomers |
| 82 | 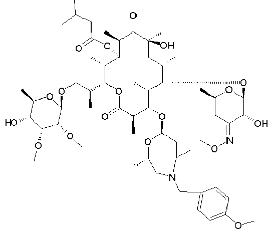 | g | 0.899 | [M+H]+: 1095.5 mixture of isomers |
| 83 | 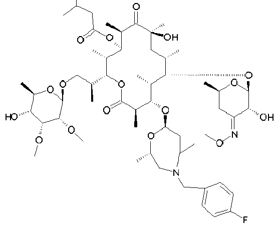 | g | 1.026 | [M+H]+: 1083.7 mixture of isomers |
| 84 | 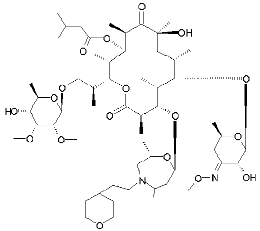 | b | 1.11 | [M+H]+: 1087 mixture of isomers |
| 85 | 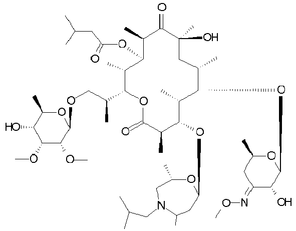 | b | 1.13 | [M+H]+: 1031 mixture of isomers |
| 86 | 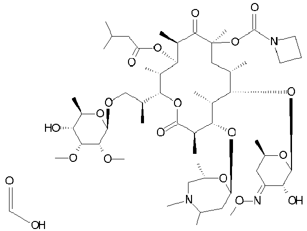 | f | 1.139 | [M+H]+: 1190.8 mixture of isomers |
| 87 | 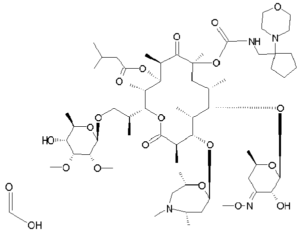 | g | 0.63 | [M+H]+: 1200.4 |
| 88 | 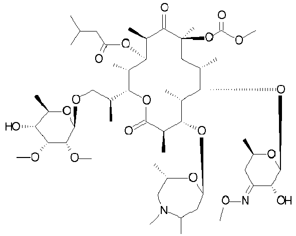 | b | 1.07 | [M+H]+: 1047 mixture of isomers |
| 89 | 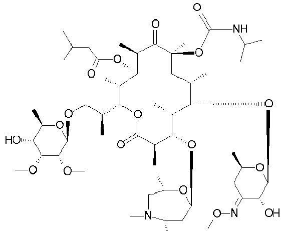 | i | 1.221 | [M+H]+: 1074.7 |
| 90 | 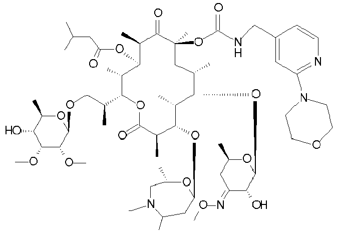 | i | 1.017 | [M+2H]/2+: 605 mixture of isomers |
| 91 | 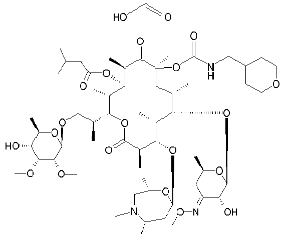 | f | 1.255 | [M+Na]+: 1152.7 mixture of isomers |
| 92 | 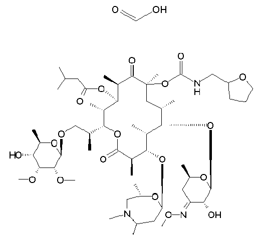 | f | 1.272 | [M+H]+: 1116.4 mixture of isomers |
| 93 | 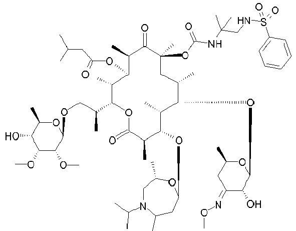 | b | 1.16 | [M+H]+: 1271 mixture of isomers |
| 94 | 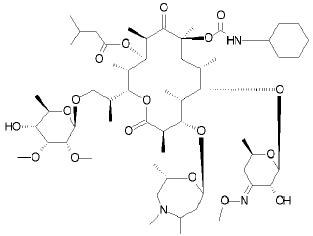 | b | 1.15 | [M+H]+: 1114 mixture of isomers |
| 95 | 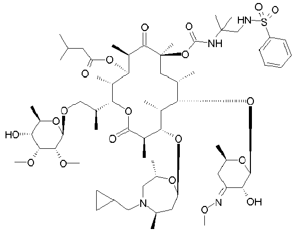 | b | 1.18 | [M+H]+: 1283 |
| 96 | 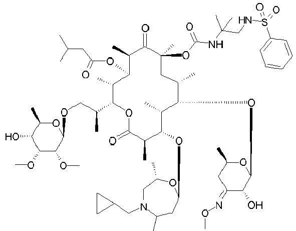 | b | 1.18 | [M+H]+: 1283 mixture of isomers |
| 97 | 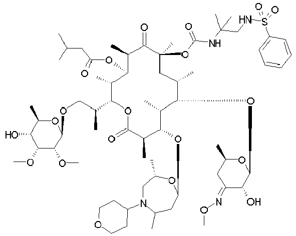 | b | 1.14 | [M+H]+: 1313 mixture of isomers |
| 98 | 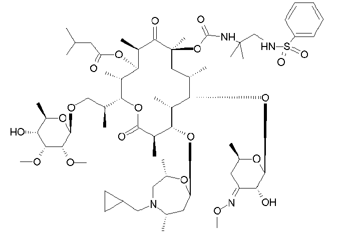 | b | 1.18 | [M+H]+: 1283 |
| 99 | 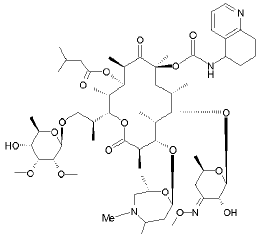 | i | 1.049 | [M+2H]/2+: 582.4 mixture of isomers |
| 100 | 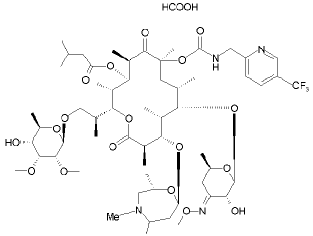 | f | 1.336 | [M+2H]/2+: 596.2 mixture of isomers |
| 101 | 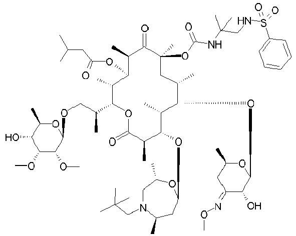 | b | 1.21 | [M+H]+: 1299 |
| 102 | 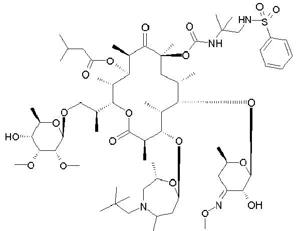 | b | 1.22 | [M+H]+: 1283 mixture of isomers |
| 103 | 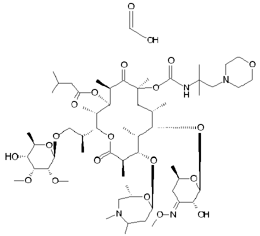 | i | 1.045 | [M+2H]/2+: 587.5 mixture of isomers |
| 104 | 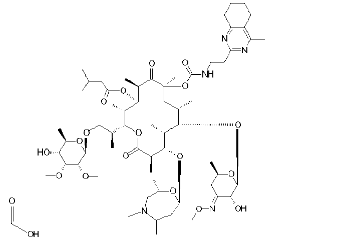 | h | 0.889 | [M+2H]/2+: 604 mixture of isomers |
| 105 | 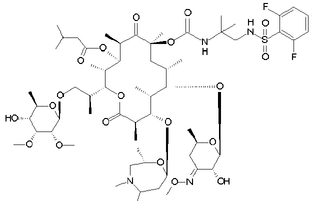 | i | 1.205 | [M+H]+: 1279.6 mixture of isomers |
| 106 | 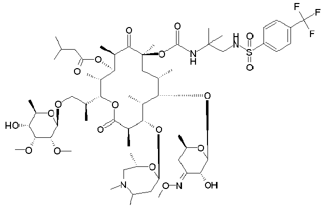 | i | 1.234 | [M+H]+: 1311.6 mixture of isomers |
| 107 | 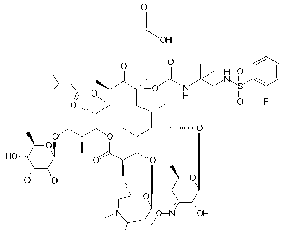 | i | 1.176 | [M+H]+: 1261.6 mixture of isomers |
| 108 | 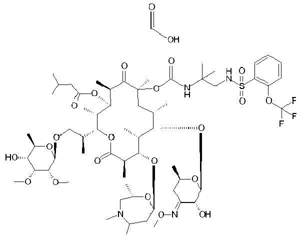 | i | 1.224 | [M+H]+: 1327.6 mixture of isomers |
| 109 | 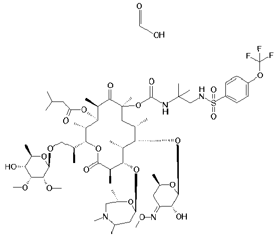 | i | 1.224 | [M+H]+: 1327.6 mixture of isomers |
| 110 | 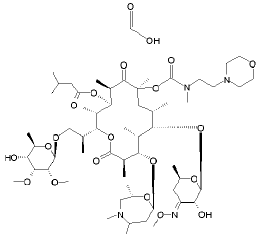 | i | 1.027 | [M+2H]/2+: 580.4 mixture of isomers |
| 111 | 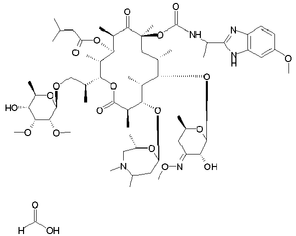 | h | 0.789 | [M+2H]/2+: 604.2 mixture of isomers |
| 112 | 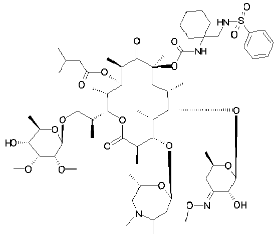 | a | 1.24 | [M+H]+: 1284 mixture of isomers |
| 113 | 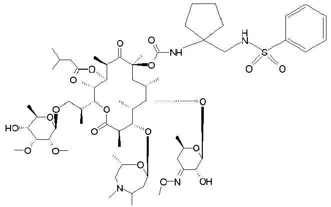 | a | 1.2 | [M+H]+: 1269 mixture of isomers |
| 114 | 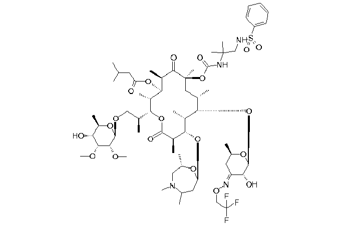 | a | 1.21 | [M+H]+: 1311 mixture of isomers |
| 115 | 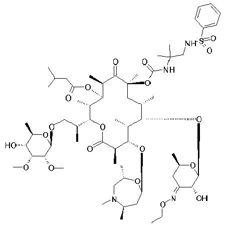 | a | 1.19 | [M+H]+: 1257 |
| 116 | 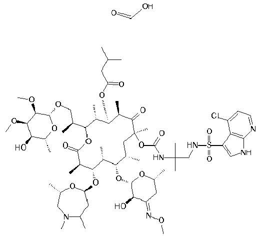 | i | 1.153 | [M+2H]/2+: 659.4 mixture of isomers |
| 117 | 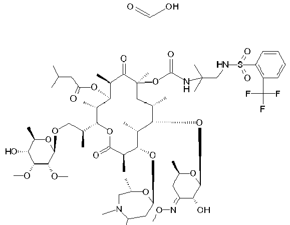 | g | 0.929 | [M+H]+: 1311.8 mixture of isomers |
| 118 | 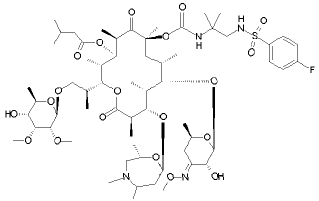 | i | 1.194 | [M+H]+: 1261.6 mixture of isomers |
| 119 | 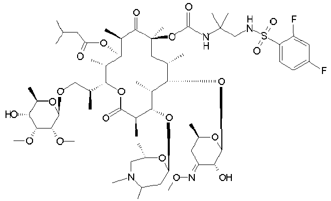 | i | 1.253 | [M+H]+: 1279.6 mixture of isomers |
| 120 | 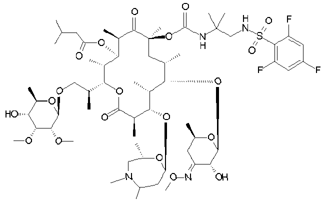 | f | 1.222 | [M+H]+: 1297.6 mixture of isomers |
| 121 | 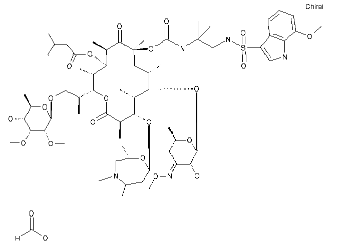 | i | 1.169 | [M+H]+: 1312.7 mixture of isomers |
| 122 | 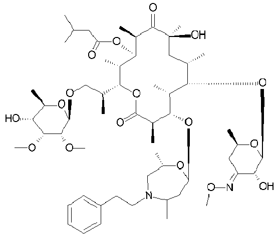 | g | 0.932 | [M+H]+: 1079.5 mixture of isomers |
| 123 | 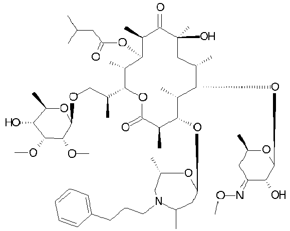 | h | 0.975 | [M+H]+: 1094.6 mixture of isomers |
| 124 | 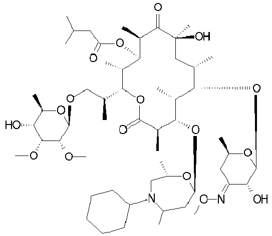 | f | 1.16 | [M+H]+: 1057.6 mixture of isomers |
| 125 | 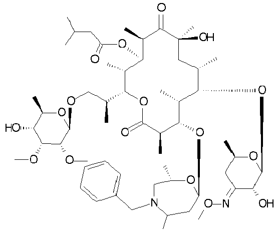 | g | 0.823 | [M+H]+: 1065.6 mixture of isomers |
| 126 | 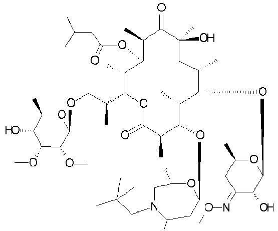 | f | 1.215 | [M+H]+: 1045.7 mixture of isomers |
| 127 | 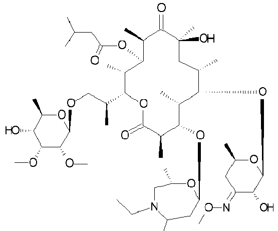 | f | 1.197 | [M+H]+: 1003.5 mixture of isomers |
| 128 | 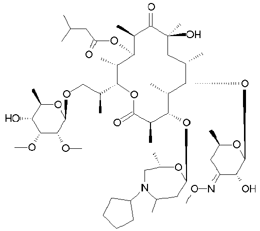 | h | 0.93 | [M+H]+: 1043.8 mixture of isomers |
| 129 | 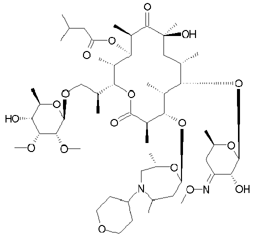 | h | 0.971 | [M+H]+: 1059.7 mixture of isomers |
| 130 | 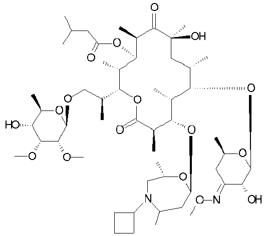 | h | 0.852 | [M+H]+: 1029.5 mixture of isomers |
| 131 | 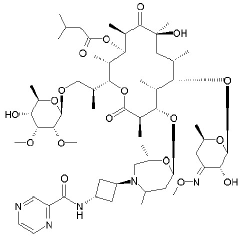 | h | 0.884 | [M+H]+: 1150.6 mixture of isomers |
| 132 | 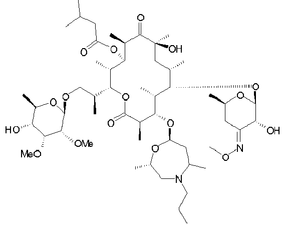 | g | 0.886 | [M+H]+: 1016.6 mixture of isomers |
| 133 | 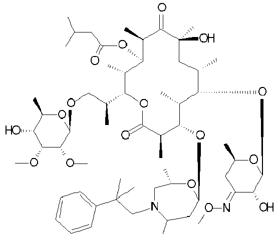 | f | 1.275 | [M+H]+: 1107.7 mixture of isomers |
| 134 | 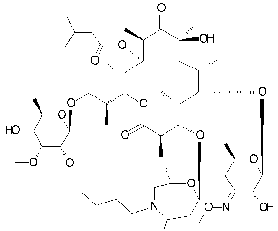 | f | 1.224 | [M+H]+: 1031.7 mixture of isomers |
| 135 | 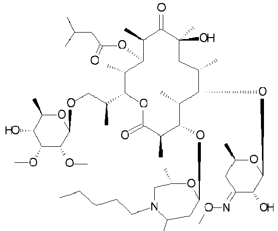 | f | 1.247 | [M+H]+: 1045.6 mixture of isomers |
| 136 | 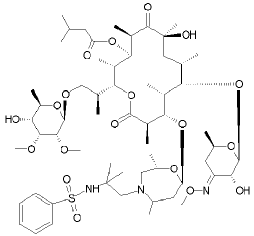 | f | 1.253 | [M+H]+: 1186.5 mixture of isomers |
| 137 | 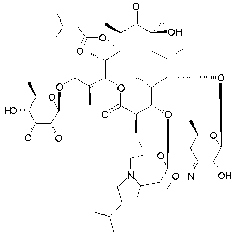 | h | 1.039 | [M+H]+: 1045.6 mixture of isomers |
| 138 | 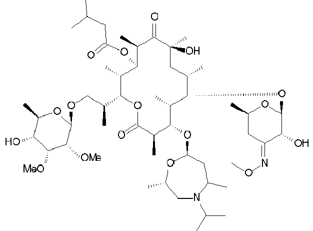 | f | 1.219 | [M+H]+: 1017.6 mixture of isomers |

### [USES]

The compounds corresponding to the general formula (I) that are the subject of the invention underwent microbiological trials which showed their value as therapeutically active substances. Specifically, they have bacteriostatic and/or bactericidal action on mycobacteria, especially against strains of *Mycobacterium* or *Corynebacterium,* which are in particular sensitive and resistant to the first-line antibiotics.

The compounds corresponding to the general formula (I) which are the subject of the invention also have bacteriostatic and/or bactericidal action on gram-positive microorganisms, in particular on staphylococci and streptococci.

More precisely, the compounds corresponding to the general formula (I) which are the subject of the invention are used for the prevention and/or treatment of bacterial infections caused by mycobacteria and gram-positive microorganisms.

### Measurement of the inhibitory activity (IC₈₀) of the compounds according to the invention towards Streptococcus pneumoniae

### Materials and methods

The test used is a bioluminescence test, the aim of which is to measure the inhibition of bacterial growth of *Streptococcus pneumoniae* by quantification of the amount of adenosine triphosphate (ATP). Specifically, ATP is a major and mandatory energy intermediate of very many reactions of cell metabolism which characterizes live media.

ATP quantification is performed at the end of the test by using an enzyme, luciferase, which, in the presence of ATP and of a specific substrate, luciferin, produces quantifiable light.

Thus, in the presence of luciferin and luciferase at non-saturating concentrations, the value obtained in relative light units (RLU) will make it possible, by means of a calibration, to estimate the amount of ATP and thus deduce the number of live bacteria at the end of the incubation period.

Thus, the more the RLU value obtained at the end of the test tends towards zero, the more the product inhibits the total growth of bacteria.

The results (Table 3) are expressed in IC₈₀. The IC₈₀ corresponds to 80% inhibition of the bacterial growth of *S*. *pneumoniae* with, as reference antibiotic, vancomycin, which has an IC₈₀ of 0.14 µM.

The experiments performed demonstrate that the compounds according to the present invention have activity on inhibiting the growth of *S*. *pneumoniae.* The IC₈₀ values are typically between 0.1 and 10 µM, or even between 0.1 and 1 µM.

### Measurement of the inhibitory activity of the compounds according to the invention towards Mycobacterium tuberculosis

The *in vitro* test used makes it possible to identify molecules having antimicrobial activity on the strain of *Mycobacterium tuberculosis* H₃₇Rᵥ. This is a bacterium of biohazard category 3.

### Materials and methods

The test used is Alamar blue (MABA). This is a colorimetric test which makes it possible to determine the MIC (minimum inhibitory concentration) of antibacterial agents. Alamar blue is a redox indicator which changes from blue to pink in the case of bacterial growth. Resazurin (blue and non-fluorescent) is reduced to resorufin (pink and fluorescent) by live bacteria. The plate is thus read visually or by fluorescence measurement. The fluorescence intensity is proportional to the number of live bacteria.

Thus, the more the fluorimetric MIC value tends towards zero, the less the amount of product necessary to inhibit the total growth of the bacteria.

The experiments performed demonstrate that the compounds according to the present invention have activity on inhibiting the growth of *M. tuberculosis.* The MIC values are typically between 0.1 and 10 µM, or even between 0.1 and 1 µM. The compounds presented as examples in the present patent application generally have MIC values of less than 1 µM.

**Table 3: Table of activities**

| Activities | | |
|---|---|---|
| Coumpounds | MIC (µM) MTb H37Rv | IC80 (µM) S. pneumoniae |
| 11 | 0.63 | ND |
| 12 | 0.6 | ND |
| 7-b | 0.52 | ND |
| 7-a | 0.54 | ND |
| 14 | 0.67 | 2.27 |
| 8-a | 0.56 | 1.84 |
| 2 | 1.25 | 0.28 |
| 24 | 0.48 | 0.88 |
| 25 | 0.42 | 2.03 |
| 26 | 0.78 | 0.69 |
| 33 | 0.35 | 1.34 |
| 35 | 0.36 | 1.81 |
| 36 | 0.49 | ND |
| 37 | 0.59 | 1.88 |
| 3 | 1.27 | ND |
| 6 | 0.91 | ND |
| 51 | 0.29 | ND |
| 4 | 0.21 | ND |
| 1-a | 2.05 | ND |
| 5-a | 0.34 | ND |
| 5-b | 0.53 | ND |
| 70 | 0.46 | ND |

| | | |
|---|---|---|
| ND: not determined. | | |

The compounds according to the invention, namely the compounds corresponding to formula (I), furthermore have good microbiological properties and are particularly suitable for use in preparing medicaments, in particular narrow-spectrum antibiotics for treating and/or preventing tuberculosis.

In particular, these antibiotics have antimicrobial action against *M. tuberculosis* for the treatment and/or prevention of tuberculosis.

Thus, according to another of its aspects, a subject of the invention is medicaments that comprise a compound of formula (I), or an addition salt thereof with a pharmaceutically acceptable acid or base of the compound of formula (I).

These medicaments find their use in therapeutics, especially in the treatment and/or prevention of tuberculosis.

According to another of its aspects, the present invention relates to pharmaceutical compositions comprising, as active ingredient, a compound according to the invention. These pharmaceutical compositions contain an effective dose of at least one compound according to the invention, or a pharmaceutically acceptable salt of the said compound, and also at least one pharmaceutically acceptable excipient.

The said excipients are chosen, according to the pharmaceutical form and the desired mode of administration, from the usual excipients which are known to those skilled in the art.

In the pharmaceutical compositions of the present invention for oral, sublingual, subcutaneous, intramuscular, intravenous, topical, local, intratracheal, intranasal, transdermal or rectal administration, the active principle of formula (I) above, or the salt thereof, may be administered in unit administration form, as a mixture with standard pharmaceutical excipients, to man and animals for the prevention or treatment of the above disorders or diseases.

The appropriate unit administration forms include oral forms, such as tablets, soft or hard gel capsules, powders, granules and oral solutions or suspensions, sublingual, buccal, intratracheal, intraocular and intranasal administration forms, forms of administration by inhalation, topical, transdermal, subcutaneous, intramuscular or intravenous administration forms, rectal administration forms, and implants. For topical application, the compounds according to the invention can be used in creams, gels, ointments or lotions.

By way of example, a unit administration form of a compound according to the invention in tablet form may comprise the following constituents:

| | |
|---|---|
| Compound according to the invention | 50.0 mg |
| Mannitol | 223.75 mg |
| Croscaramellose sodium | 6.0 mg |
| Corn starch | 15.0 mg |
| Hydroxypropylmethylcellulose | 2.25 mg |
| Magnesium stearate | 3.0 mg |

There may be particular cases where higher or lower dosages are appropriate; such dosages do not depart from the context of the invention. According to the usual practice, the dosage appropriate for each patient is determined by the physician according to the method of administration and the weight and response of the said patient.

Also described is the use of the compounds of formula (I) for the prevention and/or treatment of bacterial infections caused by gram-positive microorganisms and mycobacteria.

Also described is the use of the compounds of formula (I), or a pharmaceutically acceptable salt thereof, for the treatment and/or prevention of bacterial infections caused by mycobacteria such as *M. tuberculosis, M. smegmatis, M. phlei,* or other microorganisms such as *Nocardia brasiliensis, Nocardia absessus* or *Corynebacterium diphtheria,* for example.

Thus also described is the use of the compounds of formula (I), or a pharmaceutically acceptable salt thereof, for the treatment and/or prevention of infectious diseases such as tuberculosis, leprosy, nocardiosis, diphtheria, pulmonary mycobacterial infection, cutaneous mycobacterial infection, atypic mycobacterial infection and mycobacteriosis.

The term "tuberculosis" includes infections caused by bacilli of the tuberculosis complex (*M*. *tuberculosis, M. bovis* and *M. africanum*) that are all pathogenic to man. Pulmonary tuberculosis is far and away the most frequent and the most widespread; this is tuberculosis of the lung, of the larynx, of the trachea and of the bronchi, tuberculosis of the intrathoracic lymphatic ganglions, pleural respiratory tuberculosis, primary respiratory tuberculosis and any other respiratory tuberculosis. Although less frequent, ganglionic tuberculosis and extrapulmonary tuberculosis, tuberculosis of the nervous system such as tuberculous meningitis, tuberculous leptomeningitis, cerebral tuberculomes and any other tuberculosis of the nervous system, or bone or joint tuberculosis, tuberculosis of the urogenital system, lymphadenopathic peripheral tuberculosis, intestinal tuberculosis, peritoneal tuberculosis and/or tuberculosis of the mesenteric glands, cutaneous tuberculosis and tuberculosis of the subcutaneous tissues, tuberculosis of the eye, of the ear or of the adrenal glands, and disseminated tuberculosis, also exist.

The term "leprosy" (Hansen's disease) includes infections caused by *Mycobacterium leprae:* indeterminate leprosy, tuberculoid leprosy, borderline leprosy, borderline tuberculoid leprosy, lepromatous leprosy, and also the other forms of leprosy.

The term "diphtheria" includes pharyngeal diphtheria, nasopharyngeal diphtheria, cutaneous diphtheria, and also the other forms of diphtheria.

The term "nocardiosis" includes pulmonary nocardiosis, cutaneous nocardiosis, and the other forms of nocardiosis.

Also described is a method for treating the pathologies indicated above, which comprises the administration, to a patient, of an effective dose of a compound of formula (I).

## Claims

1. Compound of formula (I): 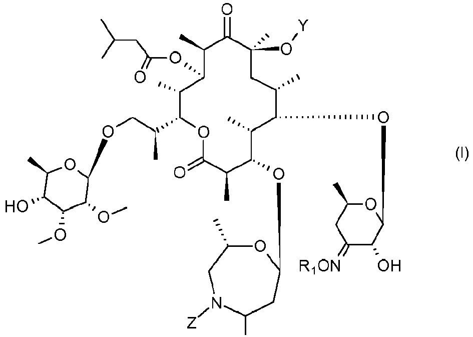 in which:
- Y represents a hydrogen atom, a group -(C=O)-NR₂R₃ or a group -(C=O)-O-R₁₈;
- Z represents:
• a hydrogen atom,
• a group -C₁₋₆-alkyl, which is unsubstituted or substituted with one or more groups R₄,
• a group -C₃₋₇-cycloalkyl, which is unsubstituted or substituted with a group -NH-(C=O)-R₁₉ or with a group -NH-SO₂-R₂₀,
• a group -C₃₋₆-heterocycloalkyl,
• a group -NH-(C=O)-R₅;
- R₁ represents a hydrogen atom, a group -C₂₋₆-alkenyl, a group -C₂₋₆-alkynyl or a group -C₁₋₆-alkyl which is unsubstituted or substituted with a group -C₁₋₄-fluoroalkyl or with a heteroaryl group which is unsubstituted or substituted with a group 3-(3-fluorophenyl)-2-oxo-1,3-oxazolidin-5-ylmethyl;
- R₂ represents a hydrogen atom or a group -C₁₋₆-alkyl;
- R₃ represents:
• a group -C₃₋₇-cycloalkyl, which is unsubstituted or substituted with a group -C₁₋₃-alkyl substituted with a group -NH-SO₂-R₂₁,
• a heteroaryl group,
• a linear or branched group -C₁₋₆-alkyl, which is unsubstituted or substituted with a group chosen from:
• a group -NH-R₆,
• a group -NH-SO₂-R₇,
• a group -NH-(C=O)-R₈,
• a group -C₃₋₇-cycloalkyl, which is unsubstituted or substituted with a group -C₃₋₆-heterocycloalkyl,
• a group -C₃₋₆-heterocycloalkyl,
• an aryl group, which is unsubstituted or substituted with one or more groups chosen independently from a halogen atom and a group -C₁₋₄-fluoroalkyl,
• a heteroaryl group, which is unsubstituted or substituted with a group -C₁₋₃-alkyl, a group -C₁₋₄-alkoxy, a group -C₁₋₄-fluoroalkyl or a group -C₃₋₆-heterocycloalkyl,
• or alternatively with one or more groups -C₁₋₄-alkoxy;
- or alternatively R₂ and R₃, together with the nitrogen atom to which they are attached, constitute a group -C₃₋₆-heterocycloalkyl chosen from: aziridine, azetidine, pyrrolidine, piperidine, morpholine, thiomorpholine or piperazine; the said heterocycloalkyl group being unsubstituted or substituted with a heteroaryl group, the said heteroaryl group being unsubstituted or substituted with a group - C₁₋₄-fluoroalkyl;
- R₄ independently represents a group chosen from:
• a hydroxyl group,
• a deuterium,
• a halogen atom,
• a group -C₃₋₇-cycloalkyl,
• an aryl group, which is unsubstituted or substituted with one or more groups -R₉,
• a heteroaryl group,
• a group -C₃₋₆-heterocycloalkyl,
• a group -C₁₋₄-alkoxy,
• a group -(C=O)-NH-R₁₀,
• a group -NH-R₁₁,
• a group -NH-(C=O)-R₁₂,
• or a group -NH(SO₁₃)-R₁₃;
- R₅ represents a heteroaryl group;
- R₆ represents a heteroaryl group, which is unsubstituted or substituted with one or more halogen atoms;
- R₇ represents a group -C₁₋₄-fluoroalkyl, an aryl group or a heteroaryl group, the said aryl and heteroaryl groups being unsubstituted or substituted with one or more groups R_{1'};
- R₈ represents a heteroaryl group, which is unsubstituted or substituted with one or more groups R_{2'};
- R₉ represents a halogen atom, a group -C₁₋₄-alkoxy, a formyl group (CHO) or a group -C₁₋₄-alkyl, which is unsubstituted or substituted with a hydroxyl group;
- R₁₀ represents a heteroaryl group, which is unsubstituted or substituted with a group -C₁₋₃-alkyl;
- R₁₁ represents:
• a group -C₃₋₁₀-heterocycloalkyl, which is unsubstituted or substituted with one or more oxide groups,
• a heteroaryl group or an aryl-C₁₋₄-alkyl group, the said heteroaryl or aryl groups being unsubstituted or substituted with one or more groups independently chosen from a halogen atom, a hydroxyl group, a nitro group and a group -C₁₋₃-alkyl;
- R₁₂ represents:
• a group -C₁₋₄-alkoxy,
• a group -C₁₋₄-alkyl, which is unsubstituted or substituted with a group -NR₁₄R₁₅ or with a heteroaryl group, the said heteroaryl group being unsubstituted or substituted with a group -C₁₋₃-alkyl,
• a heteroaryl group, which is unsubstituted or substituted with one or more groups chosen from a hydroxyl group and a group -C₁₋₃-alkyl;
- R₁₃ represents:
• a group -C₁₋₄-alkyl,
• a group -C₁₋₄-fluoroalkyl,
• an aryl group, which is unsubstituted or substituted with a nitro group,
• or a heteroaryl group, which is unsubstituted or substituted with a group -NR₁₆R₁₇;
- R₁₄, R₁₅, R₁₆ and R₁₇ each independently represent:
• a hydrogen atom,
• or a group -C₁₋₄-alkyl;
- R₁₈ represents a group -C₁₋₄-alkyl or a benzyl group;
- R₁₉ represents an aryl group or a heteroaryl group;
- R₂₀ represents a group -C₁₋₄-alkyl or an aryl group;
- R₂₁ represents an aryl group;
- R_{1'} represents:
• a halogen atom,
• a group -C₁₋₄-alkoxy,
• a group -C₁₋₄-fluoroalkyl,
• a group -OCF₃,
• a nitro group,
• a group -NH₂,
• a group -NHCH₃;
- R_{2'} represents:
• a hydroxyl group,
• a group -C₁₋₆-alkyl.

2. Compound according to Claim 1, of formula (IA): 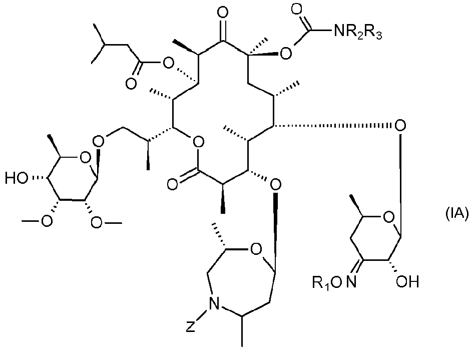 in which:
- R₁, R₂, R₃ and Z are as defined in Claim 1.

3. Compound according to Claim 1, of formula (IB): 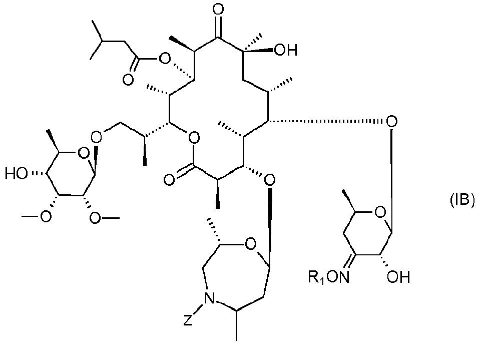 in which:
- R₁ and Z are as defined in Claim 1.

4. Compound according to Claim 1, of formula (Ii): 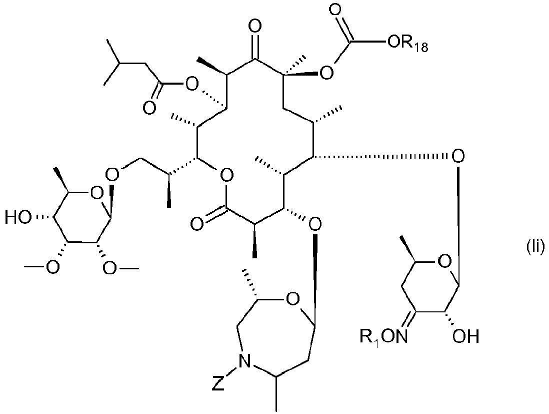 in which:
- R₁, R₁₈ and Z are as defined in Claim 1.

5. Compound of formula (I) according to Claim 1, **characterized in that**:
- Y represents a hydrogen atom, a group -(C=O)-NR₂R₃ or a group -(C=O)-OMe;
- Z represents:
• a hydrogen atom,
• a group -C₁₋₆-alkyl, which is unsubstituted or substituted with one or more groups R₄,
• a cyclopropyl group, a cyclobutyl group, a 3-(benzoylamino)cyclobutyl group, a 3-[(pyrazin-2-ylcarbonyl)amino]cyclobutyl group, a 3-[(methylsulfonyl)amino]cyclobutyl group, a 3-[(phenylsulfonyl)amino]cyclobutyl group, a cyclopentyl group, a cyclohexyl group,
• a tetrahydro-2H-pyranyl group,
• a group -NH-(C=O)-R₅;
- R₁ represents a hydrogen atom, an ethyl group, a 2,2,2-trifluoroethyl group or a methyl group, which is unsubstituted or substituted with a 1,2,3-triazole group substituted with a 3-(3-fluorophenyl)-2-oxo-1,3-oxazolidin-5-ylmethyl group;
- R₂ represents a hydrogen atom or a methyl group;
- R₃ represents:
• a cyclohexyl group, a 1-{[(phenylsulfonyl)amino]methyl}cyclohexyl group or a 1-{[(phenylsulfonyl)amino]methyl}cyclopentyl group,
• a 5,6,7,8-tetrahydroquinolin-5-yl group,
• or a linear or branched group C₁₋₄-alkyl, which is unsubstituted or substituted with a group chosen from:
• -NH-R₆,
• -NH-SO₂-R₇,
• -NH-(C=O)-R₈,
• a 1-morpholin-4-ylcyclopentyl group,
• a tetrahydro-2H-pyranyl group, a tetrahydrofuranyl group or a morpholin-4-yl group,
• a phenyl group, which is unsubstituted or substituted with one or more groups chosen independently from a chlorine atom and a group -CF₃,
• a 1 H-pyrrolo[2,3-b]pyridinyl group, a 4-methyl-5,6,7,8-tetrahydroquinazolin-2-yl group, a 6-methoxy-1H-benzimidazol-2-yl group, a pyridinyl group, which is unsubstituted or substituted with a group -CF₃ or with a morpholin-4-yl group,
• or alternatively with one or more methoxy groups;
- or alternatively R₂ and R₃, together with the nitrogen atom to which they are attached, constitute a -C₃₋₆-heterocycloalkyl group chosen from: azetidine, morpholine, 4-[5-(trifluoromethyl)pyridin-2-yl]piperazine;
- R₄ independently represents a group chosen from:
• a hydroxyl group,
• a deuterium,
• a fluorine atom,
• a cyclopropyl group,
• a phenyl group, which is unsubstituted or substituted with one or more groups chosen independently from a fluorine atom, a methoxy group, a - CH₂OH group and a -CHO group,
• a pyridyl group,
• a morpholinyl group, a tetrahydro-2H-pyranyl group,
• a methoxy group,
• a group -(C=O)-NH-R₁₀,
• a group -NH-R₁₁,
• a group -NH-(C=O)-R₁₂,
• or a group -NH(SO₂)-R₁₃;
- R₅ represents a pyridyl group;
- R₆ represents a quinolyl group, the said quinolyl group being unsubstituted or substituted with a chlorine atom;
- R₇ represents a -CF₃ group, a phenyl, pyridyl, pyrazolyl, 1 H-pyrrolo[2,3-b]pyridyl or indolyl group, the said phenyl, pyridyl, pyrazolyl, 1 H-pyrrolo[2,3-b]pyridyl or indolyl groups being unsubstituted or substituted with one or more groups R_{1'};
- R₈ represents a pyrazinyl group, the said pyrazinyl group being unsubstituted or substituted with one or more groups R_{2'};
- R₁₀ represents a 1,8-naphthyridinyl group substituted with a methyl group;
- R₁₁ represents a tetrahydrothiophene-1,1-dioxide, quinolyl, pyridyl or benzyl group, the said quinolyl, pyridyl or benzyl groups being unsubstituted or substituted with a chlorine atom, a hydroxyl group, a nitro group or a methyl group;
- R₁₂ represents:
• a tert-butoxy group,
• a group -C₁₋₄-alkyl, which is unsubstituted or substituted with a group chosen from a group -NR₁₄R₁₅, pyridyl or pyrazolyl, the said pyridyl or pyrazolyl groups being unsubstituted or substituted with a methyl group,
• a pyrazinyl or pyridyl, which is unsubstituted or substituted with one or more groups chosen from a hydroxyl group and a methyl group;
- R₁₃ represents:
• a group -CF₃,
• a phenyl group, which is unsubstituted or substituted with a nitro group,
• or a pyridyl group, which is unsubstituted or substituted with a group -NR₁₆R₁₇;
- R₁₄, R₁₅, R₁₆ and R₁₇ each independently represent:
• a hydrogen atom,
• a methyl group or an isopropyl group;
- R_{1'} represents:
• a fluorine atom, a chlorine atom,
• a methoxy group,
• a group -CF₃,
• a group -OCF₃,
• a nitro group,
• a group -NH₂,
• a group -NHCH₃;
- R_{2'} represents:
• a hydroxyl group,
• a methyl group.

6. Compound of formula (I) according to Claim 1, **characterized in that**:
- Y represents a hydrogen atom or a group -(C=O)-NR₂R₃;
- Z represents:
• a hydrogen atom,
• a methyl group, an isopropyl group, a 2,2-dimethylpropyl group,
• a group CD₃,
• a 2-fluoroethyl group,
• a cyclopropylmethyl group,
• a 2-phenylethyl group,
• a [(7-methyl-1,8-naphthyridin-2-yl)amino]-4-oxobutyl group,
• a 2-{[(2-nitrophenyl)sulfonyl]amino}ethyl group,
• a cyclopropyl group,
• a tetrahydro-2H-pyranyl group;
- R₁ represents a hydrogen atom, an ethyl group, a 2,2,2-trifluoroethyl group or a methyl group;
- R₂ represents a hydrogen atom or a methyl group;
- R₃ represents:
• a methyl group,
• a 2-{[(2,6-difluorophenyl)sulfonyl]amino}-1,1-dimethylethyl group,
• a 1,1-dimethyl-2-({[4-(trifluoromethyl)phenyl]sulfonyl}amino)ethyl group,
• a 2-{[(2-fluorophenyl)sulfonyl]amino}-1,1-dimethylethyl group,
• a 1,1-dimethyl-2-({[2-(trifluoromethoxy)phenyl]sulfonyl}amino)ethyl group,
• a 1,1-dimethyl-2-({[4-(trifluoromethoxy)phenyl]sulfonyl}amino)ethyl group,
• a 2-methyl-1-[(phenylsulfonyl)amino]propan-2-yl group,
• a 2-methyl-1-{[(5-nitro-1H-pyrazol-4-yl)sulfonyl]amino}propan-2-yl group,
• a 2-methyl-1-{[(trifluoromethyl)sulfonyl]amino}propan-2-yl group,
• a 2-methyl-1-{[(2-nitrophenyl)sulfonyl]amino}propan-2-yl group,
• a 1-{[(5-hydroxypyrazin-2-yl)carbonyl]amino}-2-methylpropan-2-yl group,
• a 1,1-dimethyl-2-morpholin-4-ylethyl group,
• a benzyl group,
• a 2-(4-pyridyl)ethyl group.

7. Compound of formula (I) according to any one of Claims 1 to 6, **characterized in that** it corresponds to the following compounds:
• (2R,3S,4R,5R,7S,9S,10S,11R,12S,13R)-7-[(benzylcarbamoyl)oxy]-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-7-[(benzylcarbamoyl)oxy]-2-(1{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5R,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-12-{[(2S,7R)-4-cyclopropyl-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-7-{[(1-{[(5-hydroxypyrazin-2-yl)carbonyl]amino}-2-methylpropan-2-yl)carbamoyl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-7-[(benzylcarbamoyl)oxy]-12-{[(2S,5R,7R)-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl3-methylbutanoate;
• (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-7-({[2-(pyridin-4-yl)ethyl]carbamoyl}oxy)-12-{[(2S,5S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-7-[(benzylcarbamoyl)oxy]-12-{[(2S,7R)-4-cyclopropyl-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-7-[(benzylcarbamoyl)oxy]-12-{[(2S,7R)-2,5-dimethyl-4-(²H₃)methyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-7-[(dimethylcarbamoyl)oxy]-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5R,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-7-[(benzylcarbamoyl)oxy]-12-{[(2S,7R)-2,5-dimethyl-4-(2-{[(2-nitrophenyl)sulfonyl]amino}ethyl)-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-7-[(benzylcarbamoyl)oxy]-12-{[(2S,7R)-4-(2-fluoroethyl)-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-7-[(benzylcarbamoyl)oxy]-12-{[(2S,7R)-2,5-dimethyl-4-{4-[(7-methyl-1,8-naphthyridin-2-yl)amino]-4-oxobutyl}-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-7-[(benzylcarbamoyl)oxy]-12-{[(2S,7R)-4-(2,2-dimethylpropyl)-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-12-{[(2S,7R)-2,5-dimethyl-4-(2-phenylethyl)-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-7-{[(1-{[(5-hydroxypyrazin-2-yl)carbonyl]amino}-2-methylpropan-2-yl)carbamoyl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(5-nitro-1H-pyrazol-4-yl)sulfonyl]amino}propan-2-yl)carbamoyl]oxy}-6,14-dioxo-12-{[(2S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-7-hydroxy-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5R,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(trifluoromethyl)sulfonyl]amino}propan-2-yl)carbamoyl]oxy}-6,14-dioxo-12-{[(2S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-12-{[(2S,7R)-2,5-dimethyl-4-(2-phenylethyl)-1,4-oxazepan-7-yl]oxy}-7-hydroxy-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(2-nitrophenyl)sulfonyl]amino}propan-2-yl)carbamoyl]oxy}-6,14-dioxo-12-{[(2S,5R,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(2-nitrophenyl)sulfonyl]amino}propan-2-yl)carbamoyl]oxy}-6,14-dioxo-12-{[(2S,5S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-[({2-methyl-1-[(phenylsulfonyl)amino]propan-2-yl}carbamoyl)oxy]-6,14-dioxo-12-{[(2S,5S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-[({2-methyl-1-[(phenylsulfonyl)amino]propan-2-yl}carbamoyl)oxy]-6,14-dioxo-12-{[(2S,5R,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-7-[({1,1-dimethyl-2-[(phenylsulfonyl)amino]ethyl}carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-12-{[(2S,7R)-4-isopropyl-2,5-dimethyl-1,4-oxazepan-7-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-12-{[(2S,5R,7R)-4-(cyclopropylmethyl)-2,5-dimethyl-1,4-oxazepan-7-yl]oxy}-7-[({1,1-dimethyl-2-[(phenylsulfonyl)amino]ethyl}carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-12-{[(2S,7R)-4-(cyclopropylmethyl)-2,5-dimethyl-1,4-oxazepan-7-yl]oxy}-7-[({1,1-dimethyl-2-[(phenylsulfonyl)amino]ethyl}carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-7-[({1,1-dimethyl-2-[(phenylsulfonyl)amino]ethyl}carbamoyl)oxy]-12-{[(2S,7R)-2,5-dimethyl-4-(tetrahydro-2H-pyran-4-yl)-1,4-oxazepan-7-yl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-12-{[(2S,5S,7R)-4-(cyclopropylmethyl)-2,5-dimethyl-1,4-oxazepan-7-yl]oxy}-7-[({1,1-dimethyl-2-[(phenylsulfonyl)amino]ethyl}carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-7-[({1,1-dimethyl-2-[(phenylsulfonyl)amino]ethyl}carbamoyl)oxy]-12-{[(2S,5R,7R)-4-(2,2-dimethylpropyl)-2,5-dimethyl-1,4-oxazepan-7-yl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-7-[({1,1-dimethyl-2-[(phenylsulfonyl)amino]ethyl}carbamoyl)oxy]-12-{[(2S,7R)-4-(2,2-dimethylpropyl)-2,5-dimethyl-1,4-oxazepan-7-yl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-yl 3-methylbutanoate;
• (2R,3S,4R,5R,7S,9S,10S,11R,12S,13R)-7-{[(1,1-dimethyl-2-morpholin-4-ylethyl)carbamoyl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-7-{[(2-{[(2,6-difluorophenyl)sulfonyl]amino}-1,1-dimethylethyl)carbamoyl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-7-({[1,1-dimethyl-2-({[4-(trifluoromethyl)phenyl]sulfonyl}amino)ethyl]carbamoyl}oxy)-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-7-{[(2-{[(2-fluorophenyl)sulfonyl]amino}-1,1-dimethylethyl)carbamoyl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-7-({[1,1-dimethyl-2-({[2-(trifluoromethoxy)phenyl]sulfonyl}amino)ethyl]carbamoyl}oxy)-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-7-({[1,1-dimethyl-2-({[4-(trifluoromethoxy)phenyl]sulfonyl}amino)ethyl]carbamoyl}oxy)-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-7-[({1,1-dimethyl-2-[(phenylsulfonyl)amino]ethyl}carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-({(2S,3R,6R)-3-hydroxy-6-methyl-4-[(2,2,2-trifluoroethoxy)imino]tetrahydro-2H-pyran-2-yl}oxy)-3,5,7,9,11,13-hexamethyl-6,14-dioxo-12-{[(2S,7R)-2,4,5-trimethyl-1,4-oxazepan-7-yl]oxy}oxacyclotetradecan-4-yl 3-methylbutanoate;
• (2R,3S,4R,5R,7S,9S,10S,11R,12S,13R)-7-[({1,1-dimethyl-2-[(phenylsulfonyl)amino]ethyl}carbamoyl)oxy]-10-{[(2S,3R,6R)-4-(ethoxyimino)-3-hydroxy-6-methyltetrahydro-2H-pyran-2-yl]oxy}-2-(2-{[(2R,3R,4R,5R,6R)-5-hydroxy-3,4-dimethoxy-6-methyltetrahydro-2H-pyran-2-yl]oxy}-1-methylethyl)-3,5,7,9,11,13-hexamethyl-6,14-dioxo-12-{[(2S,5R,7R)-2,4,5-trimethyl-1,4-oxazepan-7-yl]oxy}oxacyclotetradecan-4-yl 3-methylbutanoate.

8. Process for preparing a compound of formula (I) according to Claim 1, in which Y represents a group -(C=O)-NR₂R₃, **characterized in that**:
a compound of formula (IB): 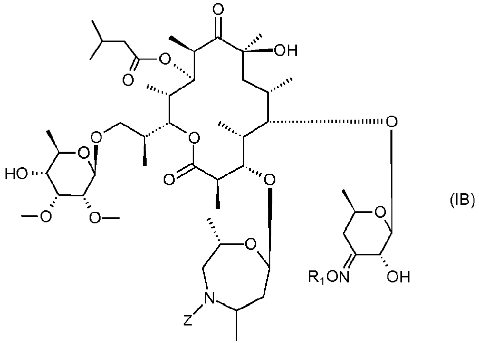 in which R₁ and Z are as defined for the compounds of formula (I) in Claim 1, is reacted with a compound of formula (II) HNR₂R₃ in which R₂ and R₃ are as defined for the compounds of formula (I) in Claim 1, in the presence of a carbonyl derivative and a base.

9. Process for preparing a compound of formula (I) according to Claim 1, in which Y represents a group -(C=O)-NR₂R₃, **characterized in that**:
b-1) a compound of formula (V): 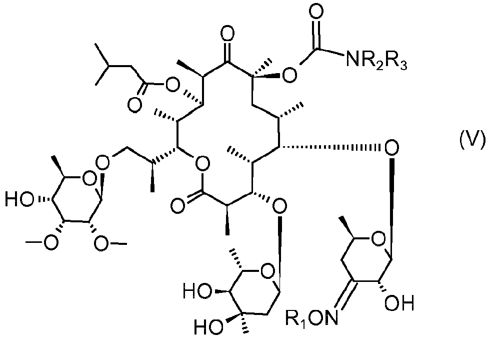 in which R₁, R₂ and R₃ are as defined for a compound of formula (I) in Claim 1, is reacted with an oxidizing agent to obtain a compound of formula (VI): 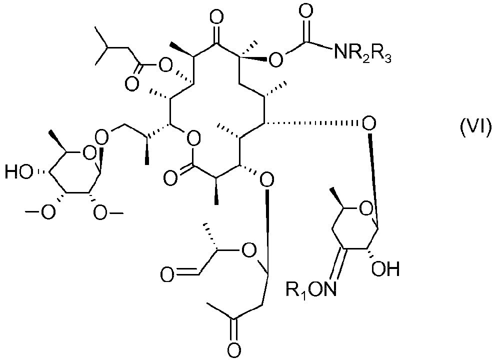
b-2) the compound of formula (VI) thus obtained is reacted with a compound of formula (VII):
ZNH₂ (VII)
in which Z is as defined for compound (I) in Claim 1, in the presence of a reducing agent.

10. Process for preparing a compound of formula (I) according to Claim 1, in which Y represents a hydrogen atom, **characterized in that**:
c-1) a compound of formula (VIII): 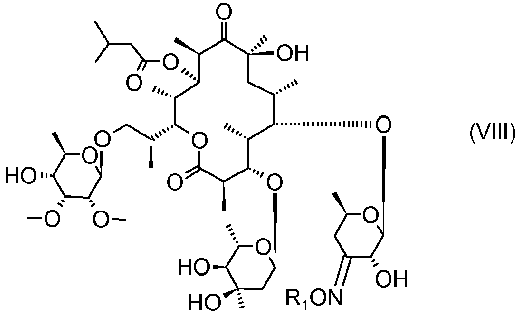 in which R₁ is as defined for compound (I) in Claim 1, is reacted with an oxidizing agent to obtain a compound of formula (IX): 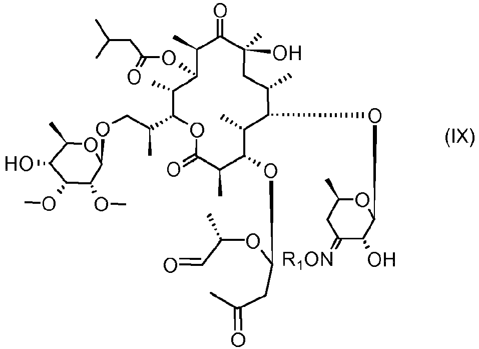
c-2) the compound of formula (IX) thus obtained is reacted with a compound of formula (VII):
ZNH₂ (VII)
in which Z is as defined for compound (I) in Claim 1, in the presence of a reducing agent.

11. Process for preparing a compound of formula (I) according to Claim 1, in which Y represents a group -(C=O)-O-R₁₈, **characterized in that**:
a compound of formula (XXI): 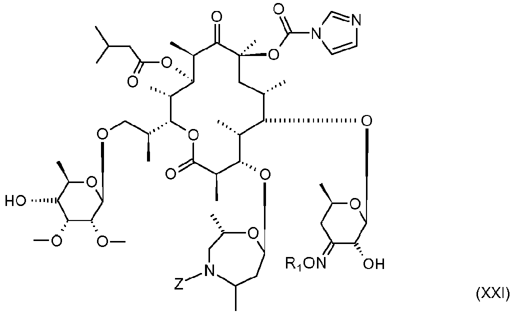 in which Z and R₁ are as defined for a compound of formula (I) in Claim 1, is reacted with an alcohol of formula HO-R₁₈ (XXII) in which R₁₈ is as defined for a compound of formula (I) in Claim 1, in the presence of a base.

12. Compound of formula (V): 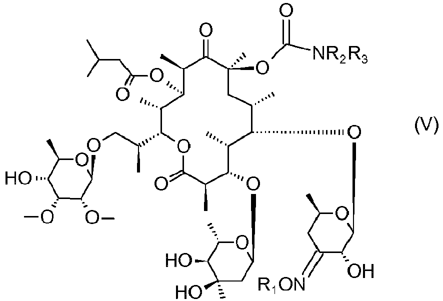 in which:
- R₁, R₂ and R₃ are as defined for the compounds of formula (I) in Claim 1.

13. Compound of formula (VIII): 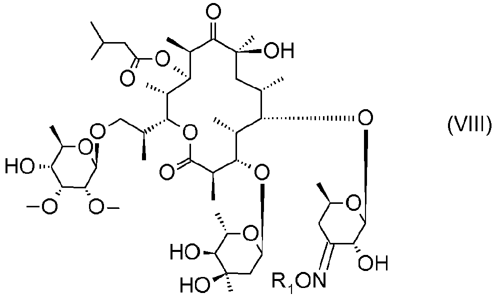 in which:
- R₁ is as defined for the compounds of formula (I) in Claim 1.

14. Medicament, **characterized in that** it comprises a compound of formula (I) according to any one of Claims 1 to 7, in the form of a base or of an acid-addition salt.

15. Pharmaceutical composition, **characterized in that** it comprises a compound of formula (I) according to any one of Claims 1 to 7, in the form of a base or of an acid-addition salt, and also at least one pharmaceutically acceptable excipient.

16. Compound according to any one of Claims 1 to 7, for its use for the prevention and/or treatment of bacterial infections caused by gram-positive microorganisms and mycobacteria.

17. Compound according to any one of Claims 1 to 7, for its use for the prevention and/or treatment of infectious diseases chosen from tuberculosis, leprosy, nocardiosis, diphtheria, pulmonary mycobacterial infection, cutaneous mycobacterial infection, atypic mycobacterial infection and mycobacteriosis.

## Patentansprüche

1. Verbindung der Formel (I): 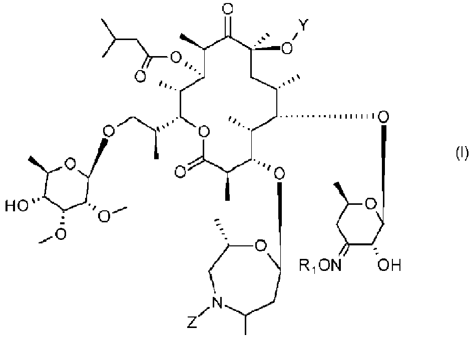 in der:
- Y für ein Wasserstoffatom, eine Gruppe -(C=O)-NR₂R₃ oder eine Gruppe -(C=O)-O-R₁₈ steht;
- Z für:
• ein Wasserstoffatom,
• eine Gruppe -C₁-₆-Alkyl, die unsubstituiert oder durch eine oder mehrere Gruppen R₄ substituiert ist,
• eine Gruppe -C₃₋₇-Cycloalkyl, die unsubstituiert oder durch eine Gruppe -NH-(C=O)-R₁₉ oder durch eine Gruppe -NH-SO₂-R₂₀ substituiert ist,
• eine Gruppe -C₃₋₆-Heterocycloalkyl,
• eine Gruppe -NH-(C=O)-R₅,
steht;
- R₁ für ein Wasserstoffatom, eine Gruppe -C₂₋₆-Alkenyl, eine Gruppe -C₂₋₆-Alkinyl oder eine Gruppe -C₁₋₆-Alkyl, die unsubstituiert oder durch eine Gruppe -C₁₋₄-Fluoralkyl oder durch eine Heteroarylgruppe, die unsubstituiert oder durch eine Gruppe 3-(3-Fluorphenyl)-2-oxo-1,3-oxazolidin-5-ylmethyl substituiert ist, substituiert ist, steht;
- R₂ für ein Wasserstoffatom oder eine Gruppe -C₁₋₆-Alkyl steht;
- R₃ für:
• eine Gruppe -C₃₋₇-Cycloalkyl, die unsubstituiert oder durch eine Gruppe -C₁₋₃-Alkyl, die durch eine Gruppe -NH-SO₂-R₂₁ substituiert ist, substituiert ist,
• eine Heteroarylgruppe,
• eine lineare oder verzweigte Gruppe -C₁₋₆-Alkyl, die unsubstituiert oder durch eine Gruppe substituiert ist, die ausgewählt ist aus:
• einer Gruppe -NH-R₆,
• einer Gruppe -NH-SO₂-R₇,
• einer Gruppe -NH-(C=O)-R₈,
• einer Gruppe -C₃₋₇-Cycloalkyl, die unsubstituiert oder durch eine Gruppe -C₃₋₆-Heterocycloalkyl substituiert ist,
• einer Gruppe -C₃₋₆-Heterocycloalkyl,
• eine Arylgruppe, die unsubstituiert oder durch eine oder mehrere Gruppen, die unabhängig aus einem Halogenatom und einer Gruppe -C₁₋₄-Fluoralkyl ausgewählt sind, substituiert ist,
• einer Heteroarylgruppe, die unsubstituiert oder durch eine Gruppe -C₁₋₃-Alkyl, eine Gruppe -C₁₋₄-Alkoxy, eine Gruppe -C₁₋₄-Fluoralkyl oder eine Gruppe -C₃₋₆-Heterocycloalkyl substituiert ist,
• oder alternativ dazu durch eine oder mehrere Gruppen -C₁₋₄-Alkoxy,
steht;
- oder alternativ dazu R₂ und R₃ zusammen mit dem Stickstoffatom, an das sie gebunden sind, eine Gruppe -C₃₋₆-Heterocycloalkyl bilden, die ausgewählt ist aus: Aziridin, Azetidin, Pyrrolidin, Piperidin, Morpholin, Thiomorpholin oder Piperazin; wobei die Heterocycloalkylgruppe unsubstituiert oder durch eine Heteroarylgruppe substituiert ist, wobei die Heteroarylgruppe unsubstituiert oder durch eine Gruppe -C₁₋₄-Fluoralkyl substituiert ist;
- R₄ unabhängig für eine Gruppe steht, die ausgewählt ist aus:
• einer Hydroxylgruppe,
• einem Deuterium,
• einem Halogenatom,
• einer Gruppe -C₃₋₇-Cycloalkyl,
• einer Arylgruppe, die unsubstituiert oder durch eine oder mehrere Gruppen -R₉ substituiert ist
• einer Heteroarylgruppe,
• einer Gruppe -C₃₋₆-Heterocycloalkyl,
• einer Gruppe -C₁₋₄-Alkoxy,
• einer Gruppe - (C=O) -NH-R₁₀,
• einer Gruppe -NH-R₁₁,
• einer Gruppe -NH-(C=O)-R₁₂,
• oder einer Gruppe -NH(SO₁₃)-R₁₃;
- R₅ für eine Heteroarylgruppe steht;
- R₆ für eine Heteroarylgruppe, die unsubstituiert oder durch ein oder mehrere Halogenatome substituiert ist, steht;
- R₇ für eine Gruppe -C₁₋₄-Fluoralkyl, eine Arylgruppe oder eine Heteroarylgruppe steht, wobei die Aryl- und Heteroarylgruppe unsubstituiert oder durch eine oder mehrere Gruppen R_{1'} substituiert sind;
- R₈ für eine Heteroarylgruppe, die unsubstituiert oder durch eine oder mehrere Gruppen R_{2'} substituiert ist;
- R₉ für ein Halogenatom, eine Gruppe -C₁₋₄-Alkoxy, eine Formylgruppe (CHO) oder eine Gruppe -C₁₋₄-Alkyl, die unsubstituiert oder durch eine Hydroxylgruppe substituiert ist, steht;
- R₁₀ für eine Heteroarylgruppe, die unsubstituiert oder durch eine Gruppe -C₁₋₃-Alkyl substituiert ist, steht;
- R₁₁ für:
• eine Gruppe -C₃₋₁₀-Heterocycloalkyl, die unsubstituiert oder durch eine oder mehrere Oxidgruppen substituiert ist,
• eine Heteroarylgruppe oder eine Aryl-C₁₋₄-alkylgruppe, wobei die Heteroaryl- oder Arylgruppen unsubstituiert oder durch ein oder mehrere Gruppen, die unabhängig aus einem Halogenatom, einer Hydroxylgruppe, einer Nitrogruppe und einer Gruppe -C₁₋₃-Alkyl ausgewählt sind, substituiert sind, steht;
- R₁₂ für:
• eine Gruppe -C₁₋₄-Alkoxy,
• eine Gruppe -C₁₋₄-Alkyl, die unsubstituiert oder durch eine Gruppe -NR₁₄R₁₅ oder durch eine Heteroarylgruppe substituiert ist, wobei die Heteroarylgruppe unsubstituiert oder durch eine Gruppe -C₁₋₃-Alkyl substituiert ist,
• eine Heteroarylgruppe, die unsubstituiert oder durch eine oder mehrere Gruppen, die aus einer Hydroxylgruppe und einer Gruppe -C₁₋₃-Alkyl ausgewählt sind, substituiert ist, steht;
- R₁₃ für:
• eine Gruppe -C₁₋₄-Alkyl,
• eine Gruppe -C₁₋₄-Fluoralkyl,
• eine Arylgruppe, die unsubstituiert oder durch eine Nitrogruppe substituiert ist,
• oder eine Heteroarylgruppe, die unsubstituiert oder durch eine Gruppe -NR₁₆R₁₇ substituiert ist, steht;
- R₁₄, R₁₅, R₁₆ und R₁₇ jeweils unabhängig für:
• ein Wasserstoffatom,
• oder eine Gruppe -C₁₋₄-Alkyl stehen;
- R₁₈ für eine Gruppe -C₁₋₄-Alkyl oder eine Benzylgruppe steht;
- R₁₉ für eine Arylgruppe oder eine Heteroarylgruppe steht;
- R₂₀ für eine Gruppe -C₁₋₄-Alkyl oder eine Arylgruppe steht;
- R₂₁ für eine Arylgruppe steht;
- R_{1'} für:
• ein Halogenatom,
• eine Gruppe -C₁₋₄-Alkoxy,
• eine Gruppe -C₁₋₄-Fluoralkyl,
• eine Gruppe -OCF₃,
• eine Nitrogruppe,
• eine Gruppe -NH₂,
• eine Gruppe -NHCH₃ steht;
- R_{2'} für:
• eine Hydroxylgruppe,
• eine Gruppe -C₁₋₆-Alkyl steht.

2. Verbindung nach Anspruch 1 der Formel (IA): 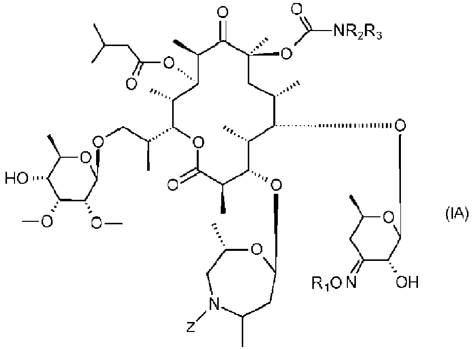 in der:
- R₁, R₂, R₃ und Z wie in Anspruch 1 definiert sind.

3. Verbindung nach Anspruch 1 der Formel (IB): 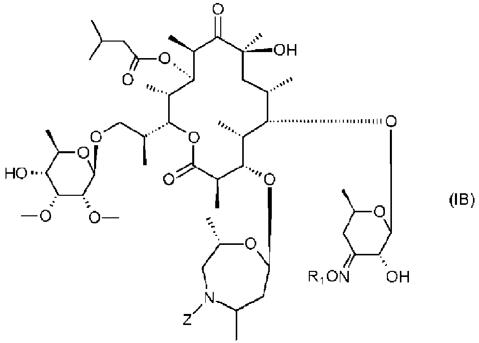 in der:
- R₁ und Z wie in Anspruch 1 definiert sind.

4. Verbindung nach Anspruch 1 der Formel (Ii): 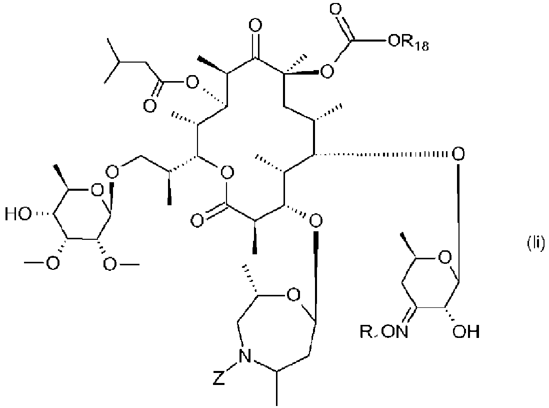 in der:
- R₁, R₁₈ und Z wie in Anspruch 1 definiert sind.

5. Verbindung der Formel (I) nach Anspruch 1, **dadurch gekennzeichnet, dass**:
- Y für ein Wasserstoffatom, eine Gruppe -(C=O)-NR₂R₃ oder eine Gruppe -(C=O)-OMe steht;
- Z für:
• ein Wasserstoffatom,
• eine Gruppe -C₁₋₆-Alkyl, die unsubstituiert oder durch eine oder mehrere Gruppen R₄ substituiert ist,
• eine Cyclopropylgruppe, eine Cyclobutylgruppe, eine 3-(Benzoylamino)cyclobutyl-gruppe, eine 3-[(Pyrazin-2-ylcarbonyl)amino]-cyclobutylgruppe, eine 3-[(Methylsulfonyl)-amino]cyclobutylgruppe, eine 3-[(Phenylsulfonyl)amino]cyclobutylgruppe, eine Cyclopentylgruppe, eine Cyclohexylgruppe,
• eine Tetrahydro-2H-pyranylgruppe,
• eine Gruppe -NH-(C=O)-R₅
steht;
- R₁ für ein Wasserstoffatom, eine Ethylgruppe, eine 2,2,2-Trifluorethylgruppe oder eine Methylgruppe, die unsubstituiert oder durch eine 1,2,3-Triazolgruppe, die durch eine 3-(3-Fluorphenyl)-2-oxo-1,3-oxazolidin-5-ylmethylgruppe substituiert ist, substituiert ist, steht;
- R₂ für ein Wasserstoffatom oder eine Methylgruppe steht;
- R₃ für:
• eine Cyclohexylgruppe, eine 1-{[(Phenylsulfonyl)amino]methyl}cyclohexylgruppe oder eine 1-{[(Phenylsulfonyl)amino]methyl}cyclopentylgruppe,
• eine 5,6,7,8-Tetrahydrochinolin-5-ylgruppe
• oder eine lineare oder verzweigte Gruppe C₁₋₄-Alkyl, die unsubstituiert oder durch eine Gruppe substituiert ist, die ausgewählt ist aus:
• -NH-R₆,
• -NH-SO₂-R₇,
• -NH-(C=O)-R₈,
• einer 1-Morpholin-4-ylcyclopentylgruppe,
• einer Tetrahydro-2H-pyranylgruppe, einer Tetrahydrofuranylgruppe oder einer Morpholin-4-ylgruppe,
• einer Phenylgruppe, die unsubstituiert oder durch eine oder mehrere Gruppen, die unabhängig aus einem Chloratom und einer Gruppe -CF₃ ausgewählt sind, substituiert ist,
• einer 1H-Pyrrolo[2,3-b]pyridinylgruppe, einer 4-Methyl-5,6,7,8-tetrahydrochinazolin-2-ylgruppe, einer 6-Methoxy-1H-benzimidazol-2-ylgruppe, einer Pyridinylgruppe, die unsubstituiert oder durch eine Gruppe -CF₃ oder durch eine Morpholin-4-ylgruppe substituiert ist,
• oder alternativ dazu durch ein oder mehrere Methoxygruppen;
steht;
- oder alternativ dazu R₂ und R₃ zusammen mit dem Stickstoffatom, an das sie gebunden sind, eine -C₃₋₆-Heterocycloalkylgruppe bilden, die ausgewählt ist aus: Azetidin, Morpholin, 4-[5-(Trifluormethyl)pyridin-2-yl]piperazin;
- R₄ unabhängig für eine Gruppe steht, die ausgewählt ist aus:
• einer Hydroxylgruppe,
• einem Deuterium,
• einem Fluoratom,
• einer Cyclopropylgruppe,
• einer Phenylgruppe, die unsubstituiert oder durch eine oder mehrere Gruppen, die unabhängig aus einem Fluoratom, einer Methoxygruppe, einer -CH₂OH-Gruppe und einer -CHO-Gruppe ausgewählt sind, substituiert ist,
• einer Pyridylgruppe,
• einer Morpholinylgruppe, einer Tetrahydro-2H-pyranylgruppe,
• einer Methoxygruppe,
• einer Gruppe -(C=O)-NH-R₁₀,
• einer Gruppe -NH-R₁₁,
• einer Gruppe -NH-(C=O)-R₁₂,
• oder einer Gruppe -NH(SO₂)-R₁₃;
- R₅ für eine Pyridylgruppe steht;
- R₆ für eine Chinolylgruppe steht, wobei die Chinolylgruppe unsubstituiert oder durch ein Chloratom substituiert ist;
- R₇ für eine -CF₃-Gruppe, eine Phenyl-, Pyridyl-, Pyrazolyl-, 1H-Pyrrolo[2,3-b]pyridyl-oder Indolylgruppe steht, wobei die Phenyl-, Pyridyl-, Pyrazolyl-, 1H-Pyrrolo[2,3-b]pyridyl-oder Indolylgruppen unsubstituiert oder durch ein oder mehrere Gruppen R_{1'} substituiert sind;
- R₈ für eine Pyrazinylgruppe steht, wobei die Pyrazinylgruppe unsubstituiert oder durch eine oder mehrere Gruppen R_{2'} substituiert ist;
- R₁₀ für eine 1,8-Naphthyridinylgruppe, die durch eine Methylgruppe substituiert ist, steht;
- R₁₁ für eine Tetrahydrothiophen-1,1-dioxid-, Chinolyl-, Pyridyl- oder Benzylgruppe steht, wobei die Chinolyl-, Pyridyl- oder Benzylgruppen unsubstituiert oder durch ein Chloratom, eine Hydroxylgruppe, eine Nitrogruppe oder eine Methylgruppe substituiert sind;
- R₁₂ für:
• eine tert-Butoxygruppe,
• eine Gruppe -C₁₋₄-Alkyl, die unsubstituiert oder durch eine Gruppe, die aus einer Gruppe -NR₁₄R₁₅, Pyridyl oder Pyrazolyl ausgewählt ist, substituiert ist, wobei die Pyridyl- oder Pyrazolylgruppen unsubstituiert oder durch eine Methylgruppe substituiert sind,
• ein Pyrazinyl oder Pyridyl, das unsubstituiert oder durch eine oder mehrere Gruppen, die aus einer Hydroxylgruppe und einer Methylgruppe ausgewählt sind, substituiert ist,
steht;
- R₁₃ für:
• eine Gruppe -CF₃,
• eine Phenylgruppe, die unsubstituiert oder durch eine Nitrogruppe substituiert ist,
• oder eine Pyridylgruppe, die unsubstituiert oder durch eine Gruppe -NR₁₆FR₁₇ substituiert ist,
steht;
- R₁₄, R₁₅, R₁₆ und R₁₇ jeweils unabhängig für:
• ein Wasserstoffatom,
• eine Methylgruppe oder eine Isopropylgruppe stehen;
- R_{1'} für:
• ein Fluoratom, ein Chloratom,
• eine Methoxygruppe,
• eine Gruppe -CF₃,
• eine Gruppe -OCF₃,
• eine Nitrogruppe,
• eine Gruppe -NH₂,
• eine Gruppe -NHCH₃
steht;
- R_{2'} für:
• eine Hydroxylgruppe,
• eine Methylgruppe
steht.

6. Verbindung der Formel (I) nach Anspruch 1, **dadurch gekennzeichnet, dass**:
- Y für ein Wasserstoffatom oder eine Gruppe - (C=O)-NR₂R₃ steht;
- Z für:
• ein Wasserstoffatom,
• eine Methylgruppe, eine Isopropylgruppe, eine 2,2-Dimethylpropylgruppe,
• eine Gruppe CD₃,
• eine 2-Fluorethylgruppe,
• eine Cyclopropylmethylgruppe,
• eine 2-Phenylethylgruppe,
• eine [(7-Methyl-1,8-naphthyridin-2-yl)amino]-4-oxobutylgruppe,
• eine 2-{[(2-Nitrophenyl)sulfonyl]amino}ethylgruppe,
• eine Cyclopropylgruppe,
• eine Tetrahydro-2H-pyranylgruppe steht;
- R₁ für ein Wasserstoffatom, eine Ethylgruppe, eine 2,2,2-Trifluorethylgruppe oder eine Methylgruppe steht;
- R₂ für ein Wasserstoffatom oder eine Methylgruppe steht;
- R₃ für:
• eine Methylgruppe,
• eine 2-{[(2,6-Difluorphenyl)sulfonyl]amino}-1,1-dimethylethylgruppe,
• eine 1,1-Dimethyl-2-({[4-(trifluormethyl)-phenyl]sulfonyl}amino)ethylgruppe,
• eine 2-{[(2-Fluorphenyl)sulfonyl]amino}-1,1-dimethylethylgruppe,
• eine 1,1-Dimethyl-2-({[2-(trifluormethoxy)-phenyl]sulfonyl}amino)ethylgruppe,
• eine 1,1-Dimethyl-2-({[4-(trifluormethoxy)-phenyl]sulfonyl}amino)ethylgruppe,
• eine 2-Methyl-1-[(phenylsulfonyl)amino]-propan-2-ylgruppe,
• eine 2-Methyl-1-{[(5-nitro-1H-pyrazol-4-yl)-sulfonyl]amino}propan-2-ylgruppe,
• eine 2-Methyl-1-{[(trifluormethyl)sulfonyl]-amino}propan-2-ylgruppe,
• eine 2-Methyl-1-{[(2-nitrophenyl)sulfonyl]-amino}propan-2-ylgruppe,
• eine 1-{[(5-Hydroxypyrazin-2-yl)carbonyl]-amino}-2-methylpropan-2-ylgruppe,
• eine 1,1-Dimethyl-2-morpholin-4-ylethyl-gruppe,
• eine Benzylgruppe,
• eine 2-(4-Pyridyl)ethylgruppe
steht.

7. Verbindung der Formel (I) nach einem der Ansprüche 1 bis 6, **dadurch gekennzeichnet, dass** sie den folgenden Verbindungen entspricht:
• 3-Methylbutansäure-(2R,3S,4R,5R,7S,9S,10S,11R,12S,13R)-7-[(benzylcarbamoyl)oxy]-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5S,7R)-2,4,5-trimethyl-1,4-oxazepan-7-yl]oxy}oxacyclotetradecan-4-ylester;
• 3-Methylbutansäure-(2R,3S,4R,5R,7S,9S,10S,11R,12S,13R)-7-[(benzylcarbamoyl)oxy]-2-(1{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5R,7R)-2,4,5-trimethyl-1,4-oxazepan-7-yl]oxy}oxacyclotetradecan-4-ylester;
• 3-Methylbutansäure-(2R,3S,4R,5R,7S,9S,10S,11R,12S,13R)-12-{[(2S,7R)-4-cyclopropyl-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-7-{[(1-{[(5-hydroxypyrazin-2-yl)carbonyl]amino}-2-methylpropan-2-yl)carbamoyl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-7-[(benzylcarbamoyl)oxy]-12-{[(2S,5R,7R)-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-7-({[2-(pyridin-4-yl)ethyl]carbamoyl}oxy)-12-{[(2S,5S,7R)-2,4,5-trimethyl-1,4-oxazepan-7-yl]oxy}oxacyclotetradecan-4-ylester;
• 3-Methylbutansäure-(2R,3S,4R,5R,7S,9S,10S,11R,12S,13R)-7-[(benzylcarbamoyl)oxy]-12-{[(2S,7R)-4-cyclopropyl-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-7-[(benzylcarbamoyl)oxy]-12-{[(2S,7R)-2,5-dimethyl-4-(²H₃)methyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-7-[(dimethylcarbamoyl)oxy]-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5R,7R)-2,4,5-trimethyl-1,4-oxazepan-7-yl]oxy}oxacyclotetradecan-4-ylester;
• 3-Methylbutansäure-(2R,3S,4R,5R,7S,9S,10S,11R,12S,13R)-7-[(benzylcarbamoyl)oxy]-12-{[(2S,7R)-2,5-dimethyl-4-(2-{[(2-nitrophenyl)sulfonyl]-amino}ethyl)-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-7-[(benzylcarbamoyl)oxy]-12-{[(2S,7R)-4-(2-fluorethyl)-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-7-[(benzylcarbamoyl)oxy]-12-{[(2S,7R)-2,5-dimethyl-4-{4-[(7-methyl-1,8-naphthyridin-2-yl)amino]-4-oxobutyl}-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-7-[(benzylcarbamoyl)oxy]-12-{[(2S,7R)-4-(2,2-dimethylpropyl)-2,5-dimethyl-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxyl-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-12-{[(2S,7R)-2,5-dimethyl-4-(2-phenylethyl)-1,4-oxazepan-7-yl]oxy}-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-7-{[(1-{[(5-hydroxypyrazin-2-yl)carbonyl]amino}-2-methylpropan-2-yl)carbamoyl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(5-nitro-1H-pyrazol-4-yl)sulfonyl]amino}propan-2-yl)carbamoyl]oxy}-6,14-dioxo-12-{[(2S,7R)-2,4,5-trimethyl-1,4-oxazepan-7-yl]oxy}oxacyclo-tetradecan-4-ylester;
• 3-Methylbutansäure-(2R,3S,4R,5R,7S,9S,10S,11R,12S,13R)-7-hydroxy-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,5R,7R)-2,4,5-trimethyl-1,4-oxazepan-7-yl]oxy}oxacyclotetradecan-4-ylester;
• 3-Methylbutansäure-(2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(trifluormethyl)sulfonyl]amino}propan-2-yl)carbamoyl]oxy}-6,14-dioxo-12-{[(2S,7R)-2,4,5-trimethyl-1,4-oxazepan-7-yl]oxy}oxacyclo-tetradecan-4-ylester;
• 3-Methylbutansäure-(2R,3S,4R,5R,7S,9S,10S,11R,12S,13R)-12-{[(2S,7R)-2,5-dimethyl-4-(2-phenylethyl)-1,4-oxazepan-7-yl]oxy}-7-hydroxy-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclo-tetradecan-4-ylester;
• 3-Methylbutansäure-(2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(2-nitrophenyl)sulfonyl]amino}propan-2-yl)carbamoyl]oxy}-6,14-dioxo-12-{[(2S,5R,7R)-2,4,5-trimethyl-1,4-oxazepan-7-yl]oxy}oxacyclotetradecan-4-ylester;
• 3-Methylbutansäure-(2R,3S,4R,5R,7S,9S,105,11R,125,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-{[(2-methyl-1-{[(2-nitrophenyl)sulfonyl]amino}propan-2-yl)carbamoyl]oxy}-6,14-dioxo-12-{[(2S,5S,7R)-2,4,5-trimethyl-1,4-oxazepan-7-yl]oxy}oxacyclotetradecan-4-ylester;
• 3-Methylbutansäure-(2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-[({2-methyl-1-[(phenylsulfonyl)amino]propan-2-yl}carbamoyl)oxy]-6,14-dioxo-12-{[(2S,5S,7R)-2,4,5-trimethyl-1,4-oxazepan-7-yl]oxy}oxacyclotetradecan-4-ylester;
• 3-Methylbutansäure-(2R,3S,4R,5R,7S,9S,10S,11R,12S,13R)-2-(1-{[(2R,3R,4R,5R,6R)-5-hydroxy-3,4-dimethoxy-6-methyltetrahydro-2H-pyran-2-yl]oxy}propan-2-yl)-10-{[(2S,3R,6R)-3-hydroxy-4-(methoxyimino)-6-methyltetrahydro-2H-pyran-2-yl]oxy}-3,5,7,9,11,13-hexamethyl-7-[({2-methyl-1-[(phenylsulfonyl)amino]propan-2-yl}carbamoyl)oxy]-6,14-dioxo-12-{[(2S,5R,7R)-2,4,5-trimethyl-1,4-oxazepan-7-yl]oxy}oxacyclotetradecan-4-ylester;
• 3-Methylbutansäure (2R,3S,4R,5R,7S,9S,10S,11R,12S,13R)-7-[({1,1-dimethyl-2-[(phenylsulfonyl)amino]ethyl}-carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-12-{[(2S,7R)-4-isopropyl-2,5-dimethyl-1,4-oxazepan-7-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-12-{[(2S,5R,7R)-4-(cyclopropylmethyl)-2,5-dimethyl-1,4-oxazepan-7-yl]oxy}-7-[({1,1-dimethyl-2-[(phenylsulfonyl)amino]ethyl}carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-12-{[(2S,7R)-4-(cyclopropylmethyl)-2,5-dimethyl-1,4-oxazepan-7-yl]oxy}-7-[({1,1-dimethyl-2-[(phenylsulfonyl)amino]ethyl}carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-7-[({1,1-dimethyl-2-[(phenylsulfonyl)amino]ethyl}-carbamoyl)oxy]-12-{[(2S,7R)-2,5-dimethyl-4-(tetrahydro-2H-pyran-4-yl)-1,4-oxazepan-7-yl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-12-{[(2S,5S,7R)-4-(cyclopropylmethyl)-2,5-dimethyl-1,4-oxazepan-7-yl]oxy}-7-[({1,1-dimethyl-2-[(phenylsulfonyl)amino]ethyl}carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-7-[({1,1-dimethyl-2-[(phenylsulfonyl)amino]ethyl}-carbamoyl)oxy]-12-{[(2S,5R,7R)-4-(2,2-dimethylpropyl)-2,5-dimethyl-1,4-oxazepan-7-yl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-7-[({1,1-dimethyl-2-[(phenylsulfonyl)amino]ethyl}-carbamoyl)oxy]-12-{[(2S,7R)-4-(2,2-dimethylpropyl)-2,5-dimethyl-1,4-oxazepan-7-yl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxooxacyclotetradecan-4-ylester;
• 3-Methylbutansäure-(2R,3S,4R,5R,7S,9S,10S,11R,12S,13R)-7-{[(1,1-dimethyl-2-morpholin-4-ylethyl)carbamoyl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-ylester;
• 3-Methylbutansäure-(2R,3S,4R,5R,7S,9S,10S,11R,12S,13R)-7-{[(2-{[(2,6-difluorphenyl)sulfonyl]amino}-1,1-dimethylethyl)carbamoyl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-ylester;
• 3-Methylbutansäure-(2R,3S,4R,5R,7S,9S,10S,11R,12S,13R)-7-({[1,1-dimethyl-2-({[4-(trifluormethyl)phenyl]-sulfonyl}amino)ethyl]carbamoyl}oxy)-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-ylester;
• 3-Methylbutansäure-(2R,3S,4R,5R,7S,9S,10S,11R,12S,13R)-7-{[(2-{[(2-fluorphenyl)sulfonyl]amino}-1,1-dimethylethyl)carbamoyl]oxy}-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-ylester;
• 3-Methylbutansäure-(2R,3S,4R,5R,7S,9S,10S,11R,12S,13R)-7-({[1,1-dimethyl-2-({[2-(trifluormethoxy)phenyl]-sulfonyl}amino)ethyl]carbamoyl}oxy)-2-(2-{[(2R,3R,4R,5R,6R)-5-hydroxy-3,4-dimethoxy-6-methyltetrahydro-2H-pyran-2-yl]oxy}-1-methylethyl)-10-{[(2S,3R,6R)-3-hydroxy-4-(methoxyimino)-6-methyltetrahydro-2H-pyran-2-yl]oxy}-3,5,7,9,11,13-hexamethyl-6,14-dioxo-12-{[(2S,7R)-2,4,5-trimethyl-1,4-oxazepan-7-yl]oxy}oxacyclotetradecan-4-ylester;
• 3-Methylbutansäure-(2R, 3S, 4R, 5R, 7S, 9S, 10S, 11R, 12S, 13R)-7-({[1,1-dimethyl-2-({[4-(trifluormethoxy)phenyl]-sulfonyl}amino)ethyl]carbamoyl}oxy)-2-(2-{[(2R, 3R, 4R, 5R, 6R)-5-hydroxy-3,4-dimethoxy-6-methyltetrahydro-2H-pyran-2-yl]oxy}-1-methylethyl)-10-{[(2S, 3R, 6R)-3-hydroxy-4-(methoxyimino)-6-methyltetrahydro-2H-pyran-2-yl]oxyl-3, 5, 7, 9, 11, 13-hexamethyl-6, 14-dioxo-12-{[(2S,7R)-2,4,5-trimethyl-1,4-oxazepan-7-yl]oxy}oxacyclotetradecan-4-ylester;
• 3-Methylbutansäure-(2R, 3S, 4R, 5R, 7S, 9S, 10S, 11R, 12S, 13R) -7-[({1,1-dimethyl-2-[(phenylsulfonyl)amino]ethyl}carbamoyl)oxy]-2-(2-{[(2R,3R,4R,5R,6R)-5-hydroxy-3,4-dimethoxy-6-methyltetrahydro-2H-pyran-2-yl]oxy}-1-methylethyl)-10-({(2S,3R,6R)-3-hydroxy-6-methyl-4-[(2,2,2-trifluorethoxy)imino]tetrahydro-2H-pyran-2-yl}oxy)-3,5,7,9,11,13-hexamethyl-6,14-dioxo-12-{[(2S,7R)-2,4,5-trimethyl-1,4-oxazepan-7-yl]oxy}oxacyclotetradecan-4-ylester;
• 3-Methylbutansäure-(2R, 3S, 4R, 5R, 7S, 9S, 10S, 11R, 12S, 13R) -7- [({1,1-dimethyl-2-[(phenylsulfonyl)amino]ethyl}-carbamoyl)oxy]-10-{[(2S,3R,6R)-4-(ethoxyimino)-3-hydroxy-6-methyltetrahydro-2H-pyran-2-yl]oxy}-2-(2-{[(2R,3R,4R,5R,6R)-5-hydroxy-3,4-dimethoxy-6-methyltetrahydro-2H-pyran-2-yl]oxy}-1-methylethyl)-3,5,7,9,11,13-hexamethyl-6,14-dioxo-12-{[(2S,5R,7R)-2,4,5-trimethyl-1,4-oxazepan-7-yl]oxy}oxacyclotetradecan-4-ylester.

8. Verfahren zur Herstellung einer Verbindung der Formel (I) nach Anspruch 1, in der Y für eine Gruppe - (C=O) -NR₂R₃ steht, **dadurch gekennzeichnet, dass** man:
eine Verbindung der Formel (IB): 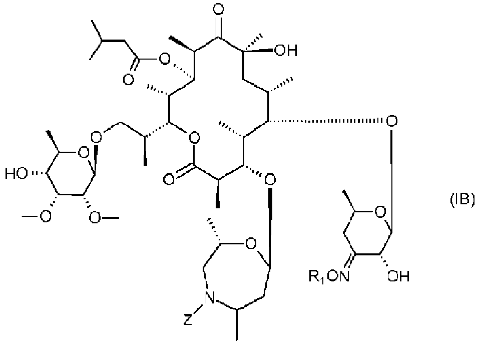 in der R₁ und Z wie für die Verbindungen der Formel (I) in Anspruch 1 definiert sind, in Gegenwart eines Carbonylderivats und einer Base mit einer Verbindung der Formel (II) HNR₂R₃, in der R₂ und R₃ wie für die Verbindungen der Formel (I) in Anspruch 1 definiert sind, umsetzt.

9. Verfahren zur Herstellung einer Verbindung der Formel (I) nach Anspruch 1, in der Y für eine Gruppe - (C=O) -NR₂R₃ steht, **dadurch gekennzeichnet, dass** man:
b-1) eine Verbindung der Formel (V): 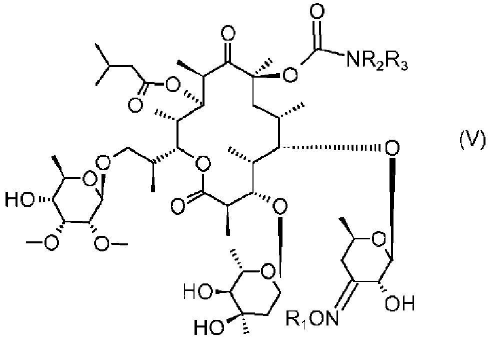 in der R₁, R₂ und R₃ wie für eine Verbindung der Formel (I) in Anspruch 1 definiert sind, mit einem Oxidationsmittel zu einer Verbindung der Formel (VI): 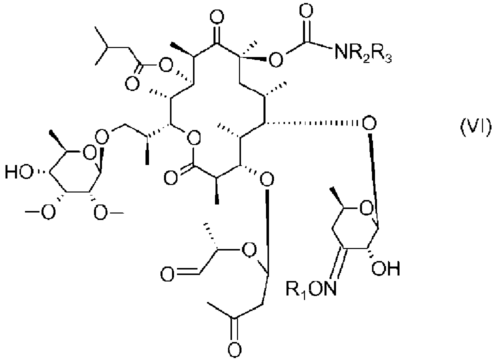 umsetzt;
b-2) die so erhaltene Verbindung der Formel (VI) in Gegenwart eines Reduktionsmittels mit einer Verbindung der Formel (VII):
ZNH₂ (VII)
in der Z wie für Verbindung (I) in Anspruch 1 definiert ist, umsetzt.

10. Verfahren zur Herstellung einer Verbindung der Formel (I) nach Anspruch 1, in der Y für ein Wasserstoffatom steht, **dadurch gekennzeichnet, dass** man:
c-1) eine Verbindung der Formel (VIII): 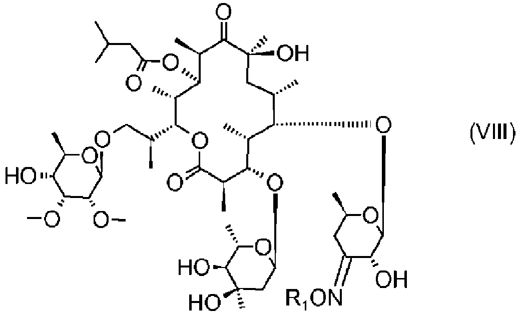 in der R₁ wie für Verbindung (I) in Anspruch 1 definiert ist, mit einem Oxidationsmittel zu einer Verbindung der Formel (IX): 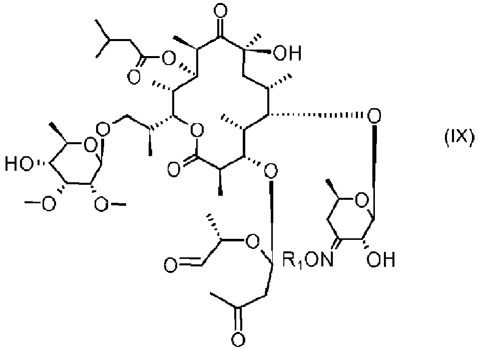 umsetzt;
c-2) die so erhaltene Verbindung der Formel (IX) in Gegenwart eines Reduktionsmittels mit einer Verbindung der Formel (VII):
ZNH₂ (VII)
in der Z wie für Verbindung (I) in Anspruch 1 definiert ist, umsetzt.

11. Verfahren zur Herstellung einer Verbindung der Formel (I) nach Anspruch 1, in der Y für eine Gruppe - (C=O) -O-R₁₈ steht, **dadurch gekennzeichnet, dass** man:
eine Verbindung der Formel (XXI): 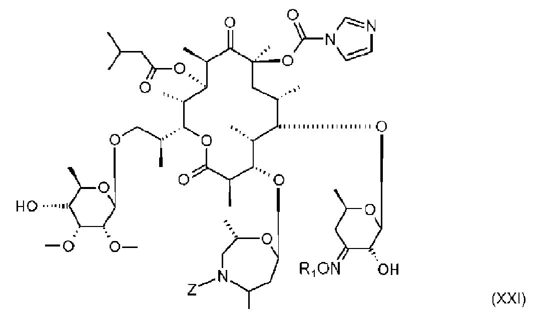 in der Z und R₁ wie für eine Verbindung der Formel (I) in Anspruch 1 definiert sind, in Gegenwart einer Base mit einem Alkohol der Formel HO-R₁₈ (XXII), in der R₁₈ wie für eine Verbindung der Formel (I) in Anspruch 1 definiert ist, umsetzt.

12. Verbindung der Formel (V): 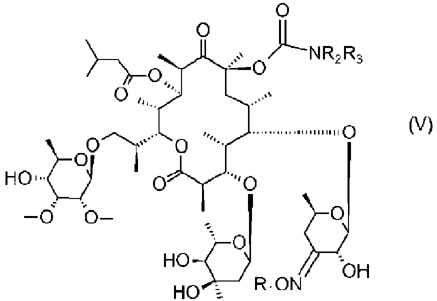 in der:
- R₁, R₂ und R₃ wie für die Verbindungen der Formel (I) in Anspruch 1 definiert sind.

13. Verbindung der Formel (VIII): 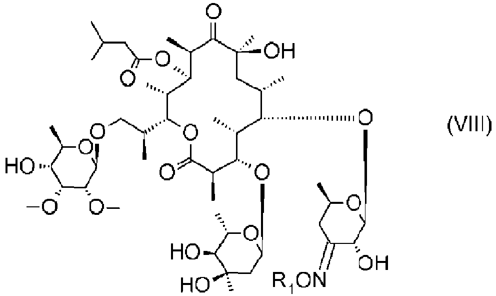 in der:
- R₁ wie für die Verbindungen der Formel (I) in Anspruch 1 definiert ist.

14. Medikament, **dadurch gekennzeichnet, dass** es eine Verbindung der Formel (I) nach einem der Ansprüche 1 bis 7 in Form einer Base oder eines Säureadditionssalzes umfasst.

15. Pharmazeutische Zusammensetzung, **dadurch gekennzeichnet, dass** sie eine Verbindung der Formel (I) nach einem der Ansprüche 1 bis 7 in Form einer Base oder eines Säureadditionssalzes sowie mindestens einen pharmazeutisch akzeptablen Hilfsstoff umfasst.

16. Verbindung nach einem der Ansprüche 1 bis 7 zur Verwendung zur Prävention und/oder Behandlung von bakteriellen Infektionen, die durch Gram-positive Mikroorganismen und Mykobakterien verursacht werden.

17. Verbindung nach einem der Ansprüche 1 bis 7 zur Verwendung zur Prävention und/oder Behandlung von Infektionskrankheiten, die aus Tuberkulose, Lepra, Nokardiose, Diphtherie, mykobakterieller Lungen-infektion, mykobakterieller Hautinfektion, atypischer mykobakterieller Infektion und Mykobakteriose ausgewählt sind.

## Revendications

1. Composé de formule (I) : 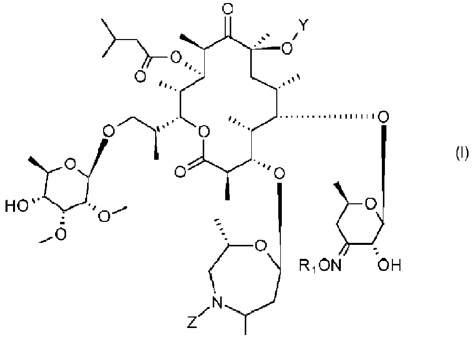 dans lequel :
- Y représente un atome d'hydrogène, un groupe - (C=O) -NR₂R₃ ou un groupe - (C=O) -O-R₁₈ ;
- Z représente :
• un atome d'hydrogène,
• un groupe - (alkyle en C₁₋₆), qui est non substitué ou substitué par un ou plusieurs groupes R₄,
• un groupe -(cycloalkyle en C₃₋₇), qui est non substitué ou substitué par un groupe -NH-(C=O)-R₁₉ ou par un groupe -NH-SO₂-R₂₀,
• un groupe - (hétérocycloalkyle en C₃₋₆),
• un groupe-NH-(C=O)-R₅ ;
- R₁ représente un atome d'hydrogène, un groupe - (alcényle en C₂₋₆), un groupe - (alcynyle en C₂₋₆) ou un groupe - (alkyle en C₁₋₆) qui est non substitué ou substitué par un groupe -(fluoroalkyle en C₁₋₄) ou par un groupe hétéroaryle qui est non substitué ou substitué par un groupe 3-(3-fluorophényl)-2-oxo-1,3-oxazolidin-5-ylméthyle ;
- R₂ représente un atome d'hydrogène ou un groupe - (alkyle en C₁₋₆) ;
- R₃ représente :
• un groupe - cycloalkyle en C₃₋₇), qui est non substitué ou substitué par un groupe -(alkyle en C₁₋₃) substitué par un groupe -NH-SO₂-R₂₁,
• un groupe hétéroaryle,
• un groupe - (alkyle en C₁₋₆) linéaire ou ramifié, qui est non substitué ou substitué par un groupe choisi parmi :
• un groupe -NH-R₆,
• un groupe -NH-SO₂-R₇,
• un groupe -NH-(C=O)-R₈,
• un groupe - cycloalkyle en C₃₋₇), qui est non substitué ou substitué par un groupe-(hétérocycloalkyle en C₃₋₆),
• un groupe - (hétérocycloalkyle en C₃₋₆),
• un groupe aryle, qui est non substitué ou substitué par un ou plusieurs groupes choisis indépendamment parmi un atome d'halogène et un groupe - (fluoroalkyle en C₁₋₄),
• un groupe hétéroaryle, qui est non substitué ou substitué par un groupe - (alkyle en C₁₋₃) , un groupe - (alcoxy en C₁₋₄), un groupe - (fluoroalkyle en C₁₋₄) ou un groupe - (hétérocycloalkyle en C₃₋₆),
• ou en variante par un ou plusieurs groupes-(alcoxy en C₁₋₄) ;
- ou en variante R₂ et R₃, conjointement avec l'atome d'azote auquel ils sont liés, constituent un groupe - (hétérocycloalkyle en C₃₋₆) choisi parmi : aziridine, azétidine, pyrrolidine, pipéridine, morpholine, thiomorpholine ou pipérazine ; ledit groupe hétérocycloalkyle étant non substitué ou substitué par un groupe hétéroaryle, ledit groupe hétéroaryle étant non substitué ou substitué par un groupe -(fluoroalkyle en C₁₋₄) ;
- R₄ représente indépendamment un groupe choisi parmi :
• un groupe hydroxyle,
• un deutérium,
• un atome d'halogène,
• un groupe - cycloalkyle en C₃₋₇),
• un groupe aryle, qui est non substitué ou substitué par un ou plusieurs groupes -R₉,
• un groupe hétéroaryle,
• un groupe - (hétérocycloalkyle en C₃₋₆),
• un groupe - (alcoxy en C₁₋₄),
• un groupe- (C=O) -NH-R₁₀,
• un groupe-NH-R₁₁,
• un groupe -NH-(C=O)-R₁₂,
• ou un groupe -NH (SO₁₃) -R₁₃ ;
- R₅ représente un groupe hétéroaryle ;
- R₆ représente un groupe hétéroaryle, qui est non substitué ou substitué par un ou plusieurs atomes d'halogène ;
- R₇ représente un groupe - (fluoroalkyle en C₁₋₄), un groupe aryle ou un groupe hétéroaryle, lesdits groupes aryle et hétéroaryle étant non substitués ou substitués par un ou plusieurs groupes R_{1'} ;
- R₈ représente un groupe hétéroaryle, qui est non substitué ou substitué par un ou plusieurs groupes R_{2'} ;
- R₉ représente un atome d'halogène, un groupe-(alcoxy en C₁₋₄), un groupe formyle (CHO) ou un groupe-(alkyle en C₁₋₄), qui est non substitué ou substitué par un groupe hydroxyle ;
- R₁₀ représente un groupe hétéroaryle, qui est non substitué ou substitué par un groupe -(alkyle en C₁₋₃) ;
- R₁₁ représente :
• un groupe - (hétérocycloalkyle en C₃₋₁₀), qui est non substitué ou substitué par un ou plusieurs groupes oxyde,
• un groupe hétéroaryle ou un groupe aryl-(alkyle en C₁₋₄), lesdits groupes hétéroaryle ou aryle étant non substitués ou substitués par un ou plusieurs groupes indépendamment choisis parmi un atome d'halogène, un groupe hydroxyle, un groupe nitro et un groupe - (alkyle en C₁₋₃) ;
- R₁₂ représente :
• un groupe- (alcoxy en C₁-₄),
• un groupe - (alkyle en C₁₋₄), qui est non substitué ou substitué par un groupe -NR₁₄R₁₅ ou par un groupe hétéroaryle, ledit groupe hétéroaryle étant non substitué ou substitué par un groupe -(alkyle en C₁₋₃),
• un groupe hétéroaryle, qui est non substitué ou substitué par un ou plusieurs groupes choisis parmi un groupe hydroxyle et un groupe - (alkyle en C₁₋₃) ;
- R₁₃ représente :
• un groupe - (alkyle en C₁₋₄),
• un groupe - (fluoroalkyle en C₁₋₄),
• un groupe aryle, qui est non substitué ou substitué par un groupe nitro,
• ou un groupe hétéroaryle, qui est non substitué ou substitué par un groupe -NR₁₆R₁₇ ;
- R₁₄, R₁₅, R₁₆ et R₁₇ représentent chacun indépendamment :
• un atome d'hydrogène,
• ou un groupe - (alkyle en C₁₋₄) ;
- R₁₈ représente un groupe - (alkyle en C₁₋₄) ou un groupe benzyle ;
- R₁₉ représente un groupe aryle ou un groupe hétéroaryle ;
- R₂₀ représente un groupe - (alkyle en C₁₋₄) ou un groupe aryle ;
- R₂₁ représente un groupe aryle ;
- R_{1'} représente :
• un atome d'halogène,
• un groupe -(alkoxy en C₁₋₄),
• un groupe - (fluoroalkyle en C₁₋₄),
• un groupe -OCF₃,
• un groupe nitro,
• un groupe -NH₂,
• un groupe -NHCH₃ ;
- R_{2'} représente :
• un groupe hydroxyle,
• un groupe - (alkyle en C₁₋₆).

2. Composé selon la revendication 1, de formule (IA) : 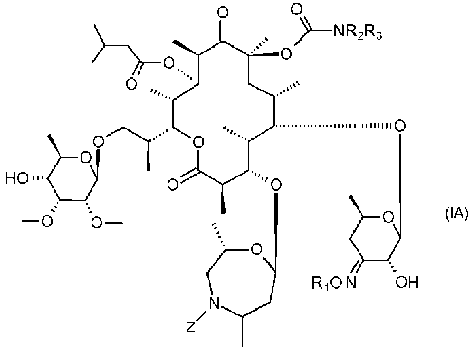 dans lequel :
- R₁, R₂, R₃ et Z sont tels que définis dans la revendication 1.

3. Composé selon la revendication 1, de formule (IB) : 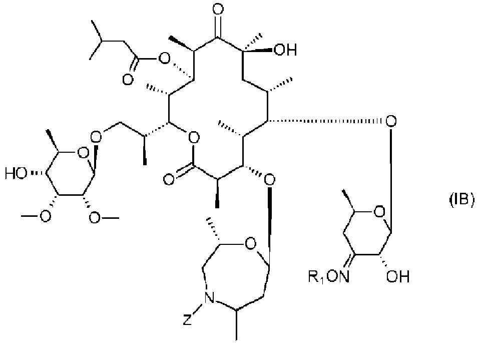 dans lequel :
- R₁ et Z sont tels que définis dans la revendication 1.

4. Composé selon la revendication 1, de formule (Ii) : 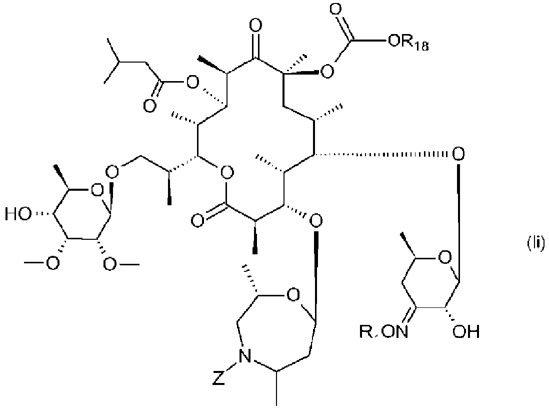 dans lequel :
- R₁, R₁₈ et Z sont tels que définis dans la revendication 1.

5. Composé de formule (I) selon la revendication 1, **caractérisé en ce que** :
- Y représente un atome d'hydrogène, un groupe - (C=O) -NR₂R₃ ou un groupe -(C=O)-OMe ;
- Z représente :
• un atome d'hydrogène,
• un groupe - (alkyle en C₁₋₆), qui est non substitué ou substitué par un ou plusieurs groupes R₄,
• un groupe cyclopropyle, un groupe cyclobutyle, un groupe 3-(benzoylamino)cyclobutyle, un groupe 3-[(pyrazin-2-ylcarbonyl)amino]cyclobutyle, un groupe 3-[(méthylsulfonyl)amino]cyclobutyle, un groupe 3-[(phénylsulfonyl)amino]cyclobutyle, un groupe cyclopentyle, un groupe cyclohexyle,
• un groupe tétrahydro-2H-pyranyle,
• un groupe -NH-(C=O)-R₅ ;
- R₁ représente un atome d'hydrogène, un groupe éthyle, un groupe 2,2,2-trifluoroéthyle ou un groupe méthyle, qui est non substitué ou substitué par un groupe 1,2,3-triazole substitué par un groupe 3-(3-fluorophényl)-2-oxo-1,3-oxazolidin-5-ylméthyle ;
- R₂ représente un atome d'hydrogène ou un groupe méthyle ;
- R₃ représente :
• un groupe cyclohexyle, un groupe 1-{[(phénylsulfonyl)amino]méthyl}cyclohexyle ou un groupe 1-{[(phénylsulfonyl)amino]méthyl}cyclopentyle,
• un groupe 5,6,7,8-tétrahydroquinoléin-5-yle,
• ou un groupe alkyle en C₁₋₄ linéaire ou ramifié, qui est non substitué ou substitué par un groupe choisi parmi :
• -NH-R₆,
• -NH-SO₂-R₇,
• -NH-(C=O)-R₈,
• un groupe 1-morpholin-4-ylcyclopentyle,
• un groupe tétrahydro-2H-pyranyle, un groupe tétrahydrofuranyle ou un groupe morpholin-4-yle,
• un groupe phényle, qui est non substitué ou substitué par un ou plusieurs groupes choisis indépendamment parmi un atome de chlore et un groupe -CF₃,
• un groupe 1H-pyrrolo[2,3-b]pyridinyle, un groupe 4-méthyl-5,6,7,8-tétrahydroquinazolin-2-yle, un groupe 6-méthoxy-1H-benzimidazol-2-yle, un groupe pyridinyle, qui est non substitué ou substitué par un groupe -CF₃ ou par un groupe morpholin-4-yle,
• ou en variante par un ou plusieurs groupes méthoxy ;
- ou en variante R₂ et R₃, conjointement avec l'atome d'azote auquel ils sont liés, constituent un groupe - (hétérocycloalkyle en C₃₋₆) choisi parmi : azétidine, morpholine, 4-[5-(trifluorométhyl)pyridin-2-yl]pipérazine ;
- R₄ représente indépendamment un groupe choisi parmi :
• un groupe hydroxyle,
• un deutérium,
• un atome de fluor,
• un groupe cyclopropyle,
• un groupe phényle, qui est non substitué ou substitué par un ou plusieurs groupes choisis indépendamment parmi un atome de fluor, un groupe méthoxy, un groupe -CH₂OH et un groupe -CHO,
• un groupe pyridyle,
• un groupe morpholinyle, un groupe tétrahydro-2H-pyranyle,
• un groupe méthoxy,
• un groupe- (C=O) -NH-R₁₀,
• un groupe-NH-R₁₁,
• un groupe-NH-(C=O)-R₁₂,
• ou un groupe -NH(SO₂)-R₁₃ ;
- R₅ représente un groupe pyridyle ;
- R₆ représente un groupe quinolyle, ledit groupe quinolyle étant non substitué ou substitué par un atome de chlore ;
- R₇ représente un groupe -CF₃, un groupe phényle, pyridyle, pyrazolyle, 1H-pyrrolo[2,3-b]pyridyle ou indolyle, lesdits groupes phényle, pyridyle, pyrazolyle, 1H-pyrrolo[2,3-b]pyridyle ou indolyle étant non substitués ou substitués par un ou plusieurs groupes R_{1'} ;
- R₈ représente un groupe pyrazinyle, ledit groupe pyrazinyle étant non substitué ou substitué par un ou plusieurs groupes R_{2'} ;
- R₁₀ représente un groupe 1,8-naphtyridinyle substitué par un groupe méthyle ;
- R₁₁ représente un groupe tétrahydrothiophéne-1,1-dioxyde, quinolyle, pyridyle ou benzyle, lesdits groupes quinolyle, pyridyle ou benzyle étant non substitués ou substitués par un atome de chlore, un groupe hydroxyle, un groupe nitro ou un groupe méthyle ;
- R₁₂ représente :
• un groupe tert-butoxy,
• un groupe - (alkyle en C₁₋₄), qui est non substitué ou substitué par un groupe choisi parmi un groupe -NR₁₄R₁₅, pyridyle ou pyrazolyle, lesdits groupes pyridyle ou pyrazolyle étant non substitués ou substitués par un groupe méthyle,
• un groupe pyrazinyle ou pyridyle, qui est non substitué ou substitué par un ou plusieurs groupes choisis parmi un groupe hydroxyle et un groupe méthyle ;
- R₁₃ représente :
• un groupe -CF₃,
• un groupe phényle, qui est non substitué ou substitué par un groupe nitro,
• ou un groupe pyridyle, qui est non substitué ou substitué par un groupe -NR₁₆R₁₇ ;
- R₁₄, R₁₅, R₁₆ et R₁₇ représentent chacun indépendamment :
• un atome d'hydrogène,
• un groupe méthyle ou un groupe isopropyle ;
- R_{1'} représente :
• un atome de fluor, un atome de chlore,
• un groupe méthoxy,
• un groupe -CF₃,
• un groupe -OCF₃,
• un groupe nitro,
• un groupe -NH₂,
• un groupe -NHCH₃ ;
- R₂' représente :
• un groupe hydroxyle,
• un groupe méthyle.

6. Composé de formule (I) selon la revendication 1, **caractérisé en ce que** :
- Y représente un atome d'hydrogène ou un groupe -(C=O)-NR₂R_{3 ;}
- Z représente :
• un atome d'hydrogène,
• un groupe méthyle, un groupe isopropyle, un groupe 2,2-diméthylpropyle,
• un groupe CD₃,
• un groupe 2-fluoroéthyle,
• un groupe cyclopropylméthyle,
• un groupe 2-phényléthyle,
• un groupe [(7-méthyl-1,8-naphtyridin-2-yl)amino]-4-oxobutyle,
• un groupe 2-{[(2-nitrophényl)sulfonyl]amino}éthyle,
• un groupe cyclopropyle,
• un groupe tétrahydro-2H-pyranyle ;
- R₁ représente un atome d'hydrogène, un groupe éthyle, un groupe 2,2,2-trifluoroéthyle ou un groupe méthyle ;
- R₂ représente un atome d'hydrogène ou un groupe méthyle ;
- R₃ représente :
• un groupe méthyle,
• un groupe 2-{[(2,6-difluorophényl)sulfonyl]amino}-1,1-diméthyléthyle,
• un groupe 1,1-diméthyl-2-({[4-(trifluorométhyl)phényl]sulfonyl}amino)éthyle,
• un groupe 2-{[(2-fluorophényl)sulfonyl]amino}-1,1-diméthyléthyle,
• un groupe 1,1-diméthyl-2-({[2-(trifluorométhoxy)phényl]sulfonyl}amino)éthyle,
• un groupe 1,1-diméthyl-2-({[4-(trifluorométhoxy)phényl]sulfonyl}amino)éthyle,
• un groupe 2-méthyl-1-[(phénylsulfonyl)amino]propan-2-yle,
• un groupe 2-méthyl-1-{[(5-nitro-1H-pyrazol-4-yl)sulfonyl]amino}propan-2-yle,
• un groupe 2-méthyl-1-{[(trifluorométhyl)sulfonyl]amino}propan-2-yle,
• un groupe 2-méthyl-1-{[(2-nitrophényl)sulfonyl]amino}propan-2-yle,
• un groupe 1-{[(5-hydroxypyrazin-2-yl)carbonyl]amino}-2-méthylpropan-2-yle,
• un groupe 1,1-diméthyl-2-morpholin-4-yléthyle,
• un groupe benzyle,
• un groupe 2-(4-pyridyl)éthyle.

7. Composé de formule (I) selon l'une quelconque des revendications 1 à 6, **caractérisé en ce qu'**il correspond aux composés suivants :
• 3-méthylbutanoate de (2R, 3S, 4R, 5R, 7S, 9S, 10S, 11R, 12S, 13R)-7-[(benzylcarbamoyl)oxy]-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxo-12-{[(2S,5S,7R)-2,4,5-triméthyl-l,4-oxazépan-7-yl]oxy}oxacyclotétradécan-4-yle ;
• 3-méthylbutanoate de (2R, 3S, 4R, 5R, 7S, 9S, 10S, 11R, 12S, 13R)-7-[(benzylcarbamoyl)oxy]-2-(1{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxo-12-{[(2S,5R,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-12-{[(2S,7R)-4-cyclopropyl-2,5-diméthyl-1,4-oxazépan-7-yl]oxy}-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-7-{[(1-{[(5-hydroxypyrazin-2-yl)carbonyl]amino}-2-méthylpropan-2-yl)carbamoyl]oxy}-3,5,7,9,11,13-hexaméthyl-6,14-dioxooxacyclotétradécan-4-yle ;
• 3-méthylbutanoate de (2R, 3S, 4R, 5R, 7S, 9S, 10S, 11R, 12S,13R)-7-[(benzylcarbamoyl)oxy]-12-{[(2S,5R,7R)-2,5-diméthyl-1,4-oxazépan-7-yl]oxy}-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxo-7-({[2-(pyridin-4-yl)éthyl]-carbamoyl}oxy)-12-{[(2S,5S,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-7-[(benzylcarbamoyl)oxy]-12-{[(2S,7R)-4-cyclopropyl-2,5-diméthyl-1,4-oxazépan-7-yl]oxy}-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-7-[(benzylcarbamoyl)oxy]-12-{[(2S,7R)-2,5-diméthyl-4-(²H₃)méthyl-1,4-oxazépan-7-yl]oxy}-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-7-[(diméthylcarbamoyl)oxy]-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxo-12-{[(2S,5R,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-7-[(benzylcarbamoyl)oxy]-12-{[(2S,7R)-2,5-diméthyl-4-(2-{[(2-nitrophényl)sulfonyl]amino}éthyl)-1,4-oxazépan-7-yl]oxy}-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-7-[(benzylcarbamoyl)oxy]-12-{[(2S,7R)-4-(2-fluoroéthyl)-2,5-diméthyl-1,4-oxazépan-7-yl]oxy}-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-7-[(benzylcarbamoyl)oxy]-12-{[(2S,7R)-2,5-diméthyl-4-{4-[(7-méthyl-1,8-naphtyridin-2-yl)amino]-4-oxobutyl}-1,4-oxazépan-7-yl]oxy}-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-7-[(benzylcarbamoyl)oxy]-12-{[(2S,7R)-4-(2,2-diméthylpropyl)-2,5-diméthyl-1,4-oxazépan-7-yl]oxy}-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-12-{[(2S,7R)-2,5-diméthyl-4-(2-phényléthyl)-1,4-oxazépan-7-yl]oxy}-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-7-{[(1-{[(5-hydroxypyrazin-2-yl)carbonyl]amino}-2-méthylpropan-2-yl)carbamoyl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-ylloxyl-3,5,7,9,11,13-hexaméthyl-7-{[(2-méthyl-1-{[(5-nitro-1H-pyrazol-4-yl)sulfonyl]amino}propan-2-yl)carbamoyl]oxy}-6,14-dioxo-12-{[(2S,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-7-hydroxy-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-ylloxyl-3,5,7,9,11,13-hexaméthyl-6,14-dioxo-12-{[(2S,5R,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-ylloxyl-3,5,7,9,11,13-hexaméthyl-7-{[(2-méthyl-1-{[(trifluorométhyl)sulfonyl]amino}propan-2-yl)carbamoyl]oxy}-6,14-dioxo-12-{[(2S,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-12-{[(2S,7R)-2,5-diméthyl-4-(2-phényléthyl)-1,4-oxazépan-7-yl]oxy}-7-hydroxy-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-2-(1-{[(2R,3R,4R,5R,6R)-5-hydroxy-3,4-diméthoxy-6-méthyitétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-7-{[(2-méthyl-1-{[(2-nitrophényl)sulfonyl]amino}propan-2-yl)carbamoyl]oxy}-6,14-dioxo-12-{[(2S,5R,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-7-{[(2-méthyl-1-{[(2-nitrophényl)sulfonyl]amino}propan-2-yl)carbamoyl]oxy}-6,14-dioxo-12-{[(2S,5S,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-7-[({2-méthyl-1-[(phénylsulfonyl)amino]propan-2-yl}carbamoyl)oxy]-6,14-dioxo-12-{[(2S,5S,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-2-(1-{[(2R,3R,4R,5R,6R)-5-hydroxy-3,4-diméthoxy-6-méthyltétrahydro-2H-pyran-2-yl]oxy}propan-2-yl)-10-{[(2S,3R,6R)-3-hydroxy-4-(méthoxyimino)-6-méthyltétrahydro-2H-pyran-2-yl]oxy}-3,5,7,9,11,13-hexaméthyl-7-[({2-méthyl-1-[(phénylsulfonyl)amino]propan-2-yl}carbamoyl)oxy]-6,14-dioxo-12-{[(2S,5R,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-7-[({1,1-diméthyl-2-[(phénylsulfonyl)amino]éthyl}carbamoyl)oxy]-2-(2-{[(2R,3R,4R,5R,6R)-5-hydroxy-3,4-diméthoxy-6-méthyltétrahydro-2H-pyran-2-yl]oxy}-1-méthyléthyl)-10-{[(2S,3R,6R)-3-hydroxy-4-(méthoxyimino)-6-méthyltétrahydro-2H-pyran-2-yl]oxy}-12-{[(2S,7R)-4-isopropyl-2,5-diméthyl-1,4-oxazépan-7-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-12-{[(2S,5R,7R)-4-(cyclopropylméthyl)-2,5-diméthyl-1,4-oxazépan-7-yl]oxy}-7-[({1,1-diméthyl-2-[(phénylsulfonyl)amino]éthyl}carbamoyl)oxy]-2-(2-{[(2R,3R,4R,5R,6R)-5-hydroxy-3,4-diméthoxy-6-méthyltétrahydro-2H-pyran-2-ylloxyl-1-méthyléthyl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-12-{[(2S,7R)-4-(cyclopropylméthyl)-2,5-diméthyl-1,4-oxazépan-7-yl]oxy}-7-[({1,1-diméthyl-2-[(phénylsulfonyl)amino]éthyl}carbamoyl)oxy]-2-(2-{[(2R,3R,4R,5R,6R)-5-hydroxy-3,4-diméthoxy-6-méthyltétrahydro-2H-pyran-2-yl]oxy}-1-méthyléthyl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-7-[({1,1-diméthyl-2-[(phénylsulfonyl)amino]éthyl}carbamoyl)oxy]-12-{[(2S,7R)-2,5-diméthyl-4-(tétrahydro-2H-pyran-4-yl)-1,4-oxazépan-7-yl]oxy}-2-(2-{[(2R,3R,4R,5R,6R)-5-hydroxy-3,4-diméthoxy-6-méthyltétrahydro-2H-pyran-2-yl]oxy}-1-méthyléthyl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-12-{[(2S,5S,7R)-4-(cyclopropylméthyl)-2,5-diméthyl-1,4-oxazépan-7-yl]oxy}-7-[({1,1-diméthyl-2-[(phénylsulfonyl)amino]éthyl}carbamoyl)oxy]-2-(2-{[(2R,3R,4R,5R,6R)-5-hydroxy-3,4-diméthoxy-6-méthyltétrahydro-2H-pyran-2-yl]oxy}-1-méthyléthyl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-7-[({1,1-diméthyl-2-[(phénylsulfonyl)amino]éthyl}carbamoyl)oxy]-12-{[(2S,5R,7R)-4-(2,2-diméthylpropyl)-2,5-diméthyl-1,4-oxazépan-7-yl]oxy}-2-(2-{[(2R,3R,4R,5R,6R)-5-hydroxy-3,4-diméthoxy-6-méthyltétrahydro-2H-pyran-2-yl]oxy}-1-méthyléthyl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-7-[({1,1-diméthyl-2-[(phénylsulfonyl)amino]éthyl}carbamoyl)oxy]-12-{[(2S,7R)-4-(2,2-diméthylpropyl)-2,5-diméthyl-1,4-oxazépan-7-yl]oxy}-2-(2-{[(2R,3R,4R,5R,6R)-5-hydroxy-3,4-diméthoxy-6-méthyltétrahydro-2H-pyran-2-yl]oxy}-1-méthyléthyl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxooxacyclotétradécan-4-yle ;
• 3-méthylbutanoate de (2R,3S,4R,5R,7S,9S,10S,11R,12S,13R)-7-{[(1,1-diméthyl-2-morpholin-4-yléthyl)carbamoyl]oxy}-2-(2-{[(2R,3R,4R,5R,6R)-5-hydroxy-3,4-diméthoxy-6-méthyltétrahydro-2H-pyran-2-ylloxyl-1-méthyléthyl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxo-12-{[(2S,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-7-{[(2-{[(2,6-difluorophényl)sulfonyl]amino}-1,1-diméthyléthyl)carbamoyl]oxy}-2-(2-{[(2R,3R,4R,5R,6R)-5-hydroxy-3,4-diméthoxy-6-méthyltétrahydro-2H-pyran-2-yl]oxy}-1-méthyléthyl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxo-12-{[(2S,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-7-({[1,1-diméthyl-2-({[4-(trifluorométhyl)phényl]sulfonyl}amino)éthyl]-carbamoyl}oxy)-2-(2-{[(2R,3R,4R,5R,6R)-5-hydroxy-3,4-diméthoxy-6-méthyltétrahydro-2H-pyran-2-yl]oxy}-1-méthyléthyl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxo-12-{[(2S,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-7-{[(2-{[(2-fluorophényl)sulfonyl]amino}-1,1-diméthyléthyl)carbamoyl]oxy}-2-(2-{[(2R,3R,4R,5R,6R)-5-hydroxy-3,4-diméthoxy-6-méthyltétrahydro-2H-pyran-2-yl]oxy}-1-méthyléthyl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxo-12-{[(2S,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-7-({[1,1-diméthyl-2-({[2-(trifluorométhoxy)phényl]sulfonyl}amino)éthyl]-carbamoyl}oxy)-2-(2-{[(2R,3R,4R,5R,6R)-5-hydroxy-3,4-diméthoxy-6-méthyltétrahydro-2H-pyran-2-yl]oxy}-1-méthyléthyl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxo-12-{[(2S,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-7-({[1,1-diméthyl-2-({[4-(trifluorométhoxy)phényl]sulfonyl}amino)éthyl]-carbamoyl}oxy)-2-(2-{[(2R,3R,4R,5R,6R)-5-hydroxy-3,4-diméthoxy-6-méthyltétrahydro-2H-pyran-2-yl]oxy}-1-méthyléthyl)-10-{[(2S,3R,6R)-3-hydroxy-4-(méthoxyimino)-6-méthyltétrahydro-2H-pyran-2-yl]oxy}-3,5,7,9,11,13-hexaméthyl-6,14-dioxo-12-{[(2S,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-7-[({1,1-diméthyl-2-[(phénylsulfonyl)amino]éthyl}carbamoyl)oxy]-2-(2-{[(2R,3R,4R,5R,6R)-5-hydroxy-3,4-diméthoxy-6-méthyltétrahydro-2H-pyran-2-ylloxyl-1-méthyléthyl)-10-({(2S,3R,6R)-3-hydroxy-6-méthyl-4-[(2,2,2-trifluoroéthoxy)imino]tétrahydro-2H-pyran-2-yl}oxy)-3,5,7,9,11,13-hexaméthyl-6,14-dioxo-12-{[(2S,7R)-2,4,5-triméthyl-1,4-oxazépan-7-yl]oxy}oxacyclotétradécan-4-yle ;
• 3-méthylbutanoate de (2R,3S,4R,5R,7S,9S,10S,11R,12S,13R)-7-[({1,1-diméthyl-2-[(phénylsulfonyl)amino]éthyl}carbamoyl)oxy]-10-{[(2S,3R,6R)-4-(éthoxyimino)-3-hydroxy-6-méthyltétrahydro-2H-pyran-2-yl]oxy}-2-(2-{[(2R,3R,4R,5R,6R)-5-hydroxy-3,4-diméthoxy-6-méthyltétrahydro-2H-pyran-2-yl]oxy}-1-méthyléthyl)-3,5,7,9,11,13-hexaméthyl-6,14-dioxo-12-{[(2S,5R,7R)-2,4,5-triméthyl-1,4-oxazépan-7-yl]oxy}oxacyclotétradécan-4-yle.

8. Procédé de préparation d'un composé de formule (I) selon la revendication 1, dans lequel Y représente un groupe - (C=O) -NR₂R₃, **caractérisé en ce que** :
un composé de formule (IB) : 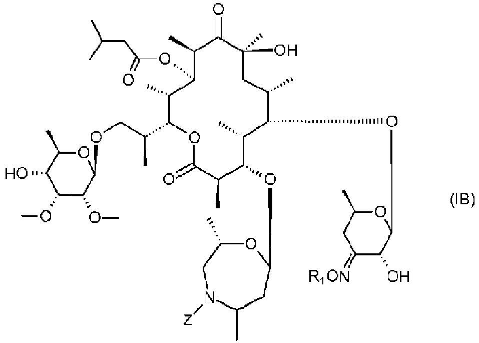 dans lequel R₁ et Z sont tels que définis pour les composés de formule (I) dans la revendication 1, réagit avec un composé de formule (II) HNR₂R₃ dans laquelle R₂ et R₃ sont tels que définis pour les composés de formule (I) dans la revendication 1, en présence d'un dérivé de carbonyle et d'une base.

9. Procédé de préparation d'un composé de formule (I) selon la revendication 1, dans lequel Y représente un groupe -(C=O)-NR₂R₃, **caractérisé en ce que** :
b-1) un composé de formule (V) : 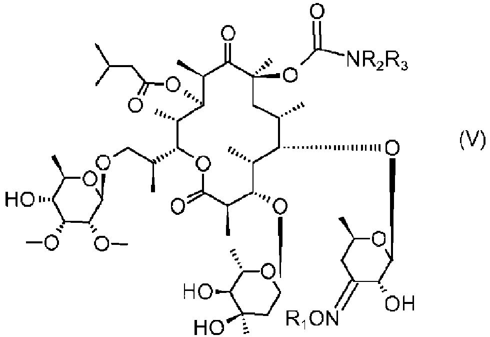 dans lequel R₁, R₂ et R₃ sont tels que définis pour un composé de formule (I) dans la revendication 1, réagit avec un agent oxydant pour obtenir un composé de formule (VI) : 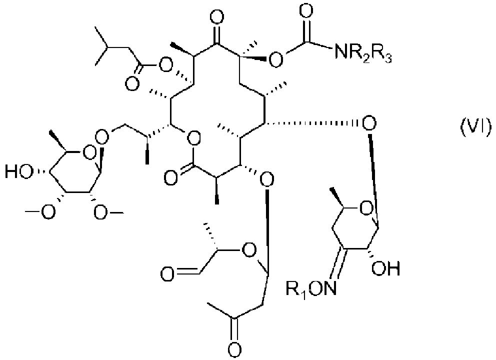
b-2) le composé de formule (VI) obtenu ainsi réagit avec un composé de formule (VII) :
ZNH₂ (VII)
dans lequel Z est tel que défini pour le composé (I) dans la revendication 1, en présence d'un agent réducteur.

10. Procédé de préparation d'un composé de formule (I) selon la revendication 1, dans lequel Y représente un atome d'hydrogène, **caractérisé en ce que** :
c-1) un composé de formule (VIII) : 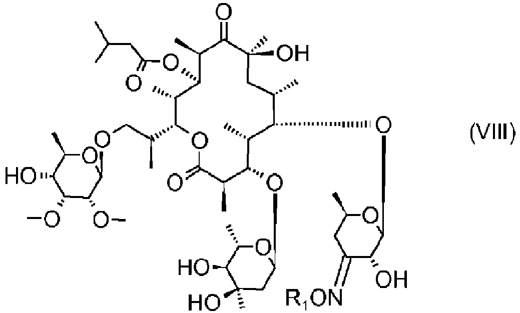 dans lequel R₁ est tel que défini pour le composé (I) dans la revendication 1, réagit avec un agent oxydant pour obtenir un composé de formule (IX) : 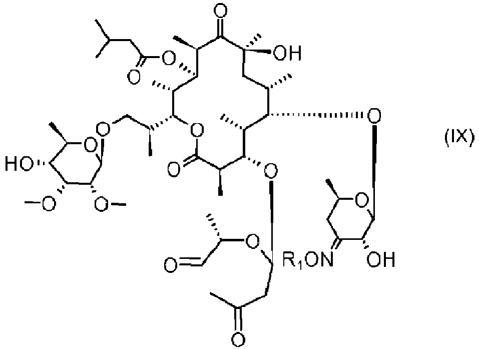
c-2) le composé de formule (IX) obtenu ainsi réagit avec un composé de formule (VII) :
ZNH₂ (VII)
dans lequel Z est tel que défini pour le composé (I) dans la revendication 1, en présence d'un agent réducteur.

11. Procédé de préparation d'un composé de formule (I) selon la revendication 1, dans lequel Y représente un groupe - (C=O) -O-R₁₈, **caractérisé en ce que** :
un composé de formule (XXI) : 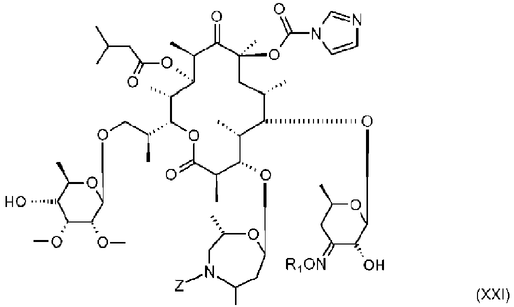 dans lequel Z et R₁ sont tels que définis pour un composé de formule (I) dans la revendication 1, réagit avec un alcool de formule HO-R₁₈ (XXII) dans lequel R₁₈ est tel que défini pour un composé de formule (I) dans la revendication 1, en présence d'une base.

12. Composé de formule (V) : 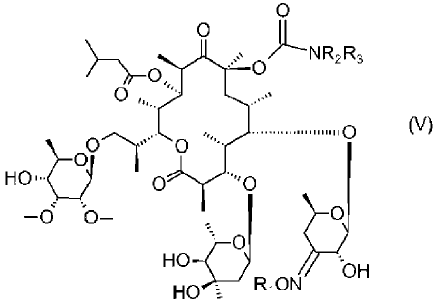 dans lequel :
- R₁, R₂ et R₃ sont tels que définis pour les composés de formule (I) dans la revendication 1.

13. Composé de formule (VIII) : 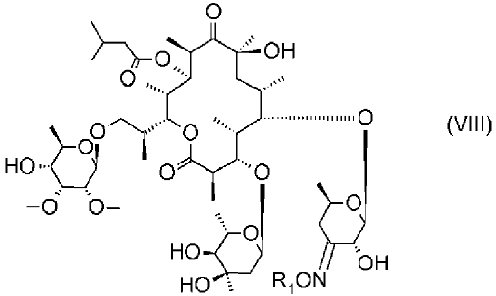 dans lequel :
- R₁ est tel que défini pour les composés de formule (I) dans la revendication 1.

14. Médicament, **caractérisé en ce qu'**il comprend un composé de formule (I) selon l'une quelconque des revendications 1 à 7, sous la forme d'un sel d'addition de base ou d'acide.

15. Composition pharmaceutique, **caractérisée en ce qu'**elle comprend un composé de formule (I) selon l'une quelconque des revendications 1 à 7, sous la forme d'un sel d'addition de base ou d'acide, et en outre au moins un excipient pharmaceutiquement acceptable.

16. Composé selon l'une quelconque des revendications 1 à 7, pour son utilisation pour la prévention et/ou le traitement d'infections bactériennes causées par des micro-organismes Gram positif et des mycobactéries.

17. Composé selon l'une quelconque des revendications 1 à 7, pour son utilisation pour la prévention et/ou le traitement de maladies infectieuses choisis parmi la tuberculose, la lèpre, la nocardiose, la diphtérie, une infection mycobactérienne pulmonaire, une infection mycobactérienne cutanée, une infection mycobactérienne atypique et une mycobactériose.
